(19) Europäisches Patentamt
European Patent Office
Office européen des brevets

(11) EP 1 116 727 B1

(12) **EUROPEAN PATENT SPECIFICATION**

(45) Date of publication and mention of the grant of the patent:
**19.03.2003 Bulletin 2003/12**

(21) Application number: **99944809.5**

(22) Date of filing: **24.09.1999**

(51) Int Cl.$^7$: **C07K 7/08**, C07K 14/72, A61K 38/10, A61K 38/16, C07K 7/06, C07K 14/47, A61K 38/08, A61K 38/17

(86) International application number:
**PCT/JP99/05216**

(87) International publication number:
**WO 00/018793 (06.04.2000 Gazette 2000/14)**

(54) **PEPTIDE DERIVATIVE**

PEPTID-DERIVAT

DERIVE DE PEPTIDE

(84) Designated Contracting States:
**AT BE CH CY DE DK ES FI FR GB GR IE IT LI LU MC NL PT SE**
Designated Extension States:
**AL LT LV MK RO SI**

(30) Priority: **25.09.1998 JP 27162698**

(43) Date of publication of application:
**18.07.2001 Bulletin 2001/29**

(73) Proprietor: **Takeda Chemical Industries, Ltd.**
**Osaka-shi, Osaka 541-0045 (JP)**

(72) Inventors:
- **KITADA, Chieko**
  **Sakai-shi, Osaka 590-0073 (JP)**
- **HINUMA, Shuji,**
  **Room 1402, Takeda Kasuga Haitsu**
  **Tsukuba-shi, Ibaraki 305-0821 (JP)**

(74) Representative: **Keller, Günter, Dr. et al**
**Lederer & Keller**
**Patentanwälte**
**Prinzregentenstrasse 16**
**80538 München (DE)**

(56) References cited:
**WO-A-99/33976**

- **KAZUHIKO TATEMOTO ET AL.: 'Isolation and Characterization of a Novel Endogenous Peptide Ligand for the Human APJ Receptor' BIOCHEM. BIOPHYS. RES. COMMUN., vol. 251, no. 2, 1998, pages 471 - 476, XP002101895**
- **BRIAN F. O'DOWD ET AL.: 'A Human Gene that Shows Identity with the Gene Encoding the Angiotensin Receptor is Located on Chromosome 11' GENE vol. 136, no. 1/2, 1993, pages 355 - 360, XP002101894**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 116 727 B1

## Description

FIELD OF THE INVENTION

The present invention relates to novel peptides that are orphan G protein-coupled receptor proteins and have ligand activities for APJ, and their use.

BACKGROUND ART

Many hormones or neurotransmitters regulate biological functions through specific receptors that exist on the cell membranes. These receptors are generically called G protein-coupled receptors or 7 transmembrane receptors, since many of them perform intracellular signal transduction by the activation of conjugated guanine nucleotide-binding proteins (abbreviated as G protein in some cases) and have common structures with 7 membrane domains.

Through interactions between these hormones or neurotransmitters and G protein-coupling receptors, the regulation of functions which are important for organisms are conducted, including the maintenance of homeostasis, the regulation of reproduction, individual development, metabolism, growth, nervous system, circulatory system, immune system, digestive system, and metabolic system. Although it has been known that receptor proteins for various hormones or neurotransmitters exist in regulating biological functions and play important roles in the regulation of those functions, it is unclear whether there are unknown active substances such as hormones or neurotransmitters, or corresponding receptors.

It has been found in recent years that DNAs, which code novel receptor proteins, can be searched by the polymerase chain reaction method (hereinafter abbreviated as PCR), taking advantage of the similarity of the amino acid sequence indicated in a part of the structures of G protein-coupled receptor proteins. This has enabled the cloning of a large number of so-called orphan G protein-coupled receptor proteins, for which ligands are unknown (Libert, F., et al., Science, 244, 569-572, 1989, Welch, S.K., et al., Biochem. Biophys. Res. Commun., 209, 606-613, 1995, Marchese, A., et al., Genomics, 23, 609-618, 1994, Marchese, A., Genomics, 29 335-344, 1995). Furthermore, novel G protein-coupled receptor proteins are found one after another by random sequencing of genome DNA or cDNA (Nomura, N., et al., DNA Research Vol. 1, 27-35, 1994). There has been no common method for determining ligands of these orphan G protein-coupled receptor proteins other than to estimate them based on the similarity in the primary structure of G protein-coupled receptor proteins. In practice, however, many orphan G protein-coupled receptor proteins showed low sequence homology with known receptors, and therefore it has been difficult to estimate the ligand simply from the similarity in the primary structure unless it was a receptor subtype of a known ligand. It is assumed that many corresponding unknown ligands exist since many orphan G protein-coupled receptor proteins have been found in gene analysis, but only a small number of ligands for orphan G protein-coupled receptor proteins have actually been identified so far.

Recently, some studies have been reported that searched for novel opioid peptides by transferring cDNAs that code orphan G protein-coupled receptor proteins into animal cells (Reinsheid, R. K. et al., Science, Vol. 270, 792-794, 1995; Menular, J.-C., et al., Nature Vol. 377, 532-535, 1995). However, in this case, it could easily be predicted that the ligand would belong to the opioid peptide family, based on its similarity to known G protein-coupled receptor proteins and the distribution in tissues. Research and development on substances that act upon biological organisms through opioid peptides have a long history, and various antagonists and agonists have been developed. Therefore, after identifying agonists for this receptor among a series of artificially synthesized compounds and verifying the expression of a receptor in receptor cDNA transferred cells using such an agonist as the probe, the activator of intracellular signal transduction system that is similar to agonists was identified and purified to determine the structure of ligands.

It has been reported that a novel physiologically active peptide was identified with the specific increase in the intracellular free calcium levels in receptor expressing cells as the indicator by transferring cDNAs that code orphan G protein-coupled receptor proteins (GRL104) of snails into CHO cells (Cox, K. J. A., et al., Neurosci., 17(4), 1197-1205, 1997). This physiologically active peptide had high homology with known leucokinins and GRL104 was found reactive to known leucokinins. Thus, there rarely were orphan G protein-coupled receptor proteins of which ligands could be roughly predicted, and when the similarity to known G protein-coupled receptor protein family was low, in particular, there has been very limited information about ligands and it has been difficult to predict the ligand.

One of those reported as orphan G protein-coupled receptors is APJ (O'Dowd, B.F., et al., Gene, 436, 355-359, 1993). APJ has low homology with the angiotensin receptor (AT1). Although a natural ligand to APJ and its partial peptide sequence were described in WO 99/33976 (Japanese Patent Application No. 220853-1998), artificially modified natural ligands, such as natural ligands with one or more constituent amino acids are substituted by other amino acids or those with side chains of one or more constituent amino acids substituted by appropriate substituents are completely unknown.

DISCLOSURE OF THE INVENTION

Peptides that have been artificially modified natural ligands to APJ, an orphan G protein-coupled receptor expressed in the central nervous system, the circulatory system, the reproductive system, the immune system or digestive organs, are considered to be more useful for pharmaceuticals compared to natural ligands, while the structure and function of peptides that are more useful as pharmaceuticals compared to natural ligands have not been demonstrated yet.

In consideration of such problems, the inventors synthesized various novel peptides by using a change in binding properties between modified product of said natural ligands and receptor proteins described above as the indicator and found modified natural ligands that are more useful as pharmaceuticals by predicting and specifying active sites of natural ligands.

Namely, the present invention relates to:

(1) A peptide represented by the formula:

$$X^1\text{-Arg-Pro-Arg-}X^2\text{-Ser-His-}X^3\text{-Gly-Pro-}X^4\text{-}X^5$$

[wherein $X^1$ represents a hydrogen atom or an amino acid residue comprising 1 to 25 amino acids each of which, whether identical or not, has a side chain which may be substituted, or a peptide chain; $X^2$ represents a neutral amino acid residue having a side chain which may be substituted; $X^3$ represents a neutral amino acid residue having a side chain which may be substituted, an aromatic amino acid residue having a side chain which may be substituted or a basic amino acid residue having a side chain which may be substituted; $X^4$ represents a bond or a neutral or aromatic amino acid residue having a side chain which may be substituted; $X^5$ represents ① an amino acid residue having a side chain which may be substituted or an amino acid derivative with the C-terminal carboxyl group reduced to a hydroxymethyl group or a formyl group, ② a hydroxyl group or ③ a dipeptide chain comprising an amino acid residue having a side chain which may be substituted and an amino acid residue havinga side chain which may be substituted, bound together, or a peptide derivative with the C-terminal carboxyl group reduced to a hydroxymethyl group or a formyl group; and a side chain in each amino acid residue in -Arg-Pro-Arg-, -Ser-His- or -Gly-Pro- in the formula may be substituted; except the case where $X^2$ represents Leu, $X^3$ represents Lys, $X^4$ represents Met, $X^5$ represents ① Pro or ② Pro-Phe, and -Arg-Pro-Arg- in the formula represents unsubstituted -Arg-Pro-Arg-, -Ser-His- represents unsubstituted -Ser-His-, and -Gly-Pro- represents unsubstituted -Gly-Pro-] or an ester thereof (hereinafter may be abbreviated as peptides of the present invention), or their salt.

(2) A peptide, as described in (1) above, wherein $X^1$ is an amino acid residue having a side chain which may be substituted, or an ester thereof or their salt.

(3) A peptide as described in (1) above, wherein $X^1$ is pGlu which may be substituted or Gln having a side chain which may be substituted, or an ester thereof or their salt.

(4) A peptide as described in (1) above, wherein $X^1$ is represented by the formula $Y^1$-$Y^2$ (wherein $Y^1$ represents an amino acid residue comprising 1 to 17 amino acids each of which, whether identical or not, has a side chain which may be substituted, or a peptide chain, and $Y^2$ represents an amino acid residue comprising 1 to 8 amino acids each of which, whether identical or not, has a side chain which may be substituted or a peptide chain), or an ester thereof or their salt.

(5) A peptide as described in (4) above, wherein $Y^2$ is a peptide chain represented by ① the formula $B^1$-$B^2$-$B^3$-$B^4$-$B^5$-$B^6$-$B^7$-$B^8$ (wherein each of $B^1$ through $B^8$, whether identical or not, represents an amino acid residue having a side chain which may be substituted) ② the formula $B^2$-$B^3$-$B^4$-$B^5$-$B^6$-$B^7$-$B^8$ (wherein the symbols have the same definitions as given above), ③ the formula $B^3$-$B^4$-$B^5$-$B^6$-$B^7$-$B^8$ (wherein the symbols have the same definitions as those given above), ④ the formula $B^4$-$B^5$-$B^6$-$B^7$-$B^8$ (wherein the symbols have the same definitions as those given above), ⑤ the formula $B^5$-$B^6$-$B^7$-$B^8$ (wherein the symbols have the same definitions as those given above), ⑥ the formula $B^6$-$B^7$-$B^8$ (wherein the symbols have the same definitions as those given above), ⑦ the formula $B^7$-$B^8$, wherein the symbols have the same definitions as those given above), or ⑧ the formula $B^8$ (wherein $B^8$ has the same definition as that given above), or an ester thereof or their salt.

(6) A peptide as described in (5) above, wherein $B^1$ is a neutral amino acid residue having a side chain which may be substituted, or an ester thereof or their salt.

(7) A peptide as described in (6) above, wherein $B^1$ is Gly which may be substituted, or an ester thereof or their salt.

(8) A peptide as described in (5) above, wherein each of $B^2$, $B^3$ and $B^4$, whether identical or not, is a basic amino acid residue having a side chain which may be substituted, or an ester thereof or their salt.

(9) A peptide as described in (5) above, wherein $B^5$ is an amino acid residue having an aromatic side chain, or an ester thereof or their salt.

(10) A peptide as described in (5) above, wherein each of $B^6$ and $B^7$, whether identical or not, is a basic amino acid residue having a side chain which may be substituted, or an ester thereof or their salt.
(11) A peptide as described in (5) above, wherein $B^8$ is Gln having a side chain which may be substituted, or an ester thereof or their salt.
(12) A peptide as described in (4) above, wherein $Y^1$ is an amino acid residue or peptide chain represented by (a) the formula $A^1$-$A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein each of $A^1$ to $A^{17}$, whether identical or not, represents an amino acid residue having a side chain which may be substituted), (b) the formula $A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (c) the formula $A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (d) the formula $A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (e) the formula $A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (f) the formula $A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (g) the formula $A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (h) the formula $A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (i) the formula $A^9$-$A^{10}$-$A^{11}$-$A^{12}$-$A^{13}$-$A^{14}$-$A^{15}$-$A^{16}$-$A^{17}$ (wherein the symbols have the same definitions as those given above), (j) the formula $A^{10}$-$A^{11}$-$A^{12}$-$A^{13}$-$A^{14}$-$A^{15}$-$A^{16}$-$A^{17}$ (wherein the symbols have the same definitions as those given above), (k) the formula $A^{11}$-$A^{12}$-$A^{13}$-$A^{14}$-$A^{15}$-$A^{16}$-$A^{17}$, wherein the symbols have the same definitions as those given above), (l) the formula $A^{12}$-$A^{13}$-$A^{14}$-$A^{15}$-$A^{16}$-$A^{17}$ (wherein the symbols have the same definitions as those given above), (m) the formula $A^{13}$-$A^{14}$-$A^{15}$-$A^{16}$-$A^{17}$ (wherein the symbols have the same definitions as those given above), (n) the formula $A^{14}$-$A^{15}$-$A^{16}$-$A^{17}$ (wherein the symbols have the same definitions as those given above), (o) the formula $A^{15}$-$A^{16}$-$A^{17}$ (wherein the symbols have the same definitions as those given above), (p) the formula $A^{16}$-$A^{17}$ (wherein the symbols have the same definitions as those given above), or (q) $A^{17}$ (wherein $A^{17}$ has the same definitions as that given above), or an ester thereof or their salt.
(13) An amino acid residue, or peptideas described in (12) above, wherein $A^1$ is an amino acid residue having an aromatic side chain, or an ester thereof or their salt.
(14) A peptide as described in (12) above, wherein $A^2$ and $A^3$ are neutral amino acid residues having a side chain which may be substituted, or an ester thereof or their salt.
(15) A peptide as described in (12) above, wherein $A^4$ is a neutral or a basic L-amino acid residue having a side chain is optionally substituted.
(16) A peptide as described in (12) above, wherein $A^5$ is Pro having a side chain which may be substituted, or an ester thereof or their salt.
(17) A peptide as described in (12) above, wherein each of $A^6$ and $A^9$-, whether identical or not, is a basic amino acid residue having a side chain which may be substituted, or an ester thereof or their salt.
(18) A peptide as described in (12) above, wherein each of $A^7$ and $A^{10}$-, whether identical or not, is an amino acid residue having a hydroxy group in a side chain thereof or a neutral amino acid residue having a side chain which may be substituted, or an ester thereof or their salt.
(19) A peptide as described in (12) above, wherein $A^8$ is an amino acid residue having a hydroxy group in a side chain thereof, or an ester thereof or their salt.
(20) A peptide as described in (12) above, wherein $A^8$ is Ser, Pro or Hyp, or an ester thereof or their salt.
(21) A peptide as described in (12) above, wherein $A^{10}$ is an amino acid residue having a hydroxy group in a side chain thereof, or an ester thereof or their salt.
(22) A peptide as described in (12) above, wherein $A^{11}$ through $A^{14}$, whether identical or not, is a neutral amino acid residue having a side chain which may be substituted, or an ester thereof or their salt.
(23) A peptide as described in (12) above, wherein $A^{15}$ is an amino acid residue having an aromatic side chain, or an ester thereof or their salt.
(24) A peptide as described in (23) above, wherein $A^{15}$ is Trp, or an ester thereof or their salt.
(25) An amino acid residue or peptide as described in (12) above, wherein each of $A^{16}$ and $A^{17}$, whether identical or not, represents a neutral amino acid residue having a side chain which may be substituted, or an ester thereof or their salt.
(26) A pharmaceutical containing a peptide, as described in (1) above, or an ester thereof or their salt.
(27) A pharmaceutical as described in (26) above which is a central nervous function regulator, a circulatory function regulator, a cardiac function regulator, an immune function regulator, a digestive function regulator, a metabolic function regulator or a reproductive organ function regulator.
(28) A pharmaceutical as described in (26) above which is an agonist of a protein containing an amino acid sequence shown by Sequence ID No.:26 or a salt thereof.

Additionally, the present invention provides:

(29) Pharmaceuticals or the like described in (26) above that are therapeutic or prophylactic drugs against disorders, such as: dementia, depression, ADHD (attention-deficit-hyperactivity disorder; minimal brain dysfunction) syndrome, disturbance of consciousness, anxiety disorder, schizophrenia, phobia, growth hormone secretion disorder, hyperphagia, polyphagia, hypercholesterolemia, hyperglyceridemia, hyperlipidemia, hyperprolactinemia, diabetes, cancer, pancreatitis, renal diseases, Turner's syndrome, neuropathy, rheumatoid arthritis, spinal cord injury, transient ischemic attack, amyotrophic lateral sclerosis, acute myocardial infarct, spinocerebellar degeneration, fracture, injury, atopic dermatitis, osteoporosis, asthma, epilepsy, infertility, arteriosclerosis, emphysema pulmonum, pulmonary edema, hypogalactia or AIDS.

## BRIEF DESCRIPTION OF THE DRAWINGS

Figure 1 shows changes in Acidification Rates of peptides in Examples 3 and 4, and of corresponding natural peptides in Example 1.

In this figure, ▲-▲ shows the amount of changes in Acidification Rates of peptides obtained in Examples 3, □-□ shows those of peptides obtained in Examples 4, and ●- ● shows those of corresponding natural peptides in Example 1.

## BEST MODE OF EMBODIMENT OF THE INVENTION

In the present invention, an amino acid residue refers to the structure of the part other than N- or C-terminals when amino acids form peptide bonds and are incorporated into proteins or peptides by losing water molecules. For example, an α-amino acid residue refers to the structure of $-NHC(R^0)(R^1)CO-$ other than N- or C-terminals when α-amino acid ($H_2NC(R^0)(R^1)COOH$: $R^0$ and $R^1$, whether identical or not, represents an optional substituent) forms peptide bond and is incorporated into proteins or peptides by losing water molecules. In this case, an amino acid residue at the N-terminal is represented by $H_2NC(R^0)(R^1)CO-$ and at the C-terminal as $-NHC(R^0)(R^1)COOH$. Similarly, a β-amino acid residue refers to the structure of $-NHC(R^0)(R^1)C(R^2)(R^3)CO-$ other than N- or C-terminals when β-amino acid ($H_2NC(R^0)(R^1)C(R^2)(R^3)COOH$. $R^0$, $R^1$, $R^2$ and $R^3$, whether identical or not, represents an optional substituent) forms peptide bonds and is incorporated into proteins or peptides by losing water molecules. In this case, an amino acid residue at the N-terminal is represented by $H_2NC(R^0)(R^1)C(R^2)(R^3)CO-$ and at the C-terminal as $-NHC(R^0)(R^1)C(R^2)(R^3)COOH$. Moreover, a γ-amino acid residue refers to the structure of $-NHC(R^0)(R^1)C(R^2)(R^3)$ $C(R^4)(R^5)CO-$ other than N- or C-terminals when ε-amino acid ($H_2NC(R^0)(R^1)C(R^2)(R^3)C(R^4)(R^5)COOH$: $R^0$, $R^1$, $R^2$, $R^3$, $R^4$ and $R^5$, whether identical or not, represents an optional substituent) forms peptide bonds and is incorporated into proteins or peptides by losing water molecules. In this case, an amino acid residue at the N-terminal is represented by $H_2NC(R^0)(R^1)C(R^2)(R^3)C(R^4)(R^5)CO-$ and at the C-terminal as $-NHC(R^0)(R^1)C(R^2)(R^3)C(R^4)(R^5)COOH$. Moreover, an ε-amino acid residue refers to the structure of $-NHC(R^0)(R^1)C(R^2)(R^3)C(R^4)(R^5)C(R^6)(R^7)C(R^8)(R^9)CO-$ other than N- or C-terminals when ε-amino acid ($H_2NC(R^0)(R^1)C(R^2)(R^3)C(R^4)(R^5)C(R^6)(R^7)C(R^8)(R^9)COOH$: $R^0$, $R^1$, $R^2$, $R^3$, $R^4$, $R^5$, $R^6$, $R^7$, $R^8$ and $R^9$, whether identical or not, represents an optional substituent) forms peptide bonds and is incorporated into proteins or peptides by losing water molecules. In this case, an amino acid residue at the N-terminal is represented by $H_2NC(R^0)(R^1)C(R^2)(R^3)C(R^4)(R^5)C(R^6)(R^7)C(R^8)(R^9)CO-$ and at the C-terminal as $-NHC(R^0)$ $(R^1)C(R^2)(R^3)C(R^4)(R^5)C(R^6)(R^7)C(R^8)(R^9)COOH$.

In this specification, an amino acid is a natural or non-natural amino acid that may be either D- or L-configuration, and also be of either type α, β, γ, or ε.

Groups that form the side chains of α-amino acids, β-amino acids, γ-amino acids, or ε-amino acids include: (1) $C_{1-6}$ alkyl group (e.g. methyl, ethyl, n-propyl, isopropyl, n-butyl, isobutyl, sec-butyl, tert-butyl, n-pentyl, and preferably $C_{1-3}$ alkyl); (2) cyano group; (3) halogen (e.g. fluorine, chlorine, bromine, and iodine); (4) hydroxy-$C_{1-6}$ alkyl group (e.g. hydroxymethyl, and hydroxyethyl); (5) $C_{1-6}$ alkoxy group (e.g, methoxy, ethoxy, n-propoxy, isopropoxy, n-butoxy, tert-butoxy, and preferably $C_{1-3}$ alkoxy); (6) $C_{1-6}$ alkoxy-carbonyl group (e.g. methoxycarbonyl, ethoxycarbonyl, isopropoxycarbonyl, tert-butoxycarbonyl, and preferably $C_{1-3}$ alkoxy-carbonyl); (7) $C_{1-4}$ acyl group (e.g. formyl such as formyl, acetyl, propionyl and butyryl, and $C_{2-4}$ alkanoyl); (8) hydroxy group; (9) groups represented by the formula: $-S(O)a-R^{21}$ [where: a represents an integer between 0 and 2; $R^{21}$ represents $C_{1-6}$ alkyl (specific examples are the same as the above)] such as methylthio, methanesulfinyl, methanesulfonyl, ethylthio, ethanesulfinyl, ethanesulfonyl; (10) benzyloxycarbonyl; (11) tosyl group; (12) carbamoyl group; (13) mercapto group; (14) amino group; (15) sulfo group; (16) phosphono group; (17) phospho group; (18) carboxyl group; (19) tetrazolyl group; (20) amino-$C_{1-6}$ alkyl group (e.g. amino methyl, amino ethyl); (21) amino allyl group; (22) thiazolyl group; (23) thienyl group; (24) oxazolyl group; (25) furyl group; (26) pyranyl group; (27) pyridyl group; (28) pyrazyl group; (29) pyrazinyl group; (30) pyrimidinyl group; (31) pyridazinyl group; (32) indolyl group; (33) indodinyl group; (34) isoindolyl group; (35) pyrrolyl group; (36) imidazolyl group; (37) isothiazolyl group; (38) pyrazolyl group; (39) chromenyl group; (40) purinyl group; (41) quinolizinyl group; (42) quinolyl group; (43) isoquinolyl group; (44) quinazolinyl group; (45) quinoxalinyl group; (46) cinnolinyl group; (47) morpholinyl group; (48) benzothienyl group; (49) benzofuranyl group; (50) benzimidazolyl; (51) benzimidazolyl group;

(52) $C_{3-8}$ cycloalkyl group; (53) $C_{1-4}$ alkyl group substituted by the substituents. described in (2), (3), (5) to (17), and (20) to (52) above; (54) $C_{1-4}$ acyl group, such as formyl and $C_{2-4}$ alkanoyl, substituted by the substituents described in (2), (3), (5) to (17), and (20) to (52) above; (55) $C_{6-10}$ aryl group such as phenyl substituted by the substituents described in (1) to (52) above (e.g. mesityl, tolyl, xylyl, styrenyl); (56) $C_{7-15}$ aralkyl group such as benzyl substituted by the substituents described in (1) to (52) above (e.g. methylbenzyl methoxybenzyl); (57) $C_{7-15}$ aralkyl group (e.g. benzyl, phenethyl, benzhydryl, naphthylmethyl); (58) $C_{6-10}$ aryl group (e.g. phenyl, naphthyl, indenyl); and (59) hydrogen atom.

The side chain that forms an amino acid residue and a nitrogen atom can bind together to form a ring (e.g. proline), or two side chains can bind together to form a ring (e.g. 3-amino-norbornane-carboxylic acid).

Examples of α-amino acid include glycine, alanine, valine, leucine, isoleucine, serine, threonine, cysteine, methionine, aspartic acid, glutamic acid, lysine, arginine, phenylalanine, tyrosine, histidine, tryptophan, asparagine, glutamine, proline, pipecolic acid, norleucine, γ-methylleucine, tert-leucine, norvaline, homoarginine, homoserine, α-aminoisobutyric acid, α-aminobutyric acid, ornithine, α-aminoadipic acid, phenylglycine, thienylglycine, cyclohexylglycine, cyclohexylalanine, thienylalanine, naphthylalanine, octa-hydro-indole-2-carboxylic acid, adamantylalanine, benzothienylalanine, pyridylalanine, piperidilalanine, pyrazylalanine, quinolylalanine, thiazolylalanine, homocysteine, homophenylalanine, citrulline, homocitrulline, oxyproline (hydroxyproline), α,β-diaminopropionic acid, α,γ-diaminobutyric acid, aminomalonic acid, 1,2,3,4-tetrahydroisoquinoline-3-carboxylic acid, penicillamine, cycloleucine, and 2-amino-4-pentenoic acid.

Examples of β-amino acid include β-alanine, β-aminobutyric acid, isoasparagine, 3-aminoadipic acid, 3-amino-phenylpropionic acid, 3-amino-2-hydroxy-4-phenyl aminobutyric acid, 3-amino-norbornane-carboxylic acid and 3-amino-bicycloheptane carboxylic acid.

Examples of γ-amino acid include γ-aminobutyric acid, isoglutamine, statin, 4-amino-3-hydroxy-5-cyclohexylpentanoic acid, 4-amino-3-hydroxy-5-phenylpentanoic acid, 6-aminopenicillanic acid and 3-aminoadamantine-1-carboxylic acid.

Examples of ε-amino acid include ε-amino caproic acid and 4-amino methyl-cyclohexane carboxylic acid.

Said natural amino acids include glycine, alanine, valine, leucine, isoleucine, serine, threonine, cysteine, cystine, methionine, aspartic acid, glutamic acid, lysine, arginine, phenylalanine, tyrosine, histidine, tryptophan, asparagine, glutamine, proline, ornithine and citrulline.

Said non-natural amino acids include norleucine, γ-methylleucine, tert-leucine, norvaline, homoarginine, homoserine, aminoisobutyric acid, aminoadipic acid (e.g. α-aminoadipic acid), phenylglycine, thienylglycine, cyclohexylglycine, aminobutyric acid, β-alanine, cyclohexylalanine, thienylalanine, naphthylalanine, adamantylalanine, benzothienylalanine, pyridylalanine, piperidilalanine, pyrazylalanine, quinolylalanine, thiazolylalanine, isoasparagine, isoglutamine, homocysteine, homophenylalanine, homocitrulline, oxyproline (hydroxyproline), diaminopropionic acid, diaminobutyric acid, aminobenzoic acid, and N-methylated amino acids of natural and non-natural amino acids described above.

Substituents that may be substituted for side chains of these amino acid residues include (1) $C_{1-6}$ alkyl group (e.g. methyl, ethyl, n-propyl, isopropyl, n-butyl, isobutyl, sec-butyl, tert-butyl, n-pentyl, and preferably $C_{1-3}$ alkyl); (2) cyano group; (3) halogen (e.g. fluorine, chlorine, bromine, and iodine); (4) hydroxy-$C_{1-6}$ alkyl group (e.g. hydroxymethyl, and hydroxyethyl); (5) $C_{1-6}$ alkoxy group (e.g, methoxy, ethoxy, n-propoxy, isopropoxy, n-butoxy, tert-butoxy, and preferably $C_{1-3}$ alkoxy); (6) $C_{1-6}$ alkoxy-carbonyl group (e.g. methoxycarbonyl, ethoxycarbonyl, isopropoxycarbonyl, tert-butoxycarbonyl, and preferably $C_{1-3}$ alkoxy-carbonyl); (7) $C_{1-4}$ acyl group (e.g. formyl such as formyl, acetyl, propionyl and butyryl, and $C_{2-4}$ alkanoyl); (8) hydroxy group; (9) groups represented by the formula: $-S(O)a-R^{21}$ [where: a represents an integer between 0 and 2; $R^{21}$ represents $C_{1-6}$ alkyl (specific examples are the same as the above)] such as methylthio, methanesulfinyl, methanesulfonyl, ethylthio, ethanesulfinyl, ethanesulfonyl; (10) benzyloxycarbonyl; (11) tosyl group; (12) carbamoyl group; (13) mercapto group; (14) amino group; (15) sulfo group; (16) phosphono group; (17) phospho group; (18) carboxyl group; (19) tetrazolyl group; (20) amino-$C_{1-6}$ alkyl group (e.g. amino methyl, amino ethyl); (21) amino allyl group; (22) thiazolyl group; (23) thienyl group; (24) oxazolyl group; (25) furyl group; (26) pyranyl group; (27) pyridyl group; (28) pyrazyl group; (29) pyrazinyl group; (30) pyrimidinyl group; (31) pyridazinyl group; (32) indolyl group; (33) indodinyl group; (34) isoindolyl group; (35) pyrrolyl group; (36) imidazolyl group; (37) isothiazolyl group; (38) pyrazolyl group; (39) chromenyl group; (40) purinyl group; (41) quinolizinyl group; (42) quinolyl group; (43) isoquinolyl group; (44) quinazolinyl group; (45) quinoxalinyl group; (46) cinnolinyl group; (47) morpholinyl group; (48) benzothienyl group; (49) benzofuranyl group; (50) benzimidazolyl; (51) benzimidazolyl group; (52) $C_{3-8}$ cycloalkyl group; (53) oxo group (54) $C_{1-4}$ alkyl group substituted by the substituents described in (2), (3), (5) to (19), and (22) to (52) above; (55) $C_{1-4}$ acyl group, such as formyl and $C_{2-4}$ alkanoyl, substituted by the substituents described in (2), (3), (5) to (19), and (22) to (52) above; (56) $C_{6-10}$ aryl group such as phenyl substituted by the substituents described in (1) to (52) above (e.g. mesityl, tolyl, xylyl, styrenyl); (57) $C_{7-15}$ aralkyl group such as benzyl substituted by the substituents described in (1) to (52) above (e.g. methylbenzyl methoxybenzyl); (58) $C_{7-15}$ aralkyl group (e.g. benzyl, phenethyl, benzhydryl, naphthylmethyl); and (59) $C_{6-10}$ aryl group (e.g. phenyl, naphthyl, indenyl).

Neutral substituents include (1) $C_{1-6}$ alkyl group (e.g. methyl, ethyl, n-propyl, isopropyl, n-butyl, isobutyl, sec-butyl, tert-butyl, n-pentyl, and preferably $C_{1-3}$ alkyl); (2) cyano group; (3) halogen (e.g. fluorine, chlorine, bromine, and iodine); (4) hydroxy-$C_{1-6}$ alkyl group (e.g. hydroxymethyl, and hydroxyethyl); (5) hydroxy group; (6) carbamoyl group; (7) mercapto group; (8) groups represented by the formula: -S(O)a-$R^{21}$ (wherein the symbols have the same definitions as those given above), (9) $C_{6-10}$ aryl group (e.g. phenyl, naphthyl, indenyl, chromenyl); (10) thienyl group; (11) oxazolyl group; (12) furyl group; (13) indolyl group; (14) indodinyl group; (15) isoindolyl group; (16) $C_{3-8}$ cycloalkyl group; (17) oxo group; (18) $C_{1-6}$ alkyl substituted by the substituents described in (2), (3), (5) to (16)above; (19) $C_{6-10}$ aryl group, such as phenyl and naphthyl, substituted by the substituents described in (1) to (16) above (e.g. mesityl, tolyl, xylyl, styrenyl); and (20) $C_{7-15}$ aralkyl group such as benzyl substituted by the substituents described in (1) to (16) above (e. g. methylbenzyl methoxybenzyl).

Acidic substituents include $C_{1-4}$ alkyl group, $C_{6-10}$ aryl group such as phenyl and naphthyl, $C_{7-15}$ aralkyl group such as benzyl, and carboxyl group that have been individually substituted by carboxyl group, sulfo group or tetrazolyl group.

Basic substituents include (1) amino-$C_{1-6}$ alkyl group (e.g. amino methyl, amino ethyl); (2) amino allyl group; (3) pyridyl group; (4) pyrazyl group; (5) pyrazinyl group; (6) pyridazinyl group (7) imidazolyl group; (8) pyrazolyl group; (9) pyrazolyl group; (10) morpholinyl group; (11) amino group; (12) $C_{1-4}$ alkyl group substituted by the substituents described in (3) to (10) above; (13) $C_{7-15}$ aralkyl group such as benzyl substituted by the substituents described in (1) to (11) above; and (14) $C_{6-10}$ aryl group, such as phenyl and naphthyl, substituted by the substituents described in (1) to (11) above.

In this specification, acidic amino acids include, for example, amino acids with side chains having acid group such as carboxyl group, sulfo group; or tetrazolyl group. The specific examples of them specifically include glutamic acid, pyroglutamic acid, aspartic acid, cysteic acid, homocysteic acid, 3-(5-tetrazolyl)alanine, and 2-amino-4-(5-tetrazolyl)butyric acid.

In this specification, basic amino acids include, for example, histidine, arginine, ornithine, lysine diaminopropionic acid, diaminobutyric acid and homoarginine.

Basic amino acids with substituted side chains specifically include $N^{\alpha}$-acetylarginine, $N^{\varepsilon}$-tosylarginine, $N^{\varepsilon}$-acetyllysine, $N^{\varepsilon}$-methyllysine, and $N^{\varepsilon}$-tosyllysine.

In this specification, neutral amino acids specifically include alanine, valine, norvaline, leucine, isoleucine, alloisoleucine, norleucine, tert-leucine, γ-methylleucine, proline, phenylglycine, phenylalanine, glutamine, asparagines, serine, threonine, glycine, cysteine, methionine, tryptophan, oxyproline (hydroxyproline) and cyclohexylalanine. Neutral amino acids with substituted side chains specifically include biphenylalanine.

In this specification, amino acid residues with aromatic side chains specifically include tryptophan, phenylalanine, tyrosine, 1-naphthylalanine, 2-naphthylalanine, 2-thienylalanine, histidine, pyridylalanine (2-pyridylalanine), and O-methyltyrosine. Amino acid residues having aromatic side chains of which side chains are substituted specifically include 3-iodotyrosine, p-phosphonomethyl phenylalanine and O-phospho tyrosine.

In this specification, amino acid residues with side chains having hydroxy group include serine, threonine, tyrosine and oxyproline (hydroxyproline).

In this specification, the left end of a peptide or peptide chain indicates the N terminal (the amino terminal) and the right end the C terminal (the carboxyl terminal) following the conventional marking rules for peptides.

The C terminals of a peptide or peptide chain in the present invention is usually a carboxyl group (-COOH) or carboxylate ($-COO^-$), but in some cases it can be an amide ($-CONH_2$) or an ester (-COOR). R in an ester represents, for example, $C_{1-6}$ alkyl group including methyl, ethyl, n-propyl, isopropyl or n-butyl; $C_{3-8}$ cycloalkyl group including cyclopentyl and cyclohexyl; $C_{6-12}$ aryl group including phenyl and α-naphthyl; and $C_{7-14}$ aralkyl group including phenyl-$C_{1-2}$ alkyl such as benzyl, phenethyl and benzhydryl, or α-naphthyl-$C_{1-2}$ alkyl such as α-naphthylmethyl, as well as pivaloyloxymethyl generally used as an ester for oral administration.

When peptides of the present invention have a carboxyl group or carboxylate other than the C terminal, those of which groups are amidated or esterified are also included among polypeptides of the present invention. For the ester, in this case, the ester at the C terminal described above may be used.

A peptide and peptide chain of the present invention include those that have an amino group in an amino acid residue at the N-terminal is substituted by a substituent such as ① $C_{2-6}$ alkanoyl group including formyl and acetyl, $C_{1-8}$ acyl group including guanidinoacetyl, thienyl acrylyl and pyridyl acetyl; ② $C_{1-6}$ alkyl group including methyl, ethyl, propyl, isopropyl, butyl, isobutyl, sec-butyl, tert-butyl, pentyl, and hexyl; ③ $C_{6-10}$ aryl group including phenyl and naphthyl, or $C_{7-16}$ aralkyl group including benzyl and phenethyl; ④ tosyl group (p-toluenesulfonyl group); ⑤ benzyloxycarbonyl group; ⑥ a group represented by the formula: -S(O)a-$R^{22}$ (where: a represents an integer between 0 and 2; $R^{22}$ represents $C_{1-6}$ alkyl (specific examples are the same as the above) such as methylthio, methanesulfinyl, methanesulfonyl, ethylthio, ethanesulfinyl, ethanesulfonyl; ⑦ t-butoxycarbonyl group; or ⑧ N-9-fluorenylmethoxycarbonyl), those with pyroglutaminated glutamine residue at the N-terminal, which is formed by being cleaved in vivo, those that have a substituent including -OH, -SH, amino group, imidazolyl group, indolyl group and guanidine group on a side chain of an amino acid in a molecule is protected by an appropriate protecting group such as $C_{1-6}$ acyl group including $C_{1-6}$ alkanoyl group such as formyl group and acetyl group), and conjugated proteins, including so-called glycoproteins that are formed by linking saccharide chains.

The formula above:

$X^1$-Arg-Pro-Arg-$X^2$-Ser-His- $X^3$-Gly-Pro-$X^4$-$X^5$ wherein a side chain of each amino acid residue in -Arg-Pro-Arg-, -Ser-His-, and -Gly-Pro- may be substituted and substituents include those described above.

In this specification, $X^1$ represents "a hydrogen atom or an amino acid residue comprising 1 to 25 amino acids each of which, whether identical or not, has a side chain which may be substituted, or a peptide chain."

Substituent of said "1 to 25 amino acids each of which , whether identical or not, has a side chain which may be substituted" includes, for example, the one similar to "a substituent may be substituted for a side chain in an amino acid residue" described above.

Preferable examples where $X^1$ represents "an amino acid residue having a side chain which may be substituted" include pyroglutamic acid which may be substituted, or glutamine having a side chain which may be substituted, and more preferably, pyroglutamic acid or glutamine.

Substituents for amino acid residues in said "an amino acid residue having a side chain which may be substituted," "pyroglutamic acid which may be substituted" and "glutamine having a side chain which may be substituted" include those similar to "a substituent which may be substituted by a side chain in an amino acid residue" above.

Preferred substituents for "pyroglutamic acid which may be substituted" include a benzyloxycarbonyl group.

Specific examples where $X^1$ represents "a peptide chain comprising 2 to 25 amino acids each of which, whether identical or not, has a side chain which may be substituted" include a peptide represented by the formula:

$$Y^1\text{-}Y^2,$$

(wherein $Y^1$ represents an amino acid residue comprising 1 to 17 amino acids each of which, whether identical or not, has a side chain which may be substituted, or a peptide chain, and $Y^2$ represents an amino acid residue comprising 1 to 8 amino acids each of, whether identical or not, has a side chain which may be substituted, or a peptide chain.

Substituents for a side chain in an amino acid residue in an amino acid residue or a peptide chain represented by $Y^1$ and $Y^2$ described above include the one similar to "a substituent which may be substituted by a side chain in an amino acid residue" described above.

Specific examples for $Y^1$ described above include an amino acid residue or peptide chainrepresented by

(a) the formula $A^1$-$A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein each of $A^1$ to $A^{17}$, whether identical or not, represents an amino acid residue having a side chain which may be substituted),
(b) the formula $A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above),
(c) the formula $A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above),
(d) the formula $A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above),
(e) the formula $A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above),
(f) the formula $A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above),
(g) the formula $A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above),
(h) the formula $A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above),
(i) the formula $A^9$-$A^{10}$-$A^{11}$-$A^{12}$-$A^{13}$-$A^{14}$-$A^{15}$-$A^{16}$-$A^{17}$ (wherein the symbols have the same definitions as those given above),
(j) the formula $A^{10}$-$A^{11}$-$A^{12}$-$A^{13}$-$A^{14}$-$A^{15}$-$A^{16}$-$A^{17}$ (wherein the symbols have the same definitions as those given above),
(k) the formula $A^{11}$-$A^{12}$-$A^{13}$-$A^{14}$-$A^{15}$-$A^{16}$-$A^{17}$, wherein the symbols have the same definitions as those given above),
(l) the formula $A^{12}$-$A^{13}$-$A^{14}$-$A^{15}$-$A^{16}$-$A^{17}$ (wherein the symbols have the same definitions as those given above),
(m) the formula $A^{13}$-$A^{14}$-$A^{15}$-$A^{16}$-$A^{17}$ (wherein the symbols have the same definitions as those given above),
(n) the formula $A^{14}$-$A^{15}$-$A^{16}$-$A^{17}$ (wherein the symbols have the same definitions as those given above),

(o) the formula $A^{15}$-$A^{16}$-$A^{17}$ (wherein the symbols have the same definitions as those given above),
(p) the formula $A^{16}$-$A^{17}$ (wherein the symbols have the same definitions as those given above), or
(q) $A^{17}$ (wherein $A^{17}$ has the same definitions as that given above) which may,

$A^1$ described above represents an amino acid residue having a side chain which may be substituted, preferably an amino acid residue having an aromatic side chain, more preferably an L-amino acid residue having an aromatic side chain, and further more preferably L-tyrosine.
Each of $A^2$ and $A^3$ described above, whether identical or not, represents an amino acid residue having a side chains which may be substituted, preferably a neutral amino acid residue having a side chain which may be substituted, more preferably an L-neutral amino acid residue having a side chain which may be substituted, and further more preferably L-leucine for $A^2$ and L-valine for $A^3$.
$A^4$ described above represents an amino acid residue havinga side chain which may be substituted, preferably a neutral or a basic amino acid residue having a side chain which may be substituted, more preferably an L-neutral or an L-basic amino acid residue having a side chain which maybe substituted, and further more preferably L-lysine and $N^{\varepsilon}$-acetyllysine.
$A^5$ described above represents an amino acid residue having a side chain which may be substituted, preferably a neutral amino acid residue having a side chain which may be substituted, more preferably L-proline which may be substituted, and further more preferably L-proline. Each of $A^6$ and $A^9$ described above, whether identical or not, represents an amino acid residue having a side chain which may be substituted, preferably a basic amino acid residue having a side chain which may be substituted, more preferably an L-basic amino acid residue having a side chain which may be substituted, and further more preferably L-arginine.
$A^7$ and $A^{10}$ described above , whether identical or not, represents an amino acid residue having a side chain which may be substituted, and preferably an amino acid residue having a hydroxy group in a side chain thereof or a neutral amino acid residue having a side chain which may besubstituted.
For $A^7$, glycine which may besubstituted, especially glycine, is preferably used.
$A^8$ described above represents an amino acid residue having a side chain which may be substituted, preferably is an amino acid residue having L-proline or a hydroxy group in a side chain thereof, and more preferably L-serine, L-proline or oxyproline (hydroxyproline).
$A^{10}$ described above represents preferably an amino acid residue having a hydroxy group in a side chain thereof or a neutral amino acid residue having a side chain which may be substituted, and more preferably L-serine, L-threonine or L-asparagine.
$A^{11}$ through $A^{14}$ described above, whether identical or not, represents an amino acid residue having a side chain which may be substituted, preferably a neutral amino acid residue having a side chain which may be substituted, and more preferably glycine for $A^{11}$, L-proline for $A^{12}$, glycine for $A^{13}$, and L-alanine and L-proline for $A^{14}$.
$A^{15}$ described above represents an amino acid residue having a side chain which may be substituted, preferably an amino acid residue having an aromatic side chain, more preferably an L-amino acid residue having an aromatic side chain, and further more preferably L-tryptophan.
$A^{16}$ described above represents an amino acid residue having a side chain which may be substituted, preferably a neutral amino acid residue of which a side chain which may be substituted, more preferably a neutral L-amino acid residue having a carbamoyl group, and further more preferably L-glutamine.
A neutral L-amino acid residue having a carbamoyl group includes L-glutamine and L-asparagine.
$A^{17}$ represents an amino acid residue having a side chain which may be substituted, preferably a neutral amino acid residue having a side chain which may be substituted, more preferably a neutral L-amino acid residue having carbamoyl group, and further more preferably glycine.
Specific examples for $Y^2$ described above include:

① the formula $B^1$-$B^2$-$B^3$-$B^4$-$B^5$-$B^6$-$B^7$-$B^8$ where $B^1$ through $B^8$, whether identical or not, represents an amino acid having a side chain which may be substituted,
② the formula $B^2$-$B^3$-$B^4$-$B^5$-$B^6$-$B^7$-$B^8$ (wherein the symbols have the same definitions as given above),
③ the formula $B^3$-$B^4$-$B^5$-$B^6$-$B^7$-$B^8$ (wherein the symbols have the same definitions as given above),
④ the formula $B^4$-$B^5$-$B^6$-$B^7$-$B^8$ (wherein the symbols have the same definitions as given above),
⑤ the formula $B^5$-$B^6$-$B^7$-$B^8$ (wherein the symbols have the same definitions as given above),
⑥ the formula $B^6$-$B^7$-$B^8$ (wherein the symbols have the same definitions as given above),
⑦ the formula $B^7$-$B^8$ wherein each symbol has the same definition as given above; or
⑧ the formula $B^8$ where $B^8$ has the same meaning as described above.

$B^1$ represents an amino acid residue having a side chain which may be substituted, preferably a neutral amino acid residue having a side chain which may be substituted, more preferably a neutral L-amino acid residue having a side chain which may be substituted, further more preferably glycine which may be substituted, and most preferably glycine.

$B^2$ through $B^4$, whether identical or not, represents an amino acid residue having a side chain which may be substituted, preferably a basic amino acid residue having a side chain which may be substituted, more preferably basic L-amino acid residue having a side chain which may be substituted, further more preferably L-arginine for $B^2$, L-arginine for $B^3$, L- lysine for $B^4$.

$B^5$ described above represents an amino acid residue having a side chain which may be substituted, preferably an amino acid residue having an aromatic side chain, more preferably an L-amino acid residue having an aromatic side chain, and further more preferably L-phenylalanine.

Each of $B^6$ and $B^7$ described above, whether identical or not, represents amino acid residues having a side chain which may be substituted, preferably a basic amino acid residue having a side chain which may be substituted, more preferably a basic L-amino acid residue having a side chain which may be substituted, and further more preferably L-arginine.

$B^8$ described above represents an amino acid residue having a side chain which may be substituted, preferably glutamine havin a side chain which may be substituted, and more preferably L-glutamine.

Combinations of $Y^1$ and $Y^2$ include the cases that are represented by:

the formula expressed as (a) above + the formula expressed as ① above, that is the case where $X^1$ is represented by $A^1$-$A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$-$B^1$-$B^2$-$B^3$-$B^4$-$B^5$-$B^6$-$B^7$-$B^8$: the explanation will be omitted in the subsequent combinations),
the formula expressed as (a) above + the formula expressed as ② above,
the formula expressed as (a) above + the formula expressed as ③ above,
the formula expressed as (a) above + the formula expressed as ④ above,
the formula expressed as (a) above + the formula expressed as ⑤ above,
the formula expressed as (a) above + the formula expressed as ⑥ above,
the formula expressed as (a) above + the formula expressed as ⑦ above,
the formula expressed as (a) above + the formula expressed as ⑧ above,
the formula expressed as (b) above + the formula expressed as ① above,
the formula expressed as (b) above + the formula expressed as ② above,
the formula expressed as (b) above + the formula expressed as ③ above,
the formula expressed as (b) above + the formula expressed as ④ above,
the formula expressed as (b) above + the formula expressed as ⑤ above,
the formula expressed as (b) above + the formula expressed as ⑥ above,
the formula expressed as (b) above + the formula expressed as ⑦ above,
the formula expressed as (b) above + the formula expressed as ⑧ above,
the formula expressed as (c) above + the formula expressed as ① above,
the formula expressed as (c) above + the formula expressed as ② above,
the formula expressed as (c) above + the formula expressed as ③ above,
the formula expressed as (c) above + the formula expressed as ④ above,
the formula expressed as (c) above + the formula expressed as ⑤ above,
the formula expressed as (c) above + the formula expressed as ⑥ above,
the formula expressed as (c) above + the formula expressed as ⑦ above,
the formula expressed as (c) above + the formula expressed as ⑧ above,
the formula expressed as (d) above + the formula expressed as ① above,
the formula expressed as (d) above + the formula expressed as ② above,
the formula expressed as (d) above + the formula expressed as ③ above,
the formula expressed as (d) above + the formula expressed as ④ above,
the formula expressed as (d) above + the formula expressed as ⑤ above,
the formula expressed as (d) above + the formula expressed as ⑥ above,
the formula expressed as (d) above + the formula expressed as ⑦ above,
the formula expressed as (d) above + the formula expressed as ⑧ above,
the formula expressed as (e) above + the formula expressed as ① above,
the formula expressed as (e) above + the formula expressed as ② above,
the formula expressed as (e) above + the formula expressed as ③ above,
the formula expressed as (e) above + the formula expressed as ④ above,
the formula expressed as (e) above + the formula expressed as ⑤ above,
the formula expressed as (e) above + the formula expressed as ⑥ above,
the formula expressed as (e) above + the formula expressed as ⑦ above,

the formula expressed as (e) above + the formula expressed as ⑧ above,
the formula expressed as (f) above + the formula expressed as ① above,
the formula expressed as (f) above + the formula expressed as ② above,
the formula expressed as (f) above + the formula expressed as ③ above,
the formula expressed as (f) above + the formula expressed as ④ above,
the formula expressed as (f) above + the formula expressed as ⑤ above,
the formula expressed as (f) above + the formula expressed as ⑥ above,
the formula expressed as (f) above + the formula expressed as ⑦ above,
the formula expressed as (f) above + the formula expressed as ⑧ above,
the formula expressed as (g) above + the formula expressed as ① above,
the formula expressed as (g) above + the formula expressed as ② above,
the formula expressed as (g) above + the formula expressed as ③ above,
the formula expressed as (g) above + the formula expressed as ④ above,
the formula expressed as (g) above + the formula expressed as ⑤ above,
the formula expressed as (g) above + the formula expressed as ⑥ above,
the formula expressed as (g) above + the formula expressed as ⑦ above,
the formula expressed as (g) above + the formula expressed as ⑧ above,
the formula expressed as (h) above + the formula expressed as ① above,
the formula expressed as (h) above + the formula expressed as ② above,
the formula expressed as (h) above + the formula expressed as ③ above,
the formula expressed as (h) above + the formula expressed as ④ above,
the formula expressed as (h) above + the formula expressed as ⑤ above,
the formula expressed as (h) above + the formula expressed as ⑥ above,
the formula expressed as (h) above + the formula expressed as ⑦ above,
the formula expressed as (h) above + the formula expressed as ⑧ above,
the formula expressed as (i) above + the formula expressed as ① above,
the formula expressed as (i) above + the formula expressed as ② above,
the formula expressed as (i) above + the formula expressed as ③ above,
the formula expressed as (i) above + the formula expressed as ④ above,
the formula expressed as (i) above + the formula expressed as ⑤ above,
the formula expressed as (i) above + the formula expressed as ⑥ above,
the formula expressed as (i) above + the formula expressed as ⑦ above,
the formula expressed as (i) above + the formula expressed as ⑧ above,
the formula expressed as (j) above + the formula expressed as ① above,
the formula expressed as (j) above + the formula expressed as ② above,
the formula expressed as (j) above + the formula expressed as ③ above,
the formula expressed as (j) above + the formula expressed as ④ above,
the formula expressed as (j) above + the formula expressed as ⑤ above,
the formula expressed as (j) above + the formula expressed as ⑥ above,
the formula expressed as (j) above + the formula expressed as ⑦ above,
the formula expressed as (j) above + the formula expressed as ⑧ above,
the formula expressed as (k) above + the formula expressed as ① above,
the formula expressed as (k) above + the formula expressed as ② above,
the formula expressed as (k) above + the formula expressed as ③ above,
the formula expressed as (k) above + the formula expressed as ④ above,
the formula expressed as (k) above + the formula expressed as ⑤ above,
the formula expressed as (k) above + the formula expressed as ⑥ above,
the formula expressed as (k) above + the formula expressed as ⑦ above,
the formula expressed as (k) above + the formula expressed as ⑧ above,
the formula expressed as (l) above + the formula expressed as ① above,
the formula expressed as (l) above + the formula expressed as ② above,
the formula expressed as (l) above + the formula expressed as ③ above,
the formula expressed as (l) above + the formula expressed as ④ above,
the formula expressed as (l) above + the formula expressed as ⑤ above,
the formula expressed as (l) above + the formula expressed as ⑥ above,
the formula expressed as (l) above + the formula expressed as ⑦ above,
the formula expressed as (l) above + the formula expressed as ⑧ above,
the formula expressed as (m) above + the formula expressed as ① above,

the formula expressed as (m) above + the formula expressed as ② above,
the formula expressed as (m) above + the formula expressed as ③ above,
the formula expressed as (m) above + the formula expressed as ④ above,
the formula expressed as (m) above + the formula expressed as ⑤ above,
the formula expressed as (m) above + the formula expressed as ⑥ above,
the formula expressed as (m) above + the formula expressed as ⑦ above,
the formula expressed as (m) above + the formula expressed as ⑧ above,
the formula expressed as (n) above + the formula expressed as ① above,
the formula expressed as (n) above + the formula expressed as ② above,
the formula expressed as (n) above + the formula expressed as ③ above,
the formula expressed as (n) above + the formula expressed as ④ above,
the formula expressed as (n) above + the formula expressed as ⑤ above,
the formula expressed as (n) above + the formula expressed as ⑥ above,
the formula expressed as (n) above + the formula expressed as ⑦ above,
the formula expressed as (n) above + the formula expressed as ⑧ above,
the formula expressed as (o) above + the formula expressed as ① above,
the formula expressed as (o) above + the formula expressed as ② above,
the formula expressed as (o) above + the formula expressed as ③ above,
the formula expressed as (o) above + the formula expressed as ④ above,
the formula expressed as (o) above + the formula expressed as ⑤ above,
the formula expressed as (o) above + the formula expressed as ⑥ above,
the formula expressed as (o) above + the formula expressed as ⑦ above,
the formula expressed as (o) above + the formula expressed as ⑧ above,
the formula expressed as (p) above + the formula expressed as ① above,
the formula expressed as (p) above + the formula expressed as ② above,
the formula expressed as (p) above + the formula expressed as ③ above,
the formula expressed as (p) above + the formula expressed as ④ above,
the formula expressed as (p) above + the formula expressed as ⑤ above,
the formula expressed as (p) above + the formula expressed as ⑥ above,
the formula expressed as (p) above + the formula expressed as ⑦ above,
the formula expressed as (p) above + the formula expressed as ⑧ above,
the formula expressed as (q) above + the formula expressed as ① above,
the formula expressed as (q) above + the formula expressed as ② above,
the formula expressed as (q) above + the formula expressed as ③ above,
the formula expressed as (q) above + the formula expressed as ④ above,
the formula expressed as (q) above + the formula expressed as ⑤ above,
the formula expressed as (q) above + the formula expressed as ⑥ above,
the formula expressed as (q) above + the formula expressed as ⑦ above, and
the formula expressed as (q) above + the formula expressed as ⑧ above, and in particular, preferably
the formula expressed as (a) above + the formula expressed as ① above,
the formula expressed as (b) above + the formula expressed as ① above,
the formula expressed as (c) above + the formula expressed as ① above,
the formula expressed as (d) above + the formula expressed as ① above,
the formula expressed as (e) above + the formula expressed as ① above,
the formula expressed as (f) above + the formula expressed as ① above,
the formula expressed as (g) above + the formula expressed as ① above,
the formula expressed as (h) above + the formula expressed as ① above,
the formula expressed as (i) above + the formula expressed as ① above,
the formula expressed as (j) above + the formula expressed as ① above,
the formula expressed as (k) above + the formula expressed as ① above,
the formula expressed as (l) above + the formula expressed as ① above,
the formula expressed as (m) above + the formula expressed as ① above,
the formula expressed as (n) above + the formula expressed as ① above,
the formula expressed as (o) above + the formula expressed as ① above,
the formula expressed as (p) above + the formula expressed as ① above, and
the formula expressed as (q) above + the formula expressed as ① above.

More preferable examples, especially, include the cases that are represented by the formula expressed as (a) above + the formula expressed as ① above and the formula expressed as (b) above + the formula expressed as ① above.

Further more preferable examples for the cases that are represented by the formula expressed as (a) above + the formula expressed as ① above and the formula expressed as (b) above + the formula expressed as ① above are described as follows.

The case that represented by the formula expressed as (b) above + the formula expressed as ① above refers to the case where $X^1$ is represented by $A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$-$B^1$-$B^2$-$B^3$-$B^4$-$B^5$-$B^6$-$B^7$-$B^8$ where $A^2$ to $A^{17}$ and $B^1$ to $B^8$ have the same meanings as described above, for which preferable specific examples include:

Each of $A^2$ and $A^3$, whether identical or not, is an L-neutral amino acid residue having a side chain which may be, and preferably L-leucine for $A^2$ and L-valine for $A^3$;
$A^4$ is an L-neutral or an L-basic amino acid residue having a side chain which may be substituted, and preferably L-glutamine, L-lysine or $N^{å}$-acetyllysine;
$A^5$ is L-proline which may be substituted, and preferably L-proline;
Each of $A^6$ and $A^9$, whether identical or not, is an L-basic amino acid residue having a side chain which may be substituted, and preferably L-arginine;
$A^7$ is glycine which may be substituted, and preferably glycine;
$A^8$ is an amino acid residue with a side chain having L-proline or a hydroxy group, and preferably L-serine, L-proline or oxyproline (hydroxyproline);
$A^{10}$ is preferably an amino acid residue with a side chain having a hydroxy group or a neutral amino acid residue having a side chainwhich may be substituted, and preferably L-serine, L-threonine or L-asparagine;
$A^{11}$ is glycine, $A^{12}$ is L-proline, and $A^{13}$ is glycine, and $A^{14}$ is L-alanine or L-proline;
$A^{15}$ is an L-amino acid residue having aromatic side chains, and preferably L-tryptophan.
$A^{16}$ is a neutral L-amino acid residue having carbamoyl group, and preferably L-glutamine;
$A^{17}$ is a neutral L-amino acid residue, and preferably glycine;
$B^1$ is a neutral L-amino acid residue having a side chain which may be substituted, preferably glycine which may be substituted, and more preferably glycine;
$B^2$ thorough $B^4$, whether identical or not, is a basic L-amino acid residue having a side chain which may be, and preferably L-arginine for $B^2$, L-arginine for $B^3$, and L-lysine for $B^4$;
$B^5$ is an L-amino acid residue having an aromatic side chain, and preferably L-phenylalanine;
Each of $B^6$ and $B^7$, whether identical or not are basic L-amino acid residues of which side chains which may be substituted, and preferably L-arginine;
$B^8$ is glutamine which may be substituted, preferably L-glutamine.

The case that represented by the formula expressed as (a) above + the formula expressed as ① above refers to the case where $X^1$ is represented by $A^1$-$A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$-$B^1$-$B^2$-$B^3$-$B^4$-$B^5$-$B^6$-$B^7$-$B^8$ where $A^1$ to $A^{17}$ and B1 to $B^8$ have the same meanings as described above, for which preferable specific examples include:

$A^1$ is an L-amino acid residue having aromatic side chains, and preferably L-tyrosine;
Each of $A^2$ and $A^3$, whether identical or not, is an L-neutral amino acid residue having a side chainwhich may be substituted, and preferably L-leucine for $A^2$ and L-valine for $A^3$;
$A^4$ is an L-neutral or an L-basic amino acid residue of which a side chain is optionally substituted, and preferably L-glutamine, L-á-aminoadipic acid, L-lysine or $N^{\varepsilon}$-acetyllysine;
$A^5$ is L-proline which may be substituted, and preferably L-proline;
Each of $A^6$ and $A^9$, whether identical or not, is an L-basic amino acid residue having a side chains which may be substituted, and preferably L-arginine;
$A^7$ is glycine which may be substituted, and preferably glycine;
$A^8$ is an amino acid residue with a side chains having L-proline or a hydroxy group, and preferably L-serine, L-proline or oxyproline (hydroxyproline);
$A^{10}$ is preferably an amino acid residue with a side chain having a hydroxy group or a neutral amino acid residue of which a side chain is optionally substituted, and preferably L-serine, L-threonine or L-asparagine;
$A^{11}$ is glycine, $A^{12}$ is L-proline, and $A^{13}$ is glycine, and $A^{14}$ is L-alanine or L-proline;
$A^{15}$ is an L-amino acid residue having aromatic side chains, and preferably L-tryptophan.
$A^{16}$ is a neutral L-amino acid residue having carbamoyl group, and preferably L-glutamine;
$A^{17}$ is a neutral L-amino acid residue, and preferably glycine;
$B^1$ is a neutral L-amino acid residue of which a side chain is optionally substituted, preferably glycine which may be substituted, and more preferably glycine;

$B^2$ through $B^4$, whether identical or not, is a basic L-amino acid residue having a side chain which may be substituted, and preferably L-arginine for $B^2$, L-arginine for $B^3$, and L- lysine for $B^4$;

$B^5$ is an L-amino acid residue having aromatic side chains, and preferably L-phenylalanine;

Each of $B^6$ and $B^7$, whether identical or not, is a basic L-amino acid residue a side chain which may be substituted, and preferably L-arginine;

$B^8$ is glutamine which may be substituted, preferably L-glutamine.

Preferable specific examples for $X^1$ include:

(1) a hydrogen atom,
(2) Leu-Val-Gln-Pro-Arg-Gly-Ser-Arg-Asn-Gly-Pro-Gly-Pro-Trp-Gln-Gly-Gly-Arg-Arg-Lys-Phe-Arg-Arg-Gln,
(3) pGlu,
(4) Leu-Val-Adi($NH_2$)-Pro-Arg-Gly-Ser-Arg-Asn-Gly-Pro-Gly-Pro-Trp-Gln-Gly-Gly-Arg-Arg-Lys-Phe-Arg-Arg-Gln,
(5) Leu-Val-Lys(Ac)-Pro-Arg-Thr-Ser-Arg-Thr-Gly-Pro-Gly-Ala-Trp-Gln-Gly-Gly-Arg-Arg-Lys-Phe-Arg-Arg-Gln,
(6) Tyr-Leu-Val-Lys-Pro-Arg-Thr-Ser-Arg-Thr-Gly-Pro-Gly-Ala-Trp-Gln-Gly-Gly-Arg-Arg-Lys-Phe-Arg-Arg-Gln, and
(7) Z-pGlu.

In this specification, $X^2$ represents a neutral amino acid residue having a side chain which may be substituted, preferably L-leucine having a side chain which may be substituted, or L-norleucine having a side chain which may be substituted, and more preferably L-leucine or L-norleucine.

In this specification, $X^3$ represents a neutral amino acid residue having a side chain which may be substituted or a basic amino acid residue having a side chain which may be substituted. Substituents for a side chain of a basic amino acid residue include a $C_{1-4}$ acyl group, a tosyl group and a $C_{1-6}$ alkyl group.

A $C_{1-4}$ acyl group includes formyl including formyl, acetyl, propionyl and butyryl, and $C_{2-4}$ alkanoyl.

A $C_{1-6}$ alkyl group includes methyl, ethyl, propyl, isopropyl, butyl, isobutyl, sec-butyl, tert-butyl, pentyl, and hexyl.

$X^3$ preferably includes L-lysine havinga side chain which may be substituted, L-norleucine having a side chain which may be substituted, and L-arginine having a side chain which may be substituted; more preferably L-lysine, L-norleucine or L-arginine, each of which side chain which may be substituted by a $C_{1-4}$ acyl group that includes formyl including formyl, acetyl, propionyl and butyryl, and $C_{2-4}$ alkanoyl, a $C_{1-6}$ alkyl group that includes methyl, ethyl, propyl, isopropyl, butyl, isobutyl, sec-butyl, tert-butyl, pentyl, and hexyl, or a tosyl group; and further more preferably L-lysine, L-norleucine, L-arginine, $N^{\varepsilon}$-acetyllysine. $N^{\varepsilon}$-methyllysine. $N^{\varepsilon}$-tosyllysine and $N^{\varepsilon}$-tosyl arginine.

In this specification, $X^4$ represents a neutral or an aromatic amino acid residue having a bond or a side chain which may be substituted or a basic amino acid residue having a side chain which may be substituted. Preferably $X^4$ includes L-norleucine having a bond or a side chain which may be substituted, L-methionine having a side chain which may be substituted, L-methioninesulfoxide havinga side chain which may be substituted, or L-alanine havinga side chain which may be substituted, and more preferably a bond, L-norleucine or L-methionine, L-methioninesulfoxide, and L-cyclohexylalanine.

In this specification, $X^5$ represents ① an amino acid residue having a side chain which may be substituted or an amino acid derivative with the C-terminal carboxyl group reduced to a hydroxymethyl group or a formyl group, ② a hydroxyl group or ③ a dipeptide chain comprising an amino acid residue having a side chain which may be substituted and an amino acid residue having a side chain which may be substituted, bound together, or a peptide derivative with the C-terminal carboxyl group reduced to a hydroxymethyl group or a formyl group.

Preferably, $X^5$ represents ① a neutral amino acid residue having a side chain which may be substituted or an amino acid derivative with the C-terminal carboxyl group reduced to a hydroxymethyl group or a formyl group, ② a hydroxyl group or ③ a dipeptide chain comprising a neutral amino acid residue having an aromatic side chain which may be substituted and an amino acid residue having a side chain which may be substituted, bound together, or a peptide derivative with the C-terminal carboxyl group reduced to a hydroxymethyl group or a formyl group.

More preferably, $X^5$ represents ① L-proline havinga side chain which may be substituted or an amino acid derivative having the C-terminal carboxyl group reduced to a hydroxymethyl group or a formyl group, ② 4-chlorophenylalanine having a side chain which may be substituted or an amino acid derivative having the C-terminal carboxyl group reduced to a hydroxymethyl group or a formyl group, ③ 2-naphthylalanine having a side chain which may be substituted or an amino acid derivative with the C-terminal carboxyl group reduced to a hydroxymethyl group or a formyl group, ④ cyclohexylalanine having a side chain which may be substituted or an amino acid derivative with the C-terminal carboxyl group reduced to a hydroxymethyl group or a formyl group, ⑤ a hydroxyl group, or ⑥ a dipeptide chain formed by binding together L-proline which may be substituted and (a) L-phenylalanine having a side chain which may be substituted, (b) L-tyrosine o having a side chain which may be substituted, (c) L-2-thienylalanine having a side chain which may be substituted, (d) L-phenylglycine having a side chain which may be substituted or (e) L-2-pyridylalanine having a side chain which may be substituted, or a peptide derivative with the C-terminal carboxyl group reduced to a hydroxymethyl group or a formyl group.

Further more preferably, $X^5$ represents ① L-proline or an amino acid derivative with the C-terminal carboxyl group reduced to a hydroxymethyl group or a formyl group, ② 4-chlorophenylalanine or an amino acid derivative with the C-terminal carboxyl group reduced to a hydroxymethyl group or a formyl group, ③ 2-naphthylalanine or an amino acid derivative with the C-terminal carboxyl group reduced to a hydroxymethyl group or a formyl group, ④ cyclohexylalanine or an amino acid derivative with the C-terminal carboxyl group reduced to a hydroxymethyl group or a formyl group, ⑤ a hydroxyl group, or ⑤ a dipeptide chain formed by binding together L-proline and L-phenylalanine, or a peptide derivative with the C-terminal carboxyl group reduced to a hydroxymethyl group or a formyl group, ⑦ a dipeptide chain formed by binding together L-proline and L-tyrosine, or a peptide derivative with the C-terminal carboxyl group reduced to a hydroxymethyl group or a formyl group, ⑧ a dipeptide chain formed by binding together L-proline and L-2-thienylalanine, or a peptide derivative with the C-terminal carboxyl group reduced to a hydroxymethyl group or a formyl group, ⑨ a dipeptide chain formed by binding together L-proline and L-phenylglycine, or a peptide derivative with the C-terminal carboxyl group reduced to a hydroxymethyl group or a formyl group, ⑩ a dipeptide chain formed by binding together L-proline and 4-chlorophenylalanine, or a peptide derivative with the C-terminal carboxyl group reduced to a hydroxymethyl group or a formyl group, ⑪ a dipeptide chain formed by binding together L-proline and 2-naphthylalanine, or a peptide derivative with the C-terminal carboxyl group reduced to a hydroxymethyl group or a formyl group, ⑫ a dipeptide chain formed by binding together L-proline and 3-iodotyrosine, or a peptide derivative with the C-terminal carboxyl group reduced to a hydroxymethyl group or a formyl group, ⑬ a dipeptide chain formed by binding together L-proline and O-methyltyrosine, or a peptide derivative with the C-terminal carboxyl group reduced to a hydroxymethyl group or a formyl group, ⑭ a dipeptide chain formed by binding together L-proline and L-2-pyridylalanine, or a peptide derivative with the C-terminal carboxyl group reduced to a hydroxymethyl group or a formyl group.

Most preferably, "$-X^4-X^5$" includes:

(1) -Nle-Pro-Phe,
(2) -Nle-Pro-Tyr,
(3) -Nle-Pro,
(4) -Nle,
(5) -Met-Pro-Phe,
(6) -Nle-Pro-Thi,
(7) -Nle-Pro-Phg,
(8) -Nle-Pro-Pya(2),
(9) -Met(O),
(10) -Met-Phe(Cl)
(11) -Met-Pro-Phe(Cl),
(12) -Met-Pro-Nal(2),
(13) -Met-Nal(2),
(14) -Met-Cha,
(15) -Cha-Pro-Phe,
(16) -Cha,
(17) -Met-Pro-Tyr(I), and
(18) -Met-Pro-Tyr(Me).

Specific examples for the peptides of the present invention include:

(1) Leu-Val-Gln-Pro-Arg-Gly-Ser-Arg-Asn-Gly-Pro-Gly-Pro-Trp-Gln-Gly-Gly-Arg-Arg-Lys-Phe-Arg-Arg-Gln-Arg-Pro-Arg-Leu-Ser-His-Lys-Gly-Pro-Nle-Pro-Phe,
(2) Leu-Val-Gln-Pro-Arg-Gly-Ser-Arg-Asn-Gly-Pro-Gly-Pro-Trp-Gln-Gly-Gly-Arg-Arg-Lys-Phe-Arg-Arg-Gln-Arg-Pro-Arg-Leu-Ser-His-Lys-Gly-Pro-Nle-Pro-Tyr,
(3) pGlu-Arg-Pro-Arg-Leu-Ser-His-Lys-Gly-Pro-Nle-Pro-Phe,
(4) pGlu-Arg-Pro-Arg-Leu-Ser-His-Lys-Gly-Pro-Nle-Pro-Tyr,
(5) pGlu-Arg-Pro-Arg-Leu-Ser-His-Lys-Gly-Pro-Nle-Pro,
(6) pGlu-Arg-Pro-Arg-Leu-Ser-His-Lys-Gly-Pro-Nle,
(7) Ac-Arg-Pro-Arg-Leu-Ser-His-Lys-Gly-Pro-Nle-Pro-Tyr,
(8) Ac-Arg-Pro-Arg-Leu-Ser-His-Lys-Gly-Pro-Nle-Pro,

(9) Ac-Arg-Pro-Arg-Leu-Ser-His-Lys-Gly-Pro-Nle,
(10) pGlu-Arg-Pro-Arg-Leu-Ser-His-Lys(Ac)-Gly-Pro-Met-Pro-Phe.
(11) pGlu-Arg-Pro-Arg-Leu-Ser-His-Lys(Me)-Gly-Pro-Met-Pro-Phe.
(12) pGlu-Arg-Pro-Arg-Leu-Ser-His-Lys(Ac)-Gly-Pro-Nle-Pro-Phe,
(13) pGlu-Arg-Pro-Arg-Leu-Ser-His-Lys(Me)-Gly-Pro-Nle-Pro-Phe,
(14) pGlu-Arg-Pro-Arg-Leu-Ser-His-Lys(Tos)-Gly-Pro-Nle-Pro-Phe,
(15) pGlu-Arg-Pro-Arg-Leu-Ser-His-Arg(Tos)-Gly-Pro-Nle-Pro-Phe,
(16) pGlu-Arg-Pro-Arg-Nle-Ser-His-Lys-Gly-Pro-Nle-Pro-Phe,
(17) pGlu-Arg-Pro-Arg-Nle-Ser-His-Lys-Gly-Pro-Nle-Pro-Tyr,
(18) pGlu-Arg-Pro-Arg-Leu-Ser-His-Lys-Gly-Pro-Nle-Pro-Thi,
(19) pGlu-Arg-Pro-Arg-Leu-Ser-His-Lys-Gly-Pro-Nle-Pro-Phg,
(20) pGlu-Arg-Pro-Arg-Leu-Ser-His-Lys-Gly-Pro-Nle-Pro-Pya(2),
(21) Arg-Pro-Arg-Leu-Ser-His-Lys-Gly-Pro-Nle-Pro-Tyr,
(22) Leu-Val-Adi($NH_2$)-Pro-Arg-Gly-Ser-Arg-Asn-Gly-Pro-Gly-Pro-Trp-Gln-Gly-Gly-Arg-Arg-Lys-Phe-Arg-Arg-Gln-Arg-Pro-Arg-Leu-Ser-His-Lys-Gly-Pro-Nle-Pro-Phe,
(23) Leu-Val-Lys(Ac)-Pro-Arg-Thr-Ser-Arg-Thr-Gly-Pro-Gly-Ala-Trp-Gln-Gly-Gly-Arg-Arg-Lys-Phe-Arg-Arg-Gln-Arg-Pro-Arg-Leu-Ser-His-Lys-Gly-Pro-Nle-Pro-Tyr,
(24) Tyr-Leu-Val-Lys-Pro-Arg-Thr-Ser-Arg-Thr-Gly-Pro-Gly-Ala-Trp-Gln-Gly-Gly-Arg-Arg-Lys-Phe-Arg-Arg-Gln-Arg-Pro-Arg-Leu-Ser-His-Lys-Gly-Pro-Nle-Pro-Phe,
(25) Z-pGlu-Arg-Pro-Arg-Leu-Ser-His-Lys(Ac)-Gly-Pro-Nle-Pro-Phe,
(26) Arg-Arg-Gln-Arg-Pro-Arg-Leu-Ser-His-Lys-Gly-Pro-Met(O),
(27) Arg-Arg-Gln-Arg-Pro-Arg-Leu-Ser-His-Lys-Gly-Pro-Nle-Pro-Tyr,
(28) pGlu-Arg-Pro-Arg-Leu-Ser-His-Lys-Gly-Pro-Met-Phe(Cl).
(29) pGlu-Arg-Pro-Arg-Leu-Ser-His-Lys-Gly-Pro-Met-Pro-Phe(Cl),
(30) Arg-Pro-Arg-Leu-Ser-His-Lys-Gly-Pro-Met-Pro-Nal(2),
(31) Arg-Pro-Arg-Leu-Ser-His-Lys-Gly-Pro-Met-Nal(2),
(32) Arg-Pro-Arg-Leu-Ser-His-Lys-Gly-Pro-Met-Pro-Phe(Cl),
(33) Arg-Pro-Arg-Leu-Ser-His-Lys-Gly-Pro-Met-Phe(Cl),
(34) Arg-Pro-Arg-Leu-Ser-His-Lys-Gly-Pro-Met-Cha,
(35) pGlu-Arg-Pro-Arg-Leu-Ser-His-Lys-Gly-Pro-Cha-Pro-Phe,
(36) Arg-Arg-Gln-Arg-Pro-Arg-Leu-Ser-His-Lys-Gly-Pro-Cha,
(37) Arg-Arg-Gln-Arg-Pro-Arg-Leu-Ser-His-Lys-Gly-Pro-Met-Pro-Phe(Cl),
(38) Arg-Arg-Gln-Arg-Pro-Arg-Leu-Ser-His-Nle-Gly-Pro-Met-Pro-Phe(Cl),
(39) Arg-Arg-Gln-Arg-Pro-Arg-Leu-Ser-His-Nle-Gly-Pro-Met-Pro-Tyr(I),
(40) Arg-Arg-Gln-Arg-Pro-Arg-Leu-Ser-His-Nle-Gly-Pro-Met-Pro-Tyr(Me)

Salts of the peptides of the present invention, may be those formed with physiologically acceptable bases such as alkaline metals, or acids (organic or inorganic acids), physiologically acceptable acid addition salts are especially preferable. These salts include salts with inorganic acids (e.g. hydrochloric. acid, phosphoric acid, hydrobromic acid, and sulfuric acid), or salts with organic acids (e.g. acetic acid, formic acid, propionic acid, fumaric acid, maleic acid, succinic acid, tartaric acid, citric acid, malic acid, oxalic acid, benzoic acid, methanesulfonic acid and benzenesulfonic acid).

Peptides of the present invention can be produced by modifying them following the peptide synthesis method described later after obtaining natural ligands from tissues or cells of humans or other warm-blooded animals through a peptide refining process. Alternatively, these peptides can also be produced not with the a natural ligand as a raw material but by following the peptide synthesis method described later.

In the case where natural ligands are produced from tissues or cells of humans or other warm-blooded animals, they can be refined and isolated by first homogenizing the aforementioned tissues or cells, extracting with acids, and then by processing the resultant extracts using a combination of chromatographies such as salting-out, dialysis, gel-filtration, reverse phase chromatography, ion exchange chromatography, and affinity chromatography.

Natural ligands can be obtained, for example, through the process in accordance with the processes described in WO 99/33976 (Japanese Patent Application No. 220853-1998).

Peptides of the present invention can be produced through known processes for peptide synthesis. For example, both solid and liquid phase synthesis methods may be used for peptide synthesis. The targeted peptide can be produced by condensing a partial peptide or an amino acid that may form the peptide of the present invention and the residual parts are condensed, and then by eliminating the protecting group if the product possesses one. Known methods of condensation or elimination of protecting groups are listed below:

1. M. Bodanszky and M.A. Ondetti, *Peptide Syntheisis*, Interscience Publishers, New York, 1966.
2. Schoroeder and Luebke, *The Peptide,* Academic Press, New York, 1965.
3. Nobuo Izumiya et al., *Fundamental and Experiments of Peptide Synthesis*, MARUZEN CO., LTD., 1975.
4. Haruaki Yajima and Shunpei Sakakibara, Lecture Series of the Biochemical Experiments 1, Chemistry of Proteins IV, 205, 1977
5. Haruaki Yajima (Edited), Development of Pharmaceutical Products (Sequel) Vol. 14, *Peptide Syntheisis*, Hirokawa Shoten

After this reaction, a combination of conventional purification processes, such as solvent extraction, distillation, column chromatography, liquid chromatography or re-crystallization, can be used to refine and isolate peptides of the present invention. If the peptide obtained through the aforementioned process is a free radical, it can be transformed into appropriate salts through known processes, and if it is a salt, it can be transformed into a free radical with a known process.

To condense protected amino acids or peptides, various activating reagents for peptide synthesis can be used, but trisphosphoniums, tetramethyluroniums, and carbodimides are especially suitable. Trisphosphoniums include benzotriazol-1-yl-oxytris(pyrrolydino)phosphonium hexafluorophosphate (PyBOP), and bromotris(pyrrolydino) phosphonium hexafluorophosphate(PyBroP). Tetramethyluroniums include 2-(1H-benzotriazol-1-yl)-1,1,3,3-tetramethyluronium-tetrafluoroborate, 2-(5-norbornene-2,3-dicarboximido)-1,1,3,3-tetramethyluronium tetrafluoroborate, and O-(N-succinimidyl)-1,1,3,3-tetramethyluronium terafluoroborate.

Carbodimides include DCC, N,N'-diisopropylcarbodimide, and N-ethyl-N'-(3-dimethylaminopropyl)carbodimide. Addition of racemization inhibitors, such as HONB, HOBt, or HOOBt, is preferable for such condensation processes. Solvents used for condensation are selected from those known to be used in peptide condensation reactions.

For example, acid amides such as anhydrous or hydrous N, N-dimethylformamide, N, N-dimethylacetamide and N-methylpyrrolidone, halogenated hydrocarbons such as methylene chloride and chloroform, alcohols such as trifluoroethanol, sulfoxides such as dimethyl sulfoxide, tertiary amine such as pyridine, ethers such as dioxane and tetrahydrofuran, nitriles such as acetonitrile and propionitrile, esters such as ethyl acetate and methyl acetate, or appropriate mixture of these are used.

Reaction temperature may be selected from a known range for peptide bonding reactions, and is normally between -20°C and 50°C. Activated amino acid derivatives are normally used in excess quantities of 1.5 to 4 times. In solid phase synthesis, sufficient condensation can be obtained by repeating condensation reactions without eliminating the protecting groups if condensation is shown to be incomplete as a result of ninhydrin reaction-based tests. If satisfactory condensation cannot be obtained through the repetition of reactions, unreacted amino acids can be acetylized with acetic anhydrides or acetylmidazole, so that later reactions will not affected.

Protecting groups for an amino group of a raw material amino acid include Z, Boc, tert-pentyloxycarbonyl, isobornyl oxycarbonyl, 4-methoxybenzyloxycarbonyl, Cl-Z, Br-Z, adamantyloxycarbonyl, trifluoroacetyl, phthaloyl, formyl, 2-nitrophenylsulfenyl, diphenylphosphinothioyl and Fmoc. Protecting groups for a carboxyl group include, for example, $C_{1-6}$ alkyl group, $C_{3-8}$ cycloalkyl group, $C_{7-14}$ aralkyl described above as R, as well as, allyl, 2-adamantyl, 4-nitrobenzyl, 4-methoxybenzyl, 4-chlorobenzyl, phenacyl group, benzyloxycarbonylhydrazide, tertiary butoxycarbonylhydrazide and tritylhydrazide.

Hydroxyl groups of serine and threonine may be protected by esterification or etherification, for instance. Groups suitable for such esterification include lower ($C_{2-4}$) alkanoyl groups such as an acetyl group and a group introduced from organic acids including aroyl groups such as benzoyl group. Groups suitable for such etherification include a benzyl group, a tetra-hydropyranyl group, a tertiary-butyl group and a trityl group (Trt).

Protecting groups for a phenolic hydroxyl group of tyrosine include Bzl, 2,6-dichlorobenzyl, 2-nitrobenzyl, Br-Z and tertiary-butyl.

Protecting groups for imidazolyl of histidine include Tos, 4-methoxy-2,3,6-trimethylbenzenesulfonyl (Mtr), DNP, Bom, Bum, Boc, Trt and Fmoc.

Protecting groups for a guanidino group of arginine include Tos, Z, 4-methoxy-2,3,6-trimethylbenzenesulfonyl (Mtr), p-methoxybenzene sulfonyl (MBS), 2,2,5,7,8-Pentamethyl chroman-6-sulfonyl (Pmc), mesitylene-2-sulfonyl (Mts), 2,2,4,6,7-pentamethyldihydrobenzofuran-5-sulfony (Pbf), Boc, Z and $NO_2$.

Protecting groups for a side chain amino group of lysine include Z, Cl-Z, trifluoroacetyl, Boc, Fmoc, Trt, Mtr and 4,4-dimethyl-2,6-dioxo cyclohexylideneyl (Dde).

Indolyl protecting groups for tryptophan include formyl (For), Z, Boc, Mts and Mtr.

Protecting groups for asparagines and glutamine include Trt, xanthyl (Xan), 4,4'-dimethoxy benzhydryl (Mbh) and 2,4,6-trimethoxybenzyl (Tmob).

Activated carboxyl groups of raw materials include, for example, corresponding acid anhydrides, azide, activated esters [alcohols (for example, ester with pentachlorophenol, 2,4,5-trichlorophenol, 2,4-dinitrophenol, cyanomethyl alcohol, paranitrophenol, HONB, N-hydroxysuximide, and 1-hydroxybenzotriazole (HOBt))]. Activated amino groups of raw materials include, for example, corresponding phosphorous-amide.

Elimination of protecting groups can be achieved by the procedures including catalytic reduction in flowing hydrogen in the presence of catalyst such as palladium black and palladium on carbon; acid treatment with anhydrous hydrogen fluoride, methanesulfonic acid, trifluoromethanesulfonic acid, trifluoroacetic acid, bromotrimethylsilane/trimethylsilyl bromide (TMSBr), trimethylsilyl trifluoromethanesulfonate, tetrafluoroboric acid, tris(trifluoro)boron, boron tribromide, or mixture of the above; base treatment with diisopropylethylamine, triethylamine, piperidine, or piperazine; and reduction by using sodium in liquid ammonia.

While the elimination reaction in the acid treatment is usually performed at a temparature range -20 to 40°C addition of cation scavengers such as anisole, phenol, thioanisole, metacresol, and paracresol; and of dimethylsulfide, 1,4-butanedithiol, or 1,2-ethanedithiol are useful. 2,4-dinitrophenyl group used as an imidazolyl protecting group for histidine can be eliminated by treatment with thiophenol, and a formyl group used as an indole protecting group for tryptophan can be eliminated by base treatment with dilute sodium hydroxide or dilute ammonia, other than the deprotection with acid treatment in the presence of 1,2-ethanedithiol or 1,4-butanedithiol.

Protection of the function group that should not be involved in reactions of raw materials and the protecting group, as well as elimination of the protecting group and activation of the function group involved in the reaction can be appropriately selected from known groups or known procedures.

The peptide amide can be obtained by solid phase synthesis using resins for synthesizing amide forms or amidating α-carboxyl group of an amino acid at the carboxyl terminal, elongating the peptide chain towards the amino group side for a desired length, producing a peptide deprived of only the protecting group of an α-amino group at the N terminal of said peptide chain and a peptide (or amino acid) deprived of only the protecting group of carboxyl group at the N terminal of said peptide chains and by condensing the both peptides are in a mixed solvent above. The details of the condensation reaction are the same as the above. After the protected peptide obtained by condensation is purified, all protecting groups are eliminated to obtain desired crude polypeptides. These crude peptides may be purified by deploying any of the various known procedures of purification and major fractions are lyophilized to obtain desired peptide amide.

To obtain the peptide ester, after condensing the α-carboxyl group of an amino acid at the carboxyl terminal with desired alcohols to obtain amino acid ester, desired peptide ester can be obtained in a similar way to that for the peptide amide.

The peptides of the present invention may be fusion proteins that are bound together with proteins of well-known functions or properties.

The peptides of the present invention may be used for ① research for physiological activities of the peptides of the present invention, ② development of receptor binding assay system using the expression system of recombinant receptor proteins and screening of candidate compounds for potent pharmaceutical products and ③ development of pharmaceuticals such as a central nervous function regulator, a circulatory function regulator, a cardiac function regulator, an immune function regulator, a digestive function regulator, a metabolic function regulator or a reproductive organ function regulator.

In particular, since screening of specific G protein-coupled receptor protein agonists or antagonists of warm-blooded animals including humans can be performed by the receptor binding assay system using the expression system of recombinant G protein-coupled receptor proteins described later, said agonists or antagonists can be used as prophylactic or therapeutic drugs.

Furthermore, regarding ③ above, the peptides of the present invention are useful as a safe medicine with low toxicity since these peptides are identified as ligands by G protein-coupled receptor proteins expressed in the central nervous system, the circulatory system, the heart, immune system, digestive system, metabolic system, or the reproductive system. Since the peptides of the present invention are involved in activities of central nervous functions, circulatory functions, cardiac functions, immune functions, digestive functions, metabolic functions, or reproductive organ functions, they can be used as therapeutic or prophylactic drugs against disorders, such as: dementia including dementia associated with senile dementia, cerebrovascular dementia, dementia associated with retroplastic diseases of the systemic degeneration type (e.g. Alzheimer's disease, Parkinson's disease, Pick's disease, Huntington's disease), dementia associated with infective diseases (e.g. slow virus infections such as Creutzfeldt-Jakob disease), dementia associated with endocrine disease, metabolic disease or toxic disease (e.g. hypothyroidism, vitamin B12 deficiency disease, alcoholism, and intoxication with various drugs, metal or organic compound), dementia associated with neoplastic diseases such (e.g. brain tumor), and dementia associated with traumatopathy (e.g. chronic subdural hematoma); depression, ADHD (minimal brain dysfunction) syndrome; disturbance of consciousness; anxiety disorder; schizophrenia; phobia, growth hormone secretion disorder (e.g. gigantism, acromegaly), hyperphagia, polyphagia, hypercholesterolemia, hyperglyceridemia, hyperlipidemia, hyperprolactinemia, diabetes (e.g. diabetic complication, diabetic nephropathy, diabetic neuropathy and diabetic retinopathy); cancer (e.g. breast cancer, lymphatic leukemia, lung cancer, bladder cancer, ovarian cancer and prostate cancer); pancreatitis, renal diseases (e.g. chronic renal failure, nephritis); Turner's syndrome; neuropathy; rheumatoid arthritis; spinal cord injury; transient ischemic attack; amyotrophic lateral sclerosis; acute myocardial infarct; spinocerebellar degeneration; fracture; injury; atopic dermatitis; osteoporosis; asthma; epilepsy; infertility; arteriosclerosis; emphysema pulmonum; pulmonary edema or hypogalactia. Also they can be applied as an agent that improves the nutritional state and a vasopressor that are used post-operatively.

In addition, the peptides of the present invention can be used as therapeutic or prophylactic drugs against AIDS (Acquired Immune Deficiency Syndrome) or the like.

When the peptides of the present invention are used as the pharmaceuticals described above, they can be used following conventional methods. For example, they may be given orally as tablets sugar-coated or enteric coated as necessary, capsules, elixirs or microcapsules, or may be given parenterally in the form of injections such as aseptic solution with water or other pharmaceutically acceptable liquid, or suspensions. For example, they may be manufactured by mixing said compounds or their salts with physiologically acceptable carriers, flavors, excipients, vehicles, preservatives, stabilizers and binders with a unit dose form required in commonly accepted manufacturing practices. The content of the active ingredient in these preparations should be determined based on the appropriate dose within the indicated range.

Additives that may be mixed into tablets or capsules include, for example, binders such as gelatin, corn starch, gum traganth and gum Arabic, excipients such as crystalline cellulose, fillers such as corn starch, gelatin, alginic acid, lubricants such as magnesium stearate, sweetening agent such as cane sugar, lactose and saccharin, and flavors such as peppermint, Gaultheria ovatifolia essence and cherry. When the preparation unit form is a capsule, liquid carriers such as oils may be contained in addition to the above-mentioned types of materials. Aseptic compositions for injections can be prepared following a usual manufacturing practice where active substances, or naturally occurring vegetable oils such as sesame oil and coconut oil in vehicles such as water for injections are dissolved or suspended.

Aqueous solutions for injection include, for example, physiologic saline, isotonic solutions containing glucose and other adjuvant (e.g. D-sorbitol, D-mannitol, sodium chloride) and may be used in combination with appropriate solubilizers including alcohol (e.g. ethanol), polyalcohol (e.g. polypropylene glycol and polyethylene glycol), nonionic surfactant (e.g. Polysorbate 80™ and HCO-50). Oily liquids include sesame oil and soybean oil, and may be used in combination with benzyl benzoate or benzyl alcohol as a solubilizer.

They may be used in combination with buffers (e.g. phosphate buffer and sodium acetate), soothing agents (e.g. benzalkonium chloride and procaine hydrochloride), stabilizers (e.g. human serum albumin and polyethylene glycol), preservatives (e.g. benzyl alcohol and phenol) and antioxidants. Preparation for injection is usually filled into appropriate ampules. Pharmaceuticals produced in such ways can be administered, for example, to human or mammals (e.g. mice, rats, guinea pigs, rabbits, sheep, pigs, cattle, cats, dogs, monkeys, hamadryas baboon and chimpanzees), because of their safety and low toxicity.

The dose of the peptides of the present invention for oral administration is generally approximately 0.1 to 100 mg/day, preferably approximately 1.0 to 50 mg/day, or more preferably approximately 1.0 to 20 m/day for adult patients of 60 kg, varying although depending on symptoms. For parenteral administration, although the dose varies depending on subjects, target organs, symptoms, or administration methods, for example, it is advantageous in the form of injections that they are administered intravenously at doses of approximately 0.01 to 30 mg/day, preferably approximately 0.1 to 20 mg/day, and more preferably approximately 0.1 to 10 mg/day for adult patients with emphysema pulmonum of the body weight of 60 kg. For other animals, they can be administered at doses that are equivalent to those values with 60 kg.

G protein-coupled receptor proteins corresponding to the above-mentioned peptides of the present invention are G protein-coupled receptor proteins derived from any tissues (e.g. hypophysis, pancreas, brain, kidney, liver, genital gland, thyroid, gallbladder, bone marrow, adrenal, skin, muscle, lung, gastrointestinal tract, blood vessel, and heart) or cells of human or mammals such as mammalian warm-blooded animals (e.g. rabbits, sheep, goats, rats, mice, guinea pigs, cattle, horses, and pigs) and birds (e.g. chickens, doves, ducks, geese, quails), and may be anything if it contains the same or practically the same amino acid sequence as the amino acid sequence represented by the Sequence ID No.:26. In other words, G protein-coupled receptor proteins contain, in addition to proteins containing the amino acid sequence represented by the Sequence ID No.:26 in this specification, proteins that contain the amino acid sequences having approximately 90 to 99.9% homology with the amino acid sequence represented by the Sequence ID No.:26 in this specification, and include a protein with practically equivalent activities as those that contain the amino acid sequence represented by the Sequence ID No.:26 in this specification.

Activities shown by these proteins include, for example, ligand binding activities and signal transduction activities. "Practically equivalent" means that ligand binding activities are qualitatively equal. Therefore, quantitative factors such as the strength of ligand binding activities or factors such as molecular weights of receptor proteins may be different.

Additionally, G protein-coupled receptor proteins include those of which Met at the N terminal is protected by protecting groups (e.g. $C_{1-6}$ acyl group including $C_{2-6}$ alkanoyl group such as formyl and acetyl), those of which the N terminal side of Gln is cleaved in vivo and said Gln pyroglutamated, those of which a side chain of an amino acid residue in the molecule is protected by appropriate protecting groups (e.g. $C_{1-6}$ acyl group such as formyl group and acetyl group), and conjugated proteins that include so-called glycoproteins formed by linking saccharide chains.

Salts of G protein-coupled receptor proteins include those that are similar to salts of the peptides described above.

G protein-coupled receptor proteins, their salts or partial peptides can be produced from tissues or cells of human or warm-blooded animals by using known purification methods of proteins, or following known peptide synthesis methods described above.

For partial peptides of G protein-coupled receptor proteins, for example, a site exposed to the outside of the cell membrane among molecules of G protein-coupled receptor proteins may be used. In other words, they are peptides containing the portion that has been analyzed to be an extracellular region (hydrophilic site) by the hydrophobic plot analysis of G protein-coupled receptor proteins. Similarly, peptides containing a hydrophobic site may also be used. Peptides that contain each domain individually or partial peptides that contain multiple domains at the same time.

As for the salts of partial peptides of G protein-coupled receptor proteins, those similar to salts of the peptides described above are used.

DNAs that code G protein-coupled receptor proteins may be any of those that contain the base sequence that codes G protein-coupled receptor proteins containing the same or practically the same amino acid sequence as the amino acid sequence of the Sequence ID No.:26 in this specification. They may also be either of genomic DNAs, genomic DNA library, cDNAs derived from tissues or cells, cDNA library derived from tissues or cells, or synthesized DNAs. Vectors used for library may be bacteriophage, plasmid, cosmid, or phagemid. They may be amplified directly by known RT-PCR method using the part prepared from RNA fractions of tissues or cells.

More specifically, as DNAs that code G protein-coupled receptor proteins containing the amino acid sequence represented by the Sequence ID No.:26 in this specification, DNA containing the base sequence represented by the Sequence ID No.:27 in this specification is used.

Here, the use of the peptides or their salts in the present invention (hereinafter may be abbreviated as the peptides or the like of the present invention) is specifically described as follows:

(1) Prophylactic or therapeutic drugs for ligand peptide deficiency

The peptides or the like of the present invention can be used as prophylactic or therapeutic drugs for deficiency of ligand peptide or G protein-coupled receptor protein (APJ) according to activities that the peptides or the like of the present invention have against G protein-coupled receptor proteins (APJ).

For example, if a patient could be not expected to have the physiological activity of ligands, such as activities of regulating central nervous functions, circulatory functions, cardiac functions, immune functions, digestive functions, metabolic functions, and reproductive organ functions, due to decreased G protein-coupled receptor protein (APJ) in vivo, the ligand peptide activity can be made sufficiently effective by administering the peptides or the like of the present invention to said patient to increase the amount of in vivo ligand peptide of said patient. Thus the peptides or the like of the present invention can be used as safe and low toxic prophylactic or therapeutic drugs for ligand peptide deficiency.

(2) Quantitative assay on G protein-coupled receptor protein (APJ) for ligand peptide

Since the peptides or the like of the present invention have binding capacities for G protein-coupled receptor proteins (APJ) or their salts and partial peptides of said receptor proteins or their salts, in vivo concentrations of G protein-coupled receptor proteins (APJ) or their salts, or partial peptides of said receptor proteins, their amides, esters, or salts thereof can be determined with a high sensitivity.

For partial peptides of said G protein-coupled receptor proteins (APJ), for example, a site that is exposed to the outside of the cell membrane among molecules of G protein-coupled receptor proteins may be used. In other words, they are peptides containing the portion that has been analyzed to be an extracellular region (hydrophilic site) by the hydrophobic plot analysis of G protein-coupled receptor proteins. Similarly, peptides containing a hydrophobic site as a part may also be used. Peptides that contain each domain individually or partial peptides contain multiple domains at the same time.

Amides and esters of partial peptides of G protein-coupled receptor proteins (APJ) can be obtained in the same way as the amides and esters of the peptides of the present invention. Salts similar to the salts of the peptides of the present invention can be used as salts of partial peptides of G protein-coupled receptor proteins (APJ).

This quantitative assay can be applied in combination with the competitive assay, for example.

In other words, concentrations of G protein-coupled receptor proteins (APJ) or their salts, or partial peptides of G protein-coupled receptor proteins (APJ), their amides, esters, or salts thereof in the specimen can be determined by bringing it into contact with the peptides or the like of the present invention. More specifically, it can be applied in accordance with known procedures such as ① and ② described below or one similar to them.

① Hiroshi Irie, editor, Radioimmunoassay, Kodansha, 1974
② Hiroshi Irie, editor, Radioimmunoassay (Sequel), Kodansha, 1979

(3) Screening procedures for compounds that may alter the binding capacities of G protein-coupled receptor proteins (APJ) and the peptides or the like of the present invention

Compounds (e.g. peptides, proteins, non-peptide compounds, synthesized compounds and fermentation products) or their salts, which may alter the binding capacities of the peptides or the like of the present invention and G protein-coupled receptor proteins (APJ) can be screened by using G protein-coupled receptor proteins (APJ) or their salts, or said partial peptides, their amides, esters, or salts thereof, or by establishing an expression system of recombinant receptor proteins (APJ) and using the receptor binding assay system with said expression system. These compounds include compounds having cell stimulation activities through G protein-coupled receptors (APJ) (e.g. activities that promote or inhibit arachidonic acid liberation, acetylcholine liberation, intracellular $Ca^{2+}$ liberation, intracellular cAMP production, intracellular cGMP production, inositol phosphate production, the variation in membrane potential, intracellular protein phosphorylation, and c-fos activation or pH decrease) (G protein-coupled . receptor agonists) and compounds not containing said cell stimulating activities (G protein-coupled receptor antagonists). "To alter binding capacities" implies both inhibiting binding with the peptides or the like in the present invention and promoting binding with the same.

Namely, the present invention provides screening procedures for compounds or their salts that may alter the binding capacities of the peptides or the like of the present invention and G protein-coupled receptor proteins (APJ) described above, which are characterized by making a comparison between (i) the case where the peptides or the like of the present invention are brought into contact with G protein-coupled receptor proteins (APJ) or their salts, or partial peptides of said receptor proteins or their salts and (ii) the case where the peptides or the like of the present invention and test compounds are brought into contact with G protein-coupled receptor proteins (APJ) described above or their salts, or partial peptides of said receptor proteins, their amides, esters, or salts thereof.

In the screening procedures of the present invention, the binding or cell stimulating activities of the peptides or the like of the present invention relative to said G protein-coupled receptor proteins (APJ) or partial peptides of said receptor proteins in (i) the case where the peptides or the like of the present invention are brought into contact with G protein-coupled receptor proteins (APJ) described above, or partial peptides of said receptor proteins and (ii) the case where the peptides or the like of the present invention and test compounds are brought into contact with G protein-coupled receptor proteins (APJ) described above, or partial peptides of said receptor proteins are determined and compared them.

The screening procedures of the present invention specifically include:

① Screening procedures for compounds or their salts that may alter the binding capacities of the peptides or the like of the present invention and G protein-coupled receptor proteins (APJ), which are characterized by determining the binding of the labeled peptides or the like of the present invention relative to said G protein-coupled receptor proteins (APJ) or said partial peptides, their amides, esters or salts thereof in the case where the labeled peptides or the like of the present invention are brought into contact with G protein-coupled receptor proteins (APJ) described above or their salts, or partial peptides of G protein-coupled receptor proteins (APJ), their amides, esters, or salts thereof, and the case where the labeled peptides or the like of the present invention and test compounds are brought into contact with G protein-coupled receptor proteins (APJ) or their salts, or partial peptides of G protein-coupled receptor proteins (APJ), their amides, esters, or salts thereof, and comparing them.

② Screening procedures for compounds or their salts that may alter the binding capacities of the peptides or the like of the present invention and G protein-coupled receptor proteins (APJ), which are characterized by determining the binding of the labeled peptides or the like of the present invention relative to the cells or the membrane fractions of the cells in the case where the labeled peptides or the like of the present invention are brought into contact with cells that contain G protein-coupled receptor proteins (APJ) or membrane fractions of said cells, and the case where the labeled peptides or the like of the present invention and test compounds are brought into contact with cells that contain G protein-coupled receptor proteins (APJ) or membrane fractions of said cells, and comparing them.

③ Screening procedures for compounds or their salts that may alter the binding capacities of the peptides or the like of the present invention and G protein-coupled receptor proteins (APJ), which are characterized by determining the binding of the labeled peptides or the like of the present invention relative to and said G protein-coupled receptor proteins in the case where the labeled peptides or the like of the present invention are brought into contact with G protein-coupled receptor proteins (APJ) expressed on the cell membrane by culturing transformants containing DNAs that code G protein-coupled receptor proteins (APJ), and the case where the labeled peptides or the like of the present invention and test compounds are brought into contact with G protein-coupled receptor proteins (APJ) expressed on the cell membrane by culturing transformants containing DNAs that code G protein-coupled receptor proteins (APJ), and comparing them.

④ Screening procedures for compounds or their salts that may alter the binding capacities of the peptides or the like of the present invention and G protein-coupled receptor proteins (APJ), which are characterized by determining cell stimulation activities through G protein-coupled receptors (APJ) (e.g. activities that promote or inhibit arachidonic acid liberation, acetylcholine liberation, intracellular $Ca^{2+}$ liberation, intracellular cAMP production, intracellular cGMP production, inositol phosphate production, the variation in membrane potential, intracellular protein phosphorylation, c-fos activation or pH decrease) in the case where compounds that activate G protein-coupled receptor (APJ) (e.g. the peptides of the present invention) are brought into contact with cells containing G protein-coupled receptor proteins (APJ), and the case where compounds that activate G protein-coupled receptor (APJ) and test compounds are brought into contact with cells containing G protein-coupled receptor proteins (APJ), and comparing them.

⑤ Screening procedures for compounds or their salts that may alter the binding capacities of the peptides or the like of the present invention and G protein-coupled receptor proteins (APJ), which are characterized by determining cell stimulation activities through G protein-coupled receptors (APJ) (e.g. activities that promote or inhibit arachidonic acid liberation, acetylcholine liberation, intracellular $Ca^{2+}$ liberation, intracellular cAMP production, intracellular cGMP production, inositol phosphate production, the variation in membrane potential, intracellular protein phosphorylation, c-fos activation or pH decrease) in the case where compounds that activate G protein-coupled receptor (APJ) (e.g. the peptides of the present invention)are brought into contact with G protein-coupled receptor proteins (APJ) expressed on the cell membrane by culturing transformants containing DNAs that code G protein-coupled receptor proteins (APJ), and the case where compounds that activate G protein-coupled receptor (APJ) and test compounds are brought into contact with G protein-coupled receptor proteins (APJ) expressed on the cell membrane by culturing transformants containing DNAs that code G protein-coupled receptor proteins (APJ), and comparing them.

Here, the screening procedures of the present invention is specifically described as follows:

First, G protein-coupled receptor proteins (APJ) that are used for the screening procedures of the present invention may be any of those that contain G protein-coupled receptor proteins described above or partial peptides of G protein-coupled receptor proteins, preferably membrane fractions of human or warm-blooded animal organs. However, since the availability of human-derived organs especially is extremely limited, G protein-coupled receptor proteins (APJ) that are massively expressed by using recombinants are suitable for screening.

Preparation methods described later may be followed when cells or fractions of said cell membranes that contain G protein-coupled receptor proteins are used for the screening procedures of the present invention.

When cells that contain G protein-coupled receptor proteins are used, said cells may be fixated with glutaraldehyde or folmalin. Fixation method may be carried out following any known method.

A cell that contains G protein-coupled receptor proteins refers to a host cell expressing G protein-coupled receptor proteins. *Esherichia*, *Bacillus,* yeast, an insect or insect cell, and an animal cell may be used as a host.

As *Esherichia*, *Esherichia coli* K12/DH1 [Proceedings of the National Academy of Sciences of the USA (Proc. Natl. Acad. Sci. USA), Vol. 60, 160(1968)], JM103 [Nucleic Acids Research, Vol. 9, 309(1981)], JA221 [Journal of Molecular Biology, Vol. 120, 517 (1978)], HB101 [Journal of Molecular Biology, Vol. 41, 459 (1969)], and C600 [Genetics, Vol. 39, 440 (1954)] are used.

As *Bacillus,* for example, *Bacillus subtilis* MI114 [Gene, Vol. 24, 255(1983)] and 207-21 [Journal of Biochemistry, Vol. 95, 87(1984)] are used.

As yeasts, for example, *Saccharomyces cerevisiae* AH22, AH22R$^-$, NA87-11A, DKD-5D and 20B-12 are used.

As insects, for example, larvae of *Bombyx* are used [Nature, Vol. 315, 592(1985)].

As insect cells, in case of virus if the virus is, for example, AcNPV, *Spondoptera frugiperda* cells (Sf cells), *Trichoplusia ni* mesogaster-derived MG1 cells, *Trichoplusia ni* ovum-derived High Five™, cells, *Mamestra brassicae*-derived cells or *Estigmena acrea*-derived cells are used. If the virus is BmNPV, *Bombyx mori* N cells are used. As the Sf cells, for example, Sf9 cells (ATCC CRL1711), Sf21 cells are used [all by Vaughn, J.L. et al., in Vitro, Vol. 13, 213-217 (1977)].

As animal cells, for example, monkey COS-7 cells, Vero cells, Chinese hamster cell CHO, DHFR-deficient Chinese hamster cell CHO (dhfr-CHO cell), mouse L cells, mouse 3T3 cells, mouse myeloma cells, human HEK293 cells, human FL cells, 293 cells, C127 cells, BALB3T3 cells and Sp-2/O cells are used.

A membrane fraction refers to a fraction that contain an abundance of cell membranes that are obtained by means of any of known methods after disrupting cells. Methods for cell disruption include those where cells are crushed using a Potter-Elvehjem type homogenizer, cell disruption by Waring blender or Polytron (Kinematica AG), by ultrasound or by blowing cells out from a narrow nozzle while pressurizing using a French press.

For fractionation of cell membranes, centrifugal fractionations such as a differential centrifugation and a fractionation density gradient centrifugation are mainly applied. For example, after cell disrupter fluid is centrifuged at a low speed (500 rpm to 3,000 rpm) for a short time (usually approx. 1 to 10 minutes), the supernatant is centrifuged at higher speed (15,000 rpm to 30,000 rpm) usually for 30 minutes to 2 hours to obtain precipitation that is to be membrane fraction. Said membrane fraction contains an abundance of expressed G protein-coupled receptor proteins and membrane components such as cell-derived phospholipid and membrane protein.

The amount of G protein-coupled receptor proteins in the cells and membrane fractions that contain said G protein-coupled receptor proteins is preferably $10^3$ to $10^8$ molecules per cell, and optimally $10^5$ to $10^7$ molecules. It is noted that the more the expressed amount is, the higher is the ligand binding activity per membrane fraction (specific activity), so that a screening system with higher sensitivity can be constructed and the more specimens can be determined in the same lot.

To perform ① to ③ described above that are means for screening compounds that may alter the binding capacities of the peptides or the like of the present invention and G protein-coupled receptor, appropriate G protein-coupled receptor fractions and labeled peptides or the like of the present invention are used. As G protein-coupled receptor fractions, G protein-coupled receptor fractions of natural type, or G protein-coupled receptor fractions of recombinant type having an equivalent activity are desirable. An equivalent activity here refers to an equivalent ligand binding activity or the like. As a labeled ligand, a labeled ligand, a labeled ligand analogous compound and the like are used. For example, ligands labeled with [$^3$H], [$^{125}$I], [$^{14}$C] or [$^{35}$S] are may be used.

More specifically, to perform screening for compounds that may alter the binding capacities of the peptides or the like of the present invention and G protein-coupled receptor proteins, first, the receptor preparation is prepared by suspending cells and membrane fractions that contain said G protein-coupled receptor proteins (APJ) in an appropriate buffer for screening. The buffer may be any of buffers such as phosphate buffer pH 4 to 10 (preferably pH 6 to 8) and Tris-HCl buffer, which do not inhibit binding ligands with receptors. For the purpose of reducing non-specific binding, surfactants such as CHAPS, Tween 80™ (Kao-Atlas), digitonin and deoxycholate may be added to the buffer. Moreover, for the purpose of inhibiting decomposition of receptor or the peptides or the like of the present invention, protease inhibitors such as PMSF, leupeptin, E-64 (Peptide Institute, Inc.) and pepstatin may be added. Certain amount (5,000 cpm to 50,000 cpm) of labeled peptides or the like of the present invention is added to 0.01 ml to 10 ml of said receptor solution and $10^{-4}$ to $10^{-1}$ μM of test compound are made to coexist. To know Non-specific binding (NSB), reaction tubes added by largely excessive amounts of unlabeled peptides or the like of the present invention are prepared. Reactions are carried out at 0°C to 50°C, preferably at 4°C to 37°C, for 20 minutes to 24 hours, preferably, for 30 minutes to 3 hours. After reaction, it is filtrated with a glass fiber filter paper, washed with an appropriate amount of the same buffer, and the residual radioactivity in the glass fiber filter paper is measured with a liquid scintillation counter or a γ-counter. Candidate compounds with competitive inhibitory capacity can be selected if Non-specific binding (NSB) of the compounds tested is not more than, for example, 50%, taking the count (B0 - NSB), the difference between the count without competitive substance (B0) and that of Non-specific binding (NSB), as 100%.

To perform screening for compounds that may alter the binding capacities of the peptides or the like of the present invention and G protein-coupled receptor proteins (APJ), cell stimulation activities through G protein-coupled receptor proteins (e.g. activities that promote or inhibit arachidonic acid liberation, acetylcholine liberation, intracellular $Ca^{2+}$ liberation, intracellular cAMP production, intracellular cGMP production, inositol phosphate production, the variation in membrane potential, intracellular protein phosphorylation, c-fos activation or pH decrease) can be determined by using known methods or commercially available assay kits.

More specifically, first, cells containing G protein-coupled receptor proteins are cultured in multi-well plates. When performing screening, a fresh medium or an appropriate buffer that have no toxicity to cells are replaced for in advance, a test compound is added and incubated for a certain period, before cells are extracted or the supernatant is recovered to determine resulting products following appropriate procedures. When it is difficult to assay production of a substance that is to be an indicator for cell stimulation activities (e.g. arachidonic acid) by the catabolic enzyme contained in cells, an inhibitor against said catabolic enzyme may be added to perform assay. Activities such as inhibition of cAMP production can be detected as inhibiting activities for cells of which the amount of basic production has been increased by forskolin or the like.

To perform screening by measuring cell stimulation activities, cells expressing appropriate G protein-coupled receptor proteins are required. As cells expressing the G protein-coupled receptor proteins of the present invention, recombinant G protein-coupled receptor proteins (APJ) expressed cell strain described above are desirable.

Test compounds include peptides, proteins, non-peptide compounds, synthesized compounds, fermentation products, cell extract, plant extract and animal-tissue extract, and may be either novel compounds or known compounds.

Screening kits for compounds that may alter the binding capacities of the peptides or the like of the present invention and G protein-coupled receptor proteins (APJ) are those that contain G protein-coupled receptor proteins or their salts; partial peptides of G protein-coupled receptor proteins, their amides, esters, or salts thereof; cells that contain G protein-coupled receptor proteins; or membrane fractions of cells containing G protein-coupled receptor proteins; and the peptides or the like of the present invention.

Screening kits of the present invention include the followings:

1. Screening reagents

① Screening buffer and washing buffer

Hanks' Balanced Salt Solution (Gibco) that added with 0.05% bovine serum albumin (Sigma)

It may be sterilized by filtration through a filter with 0.45 μm pore size and stored at 4°C, or may be prepared just before use.

② G protein-coupled receptor (APJ) preparation

CHO cells expressing G protein-coupled receptor proteins (APJ) are subcultured in 12-well plates with $5 \times 10^5$ cells per well and incubated at 37°C, 5% $CO_2$ and 95% air for 2 days.

③ Labeled peptides or the like of the present invention

The peptides or the like of the present invention are labeled with [$^3$H], [$^{125}$I], [$^{14}$C] ] or [$^{35}$S], dissolved in appropriate solvent or buffer, stored at 4°C or -20°C, and diluted with assay buffer to 1 μM just before use.

④ Peptides or the like of the present invention standard solution

Peptides or the like of the present invention are dissolved to 1 mM with PBS containing 0.1% bovine serum albumin (Sigma) and stored at -20°C.

2. Assay

① After cells expressing G protein-coupled receptor proteins (APJ) cultured in 12-well tissue culture plates are washed twice with 1 ml assay buffer, 490 μl of assay buffer is added to each well.
② After adding 5 μl of $10^{-3}$ to $10^{-10}$ M test compound solution, 5 μl of labeled peptides or the like of the present invention is added and made reacted at room temperature for 1 hour. To know the amount of non-specific binding, 5 μl of $10^{-3}$ M ligand is added instead of a test compound.
③ After removing the reactant solution, wash three times with 1 ml washing buffer. The peptides or the like of the present invention bound with cells are dissolved with 0.2N NaOH-1% SDS and mixed with 4 ml of liquid scintillator (Wako Pure Chemical Industries, Ltd.).
④ The radioactivity is measured by using a liquid scintillation counter (Beckman) and calculated by the following formula [数1]

[Formula 1]

$$PBM = [(B - NSB)/(B0 - NSB)] \times 100$$

PMB: Percent Maximum Binding
B : a value when the specimen is added
NSB: Non-specific Binding
B0 : Maximum Binding

Compounds and their salts that are obtained by using with the screening procedures or the screening kits of the present invention are compounds that alter binding (inhibit or promote binding) of the peptides or the like of the present invention with G protein-coupled receptor (APJ), or more specifically, compounds and their salts that have cell stimulation activities through G protein-coupled receptors (so-called G protein-coupled receptor agonist) or compounds that have said stimulation activities (so-called G protein-coupled receptor antagonist). Said compounds include peptides, proteins, non-peptide compounds, synthesized compounds, fermentation products, cell extract, and may be either novel compounds or known compounds.

The methods for evaluation whether it is a G protein-coupled receptor agonist or antagonist may be accordance with (i) or (ii) described below.

(i) After performing the binding assay shown in the screening procedures of ① to ③ described above to obtain compounds that alter the binding capacities of the peptides or the like of the present invention with G protein-coupled receptors (APJ) (especially, that inhibit binding), assay whether said compounds have cell stimulation activities through G protein-coupled receptors (APJ). A compound or its salt that has a cell stimulation activity is a G protein-coupled receptor agonist, while a compound or its salt that does not have said activity is a G protein-coupled receptor antagonist.
(ii) (a) A test compound is brought into contact with cells that contain G protein-coupled receptor proteins (APJ) and cell stimulation activities through G protein-coupled receptors (APJ) are determined. A compound or its salt that has a cell stimulation activity is a G protein-coupled receptor agonist. (b) Cell stimulation activities through G protein-coupled receptors (APJ) in the case where a compound that activates a G protein-coupled receptor (e.g. the peptides or the like of the present invention or G protein-coupled receptor agonist) is brought into contact with cells that contain G protein-coupled receptor proteins (APJ), and the case where a compound that activates a G protein-coupled receptor and a test compound are brought into contact with cells that contain G protein-coupled receptor proteins (APJ) are determined and compared. A compound or its salts that can reduce cell stimulation activities by a compound that activates a G protein-coupled receptor coupled receptors (APJ) is a G protein-coupled receptor antagonist.

Since said G protein-coupled receptor agonist has activities similar to the physiological activities the peptides or the like of the present invention against G protein-coupled receptors (APJ), it is useful as a safe and low toxic pharmaceuticals, as well as the peptides or the like of the present invention.
On the contrary, since G protein-coupled receptor antagonist can inhibit the physiological activities the peptides or the like of the present invention against G protein-coupled receptors (APJ), it is useful as a safe and low toxic pharmaceuticals that inhibit said receptor activities.
Since the peptides or the like of the present invention are involved in activities of central nervous functions, circulatory functions, cardiac functions, immune functions, digestive functions, metabolic functions, or reproductive organ functions, agonists or antagonists described above can be used as therapeutic or prophylactic drugs against disorders, such as: dementia including dementia associated with senile dementia, cerebrovascular dementia, dementia associated with retroplastic diseases of the systemic degeneration type (e.g. Alzheimer's disease, Parkinson's disease, Pick's disease, Huntington's disease), dementia associated with infective diseases (e.g. slow virus infections such as Creutzfeldt-Jakob disease), dementia associated with endocrine disease, metabolic disease or toxic disease (e.g. hypothyroidism, vitamin B12 deficiency disease, alcoholism, and intoxication with various-drugs, metal or organic compound), dementia associated with neoplastic diseases such (e.g. brain tumor), and dementia associated with traumatopathy (e.g. chronic subdural hematoma); depression, ADHD (minimal brain dysfunction) syndrome; disturbance of consciousness; anxiety disorder; schizophrenia; phobia, growth hormone secretion disorder (e.g. gigantism, acromegaly), hyperphagia, polyphagia, hypercholesterolemia, hyperglyceridemia, hyperlipidemia, hyperprolactinemia, hypoglycemia, hypopituitarism, hypophysial dwarfism, diabetes (e.g. diabetic complication, diabetic nephropathy, diabetic neuropathy and diabetic retinopathy); cancer (e.g. breast cancer, lymphatic leukemia, lung cancer, bladder cancer, ovarian cancer and prostate cancer); pancreatitis, renal diseases (e.g. chronic renal failure, nephritis); Turner's syndrome; neuropathy; rheumatoid arthritis; spinal cord injury; transient ischemic attack; amyotrophic lateral sclerosis; acute myocardial infarct; spinocerebellar degeneration; fracture; injury; atopic dermatitis; osteoporosis; asthma; epilepsy; infertility; arteriosclerosis; emphysema pulmonum; pulmonary edema or hypogalactia. Also they can be applied as a sedative hypnotic, an agent that improves the nutritional status of post-operative patients, vasopressor and depressor drug.
In addition, the peptides of the present invention can be used as therapeutic or prophylactic drugs against AIDS (Acquired Immune Deficiency Syndrome) or the like.
As salts of the compounds that are obtained by using with the screening procedures or the screening kits described above are, for example, pharmaceutically acceptable salts are used. For example, these salts include salts with inorganic bases, salts with organic bases, salts with inorganic acids, salts with organic acids, and salts with basic or acidic amino acids.
Preferable examples salts with inorganic bases include salts with alkali metal salts such as sodium salts and potassium salts, alkaline earth metal salts such as calcium salts and magnesium salts, aluminum salts, or ammonium salts.
Preferable examples salts with organic bases include salts with trimethylamine, triethylamine, pyridine, picoline, 2,6-lutidine, ethanolamine, diethanolamine, triethanolamine, cyclohexylamine. dicyclohexylamine or N,N'-dibenzylethylenediamine.

Preferable examples of salts with inorganic aids include salts with hydrochloric acid, hydrobromic acid, sulfuric acid or phosphoric acid.
Preferable examples of salts with organic acids include salts with formic acid, acetic acid, propionic acid, fumaric acid, oxalic acid, tartaric acid, maleic acid, citric acid, succinic acid, malic acid, methanesulfonic acid, benzenesulfonic acid or benzoic acid.
Preferable examples of salts with basic amino acids include salts with arginine, lysine or ornithine.
Preferable examples of salts with acidic amino acids include salts with aspartic acid or glutamic acid.
When compounds and their salts that are obtained by using the screening procedures or the screening kits of the present invention are used as pharmaceuticals described above, they can be embodied in the same way as the case where the peptides or the like are embodied as pharmaceuticals.
In the present specification and drawings thereof, abbreviations for bases, amino acids and others used in the present specification are based on abbreviations specified by the IUPAC-IUB Commission on Biochemical Nomenclature or abbreviations in common use in the relevant fields. Some examples are given below.
When an optical isomer is present in amino acid, it is of the L-configuration, unless specifically stated.

| | |
|---|---|
| DNA : | deoxyribonucleic acid |
| cDNA : | complementary deoxyribonucleic acid |
| A : | Adenine |
| T : | Thymine |
| G : | Guanine |
| C : | Cytosine |
| Y : | Thymine or Cytosine |
| N : | Thymine, Cytosine, Adenine or Guanine |
| R : | Adenine or Guanine |
| M : | Cytosine or Adenine |
| W : | Thymine or Adenine |
| S : | Cytosine or Guanine |
| RNA : | Ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | adenosine triphosphate |
| EDTA : | ethylenediaminetetraacetic acid |
| SDS : | sodium dodecyl sulfate |
| EIA : | enzyme immunoassay |
| Gly or G : | Glycine |
| Ala or A : | Alanine |
| Val or V : | Valine |
| Leu or L : | Leucine |
| Ile or I : | Isoleucine |
| Ser or S : | Serine |
| Thr or T : | Threonine |
| Cys or C : | Cysteine |
| Met or M : | Methionine |
| Glu or E : | Glutamic acid |
| Asp or D : | Aspartic acid |
| Lys or K : | Lysine |
| Arg or R : | Arginine |
| His or H : | Histidine |
| Phe or F : | Phenylalanine |
| Tyr or Y : | Tyrosine |
| Trp or W : | Tryptophan |
| Pro or P : | Proline |
| Asn or N : | Asparagine |
| Gln or Q : | Glutamine |
| pGlu : | Pyroglutamic acid |

| | |
|---|---|
| Me : | Methyl group |
| Et : | Ethyl group |
| Bu : | Butyl group |
| Ph : | Phenyl group |
| Nle : | Norleucine |
| Thi : | 2-Thienylalanine |
| Phg : | Phenylglycine |
| Pya (2) : | 2-pyridylalanine |
| Adi ($NH_2$) : | 2-aminoadipic acid-6 amide |
| Hyp : | Oxyproline (Hydroxyproline) |
| Ac-Arg : | $N^{\alpha}$-acetylarginine |
| Lys (Ac) : | $N^{\varepsilon}$-acetyllysine |
| Lys (Me) : | $N^{\varepsilon}$-metyllysine |
| Lys (Tos) : | $N^{\varepsilon}$-tosyllysine |
| Arg (Tos) : | $N^{\varepsilon}$-Tosylarginine |
| Phe (Cl) : | 4-Chlorophenylalanine |
| Nal (2) : | 2-naphthylalanine |
| Cha : | Cyclohexylalanine |
| Met (O) : | Methioninesulfoxide |
| Tyr (Me) : | O-Methyltyrosine |
| Tyr (I) : | 3-Iodotyrosine |

Substituents, protecting groups, reagents that are used in this specification are represented by symbols described below.

| | |
|---|---|
| Tos : | p-Toluenesulfonyl |
| HONB : | N-hydroxy-5-norbornene-2,3-dicarboximido |
| Bzl : | Benzyl |
| Z : | Benzyloxycarbonyl |
| Br-Z : | 2-Bromobenzyloxycarbonyl |
| Cl-z : | 2-Chlorobenzyloxycarbonyl |
| Boc : | t-Butyloxycarbonyl |
| HOBt : | 1-Hydroxybenztriazole |
| DCC : | N,N'-Dicyclohexylcarbodiimide |
| TFA : | Trifluoroacetic acid |
| Fmoc : | N-9-fluorenylmethoxycarbonyl |
| DNP : | Dinitrophenyl |
| Bum : | tertiary butoxymethyl |
| Trt : | Trityl |
| Pbf : | 2,2,4,6,7-Pentamethyldihydrobenzofuran-5-sulfonyl |
| HOOBt : | 3-Hydroxy-4-oxo-3,4-dihydro-1,2,3-benzotriazine |
| TFE : | trifluoroethanol |
| HOAt : | 1-hydroxy-7-azabenzotriazole |
| PyBrop : | bromotris(pyrrolydino)phosphonium hexafluorophosphate |
| TMS-Br : | Trimethylsilyl bromide |
| TC : | Thiazolidine-4(R)-carboxamide group |
| Bom : | Benzyloxymethyl |
| NMP : | N-Methylpyrrolidone |
| PAM : | Phenylacetamidemethyl |
| DCM : | Dichloromethane |
| DMF : | N,N-Dimethylformamide |
| DIEA : | N,N-Diisopropylethylamine |
| Clt : | 2-Chlorotrityl |
| For : | Formyl |

Sequence ID numbers in the sequence listing in this specification indicate the following sequences:

[Sequence ID No.:1] This Sequence ID number shows the amino acid sequence of the peptide obtained in Example 1 described below.

[Sequence ID No.:2] This Sequence ID number shows the amino acid sequence of the peptide obtained in Example 2 described below.

[Sequence ID No.:3] This Sequence ID number shows the amino acid sequence of the peptide obtained in Example 3 described below.

[Sequence ID No.:4] This Sequence ID number shows the amino acid sequence of the peptide obtained in Example 4 described below.

[Sequence ID No.:5] This Sequence ID number shows the amino acid sequence of the peptide obtained in Example 5 described below.

[Sequence ID No.:6] This Sequence ID number shows the amino acid sequence of the peptide obtained in Example 6 described below.

[Sequence ID No.:7] This Sequence ID number shows the amino acid sequence of the peptide obtained in Example 7 described below.

[Sequence ID No.:8] This Sequence ID number shows the amino acid sequence of the peptide obtained in Example 8 described below.

[Sequence ID No.:9] This Sequence ID number shows the amino acid sequence of the peptide obtained in Example 9 described below.

[Sequence ID No.:10] This Sequence ID number shows the amino acid sequence of the peptide obtained in Example 10 described below.

[Sequence ID No.:11] This Sequence ID number shows the amino acid sequence of the peptide obtained in Example 11 described below.

[Sequence ID No.:12] This Sequence ID number shows the amino acid sequence of the peptide obtained in Example 12 described below.

[Sequence ID No.:13] This Sequence ID number shows the amino acid sequence of the peptide obtained in Example 13 described below.

[Sequence ID No.:14] This Sequence ID number shows the amino acid sequence of the peptide obtained in Example 14 described below.

[Sequence ID No.:15] This Sequence ID number shows the amino acid sequence of the peptide obtained in Example 15 described below.

[Sequence ID No.:16] This Sequence ID number shows the amino acid sequence of the peptide obtained in Example 16 described below.

[Sequence ID No.:17] This Sequence ID number shows the amino acid sequence of the peptide obtained in Example 17 described below.

[Sequence ID No.:18] This Sequence ID number shows the amino acid sequence of the peptide obtained in Example 18 described below.

[Sequence ID No.:19] This Sequence ID number shows the amino acid sequence of the peptide obtained in Example 19 described below.

[Sequence ID No.:20] This Sequence ID number shows the amino acid sequence of the peptide obtained in Example 20 described below.

[Sequence ID No.:21] This Sequence ID number shows the amino acid sequence of the peptide obtained in Example 21 described below.

[Sequence ID No.:22] This Sequence ID number shows the amino acid sequence of the peptide obtained in Example 22 described below.

[Sequence ID No.:23] This Sequence ID number shows the amino acid sequence of the peptide obtained in Example 23 described below.

[Sequence ID No.:24] This Sequence ID number shows the amino acid sequence of the peptide obtained in Example 24 described below.

[Sequence ID No.:25] This Sequence ID number shows the amino acid sequence of the peptide obtained in Example 25 described below.

[Sequence ID No.:26] This Sequence ID number shows the amino acid sequence of APJ.

[Sequence ID No.:27] This Sequence ID number shows the DNA sequence that codes the amino acid sequence of the Sequence ID No.:26.

[Sequence ID No.:28] This Sequence ID number shows the amino acid sequence of the peptide obtained in Example 26 described below.

[Sequence ID No.:29] This Sequence ID number shows the amino acid sequence of the peptide obtained in Example 27 described below.

[Sequence ID No.:30] This Sequence ID number shows the amino acid sequence of the peptide obtained in Example 28 described below.

[Sequence ID No.:31] This Sequence ID number shows the amino acid sequence of the peptide obtained in Example 29 described below.

[Sequence ID No.:32] This Sequence ID number shows the amino acid sequence of the peptide obtained in Example 30 described below.

[Sequence ID No.:33] This Sequence ID number shows the amino acid sequence of the peptide obtained in Example 31 described below.

[Sequence ID No.:34] This Sequence ID number shows the amino acid sequence of the peptide obtained in Example 32 described below.

[Sequence ID No.:35] This Sequence ID number shows the amino acid sequence of the peptide obtained in Example 33 described below.

[Sequence ID No.:36] This Sequence ID number shows the amino acid sequence of the peptide obtained in Example 34 described below.

[Sequence ID No.:371 This Sequence ID number shows the amino acid sequence of the peptide obtained in Example 35 described below.

[Sequence ID No.:38] This Sequence ID number shows the amino acid sequence of the peptide obtained in Example 36 described below.

[Sequence ID No.:39] This Sequence ID number shows the amino acid sequence of the peptide obtained in Example 37 described below.

[Sequence ID No.:40] This Sequence ID number shows the amino acid sequence of the peptide obtained in Example 38 described below.

[Sequence ID No.:41] This Sequence ID number shows the amino acid sequence of the peptide obtained in Example 39 described below.

[Sequence ID No.:42] This Sequence ID number shows the amino acid sequence of the peptide obtained in Example 40 described below.

## Examples

Although the present invention is hereinafter described in more detail by showing examples and experimental examples, they are not to be construed as limitative to the scope of the present invention.

Example 1 (Production of Leu-Val-Gln-Pro-Arg-Gly-Ser-Arg-Asn-Gly-Pro-Gly-Pro-Trp-Gln-Gly-Gly-Arg-Arg-Lys-Phe-Arg-Arg-Gln-Arg-Pro-Arg-Leu-Ser-His-Lys-Gly-Pro-Nle-Pro-Phe)

A portion of 0.25 mmol of Fmoc-Phe-O-Clt resin (0.32 mmol/g) that was obtained by introducing Fmoc-Phe-OH into commercially available 2-chlorotrityl resin (Clt resin, 1.3 mmol/g) was set in a reaction chamber of a peptide synthesizer ABI 433A to carry out solid phase synthesis by means of a Fmoc/DCC/HOBt method. As protecting groups for side chains of a Fmoc amino acid, Pbf group was used for Arg, tBu group for Ser, Boc group for Trp and Lys, Trt group for His, Asn and Gln, respectively. Other amino acids used were with side chains unprotected, to which peptide chains are introduced in order towards the N terminal, from Phe to Leu of the sequence given above to obtain the desired protected peptide resin.

After stirring 50 mg (4.45 mmol) of the resin in 1 ml of a mixture of TFA, thioanisole, m-cresol, $H_2O$ and ethanedithiol (82.5:5:5:5:2.5) at room temperature for two hours, ether was added to precipitate white powder, which was centrifuged, and then the supernatant was removed. These processes were repeated three times. The residue was extracted with water and lyophilized to obtain 23.1 mg of white powder. The obtained crude peptide was subjected to preparative chromatography with TSK GEL ODS 120T column (20 × 300 mm) and eluted for 60 minutes with a linear concentration gradient of the solution A: 0.1% TFA-water and the solution B: acetonitrile containing 0.1% TFA at A/B: 85/15 to 75/25, to collect fractions containing the desired product to yield 10.2 mg of white powder by lyophilizing.

| | |
|---|---|
| $(M+H)^+$ by mass spectrometry: | 4176.0<br>(calculated value: 4176.3) |
| HPLC elution time: | 17.8 minutes |

Elution condition:

| | |
|---|---|
| Column : | YMC A-301-3 (4.6 × 100 mm) |
| Eluant : | With solution A: 0.1% TFA-water and solution B: acetonitrile containing 0.1% TFA, elution with a linear |

concentration gradient at A/B: 100/0 to 50/50 (25 min.)
Flow rate: 1.0 ml/min.

Example 2 (Production of Leu-Val-Gln-Pro-Arg-Gly-Ser-Arg-Asn-Gly-Pro-Gly-Pro-Trp-Gln-Gly-Gly-Arg-Arg-Lys-Phe-Arg-Arg-Gln-Arg-Pro-Arg-Leu-Ser-His-Lys-Gly-Pro-Nle-Pro-Tyr)

The amino acid to be introduced to 2-chlorotrityl resins (Clt resin, 1.3 mmol/g) in Example 1 was changed to Fmoc-Tyr(tBu)-OH, and synthesis and purification were carried out in the same manner to obtain 13.3 mg of white powder of the desired product by lyophilizing.

| | |
|---|---|
| $(M+H)^+$ by mass spectrometry: | 4192.0 (calculated value: 4192.3) |
| HPLC elution time: | 16.9 minutes |

Elution condition:

| | |
|---|---|
| Column : | YMC A-301-3 (4.6 × 100 mm) |
| Eluant : | With solution A: 0.1% TFA-water and solution B: acetonitrile containing 0.1% TFA, elution with a linear concentration gradient at A/B: 100/0 to 50/50 (25 min.) |
| Flow rate: | 1.0 ml/minute |

Example 3 (Production of pGlu-Arg-Pro-Arg-Leu-Ser-His-Lys-Gly-Pro-Nle-Pro-Phe)

A portion of 0.25 mmol of Fmoc-Gly-O-Clt resin (0.392 mmol/g) that was obtained by introducing Fmoc-Gly-OH into commercially available 2-chlorotrityl resin (Clt resin, 1.3 mmol/g) was set in a reaction chamber of a peptide synthesizer ABI 433A and the desired protected peptide resin was obtained by introducing Fmoc-Lys(Boc), Fmoc-His(Trt), Fmoc-Ser(tBu), Fmoc-Leu, Fmoc-Arg(Pbf), Fmoc-Pro, Fmoc-Arg(Pbf) and Boc-Gln in this order by means of Fmoc/DCC/HOBt method.
After 1 g of the resin was put into 20 ml of the mixture of AcOH:TFA:DCM (1:2:7) and stirred at room temperature for two hours, the mixture was filtrated to remove the resin, evaporated to remove the solvent and crystallized to obtain 362 mg of protected peptides (Boc-Gln-Arg(Pbf)-Pro-Arg(Pbf)-Leu-Ser(tBu)-His(Trt)-Lys(Boc)-Gly-OH).
H-Phe-OBzl·Hcl was condensed with Boc-Pro, Boc-Nle and Boc-Pro in this order to obtain 180 mg of Boc-Pro-Nle-Pro-Phe-Bzl.
50 mg of Boc-Gln-Arg(Pbf)-Pro-Arg(Pbf)-Leu-Ser(tBu)-His(Trt)-Lys(Boc)-Gly-OH and 3.96 mg of HOAt were dissolved in 700 ml of the mixture of DCM:DMF(4:1), added with 19.7 ml of DIEA, 13.5 mg of PyBrop and 18.2 mg of H-Pro-Nle-Pro-Phe-OBzl·Hcl (prepared by treating Boc-Pro-Nle-Pro-Phe-Bzl with 4N-HCl/dioxane) while cooling with ice, and were then stirred at room temperature for one hour after removing the ice bath. After adding citric acid crystals to neutralize, the mixture was freed from the solvent by evaporation and the solid precipitated with addition of water was extracted with chloroform. This was washed with 1N hydrochloric acid, saturated sodium bicarbonate solution and saturated brine, dried with anhydrous sodium sulfate, freed from the solvent by evaporation and filtrated to collect powder by adding ether and further purified by reprecipitation from ethyl acetate and ether to obtain 56 mg of Boc-Gln-Arg(Pbf)-Pro-Arg(Pbf)-Leu-Ser(tBu)-His(Trt)-Lys(Boc)-Gly-Pro-Nle-Pro-Phe-Obzl.
After the above product was put into the mixture of 982 μl of thioanisole, 110 μl of m-cresol, 215 μl of triisopropylsilane and 4 ml of TFA and stirred at room temperature for 90 minutes, it was added with 1.1 ml of TMS-Br, stirred for one hour while cooling with ice, and then stirred on the water bath of 20°C another one hour after removing the ice bath. After the reaction, the reaction solution was removed by evaporation and white powder was precipitated by adding ether to the residue to be centrifuged, the supernatant was removed. These processese were repeated three times. The residue was extracted with water and lyophilized to obtain white powder. Subsequently, the obtained white powder was dissolved in 80% AcOH to be heated at 70°C for 2 hours, and the solution was diluted with water and lyophilized. The obtained crude peptide were subjected to preparative chromatography with TSK GEL ODS 120T column (20 × 300 mm) and eluted for a 60 minutes with a linear concentration gradient of solution A: 0.1% TFA-water and solution B: acetonitrile containing 0.1% TFA at A/B: 80/29 to 70/30, to collect fractions containing the desired product to yield 14 mg of white powder by lyophilizing.

| | |
|---|---|
| $(M+H)^+$ by mass spectrometry: | 1515.7 (calculated value: 1515.9) |

HPLC elution time: 16.8 minutes

Elution condition:

| | |
|---|---|
| Column : | Wakosil 5C18T (4.6 × 100 mm) |
| Eluant : | With solution A: 0.1% TFA-water and solution B: acetonitrile containing 0.1% TFA, elution with a linear concentration gradient at A/B: 95/5 to 45/55 (25 min.) |
| Flow rate: | 1.0 ml/minute |

Example 4 (Production of pGlu-Arg-Pro-Arg-Leu-Ser-His-Lys-Gly-Pro-Nle-Pro-Tyr)

H-Phe-OBzl·Hcl in Example 3 was changed to H-Tyr(tBu)·Obzl.Hcl, and synthesis and purification were carried out in the same manner to obtain 29 mg of white powder.

| | |
|---|---|
| $(M+H)^+$ by mass spectrometry: | 1532.0 (calculated value: 1531.9) |
| HPLC elution time: | 14.6 minutes |

Elution condition:

| | |
|---|---|
| Column : | Wakosil 5C18T (4.6 × 100 mm) |
| Eluant : | With solution A: 0.1% TFA-water and solution B: acetonitrile containing 0.1% TFA, elution with a linear concentration gradient at A/B: 95/5 to 45/55 (25 min.) |
| Flow rate: | 1.0 ml/minute |

Example 5 (Production of pGlu-Arg-Pro-Arg-Leu-Ser-His-Lys-Gly-Pro-Nle-Pro)

Boc-Pro-Nle-Pro-OBzl synthesized by using H-Pro-OBzl·Hcl in stead of H-Phe-OBzl·Hcl in Example 3 was used, and synthesis and purification were carried out in the same manner to obtain 8 mg of white powder.

| | |
|---|---|
| $(M+H)^+$ by mass spectrometry: | 1368.4 (calculated value: 1368.8) |
| HPLC elution time: | 13.8 minutes |

Elution condition:

| | |
|---|---|
| Column : | Wakosil 5C18T (4.6 × 100 mm) |
| Eluant : | With solution A: 0.1% TFA-water and solution B: acetonitrile containing 0.1% TFA, elution with a linear concentration gradient at A/B: 95/5 to 45/55 (25 min.) |
| Flow rate: | 1.0 ml/minute |

Example 6 (Production of pGlu-Arg-Pro-Arg-Leu-Ser-His-Lys-Gly-Pro-Nle)

Boc-Pro-Nle-OBzl synthesized by using H-Nle-OBzl·Hcl in stead of H-Phe-OBzl·Hcl in Example 3 was used, and synthesis and purification were carried out in the same manner to obtain 9 mg of white powder.

| | |
|---|---|
| $(M+H)^+$ by mass spectrometry: | 1272.0 (calculated value: 1271.7) |
| HPLC elution time: | 12.9 minutes |

Elution condition:

| | |
|---|---|
| Column : | Wakosil 5C18T (4.6 × 100 mm) |
| Eluant : | With solution A: 0.1% TFA-water and solution B: acetonitrile containing 0,1% TFA, elution with a linear concentration gradient at A/B: 95/5 to 45/55 (25 min.) |
| Flow rate: | 1.0 ml/minute |

Example 7 (Production of Ac-Arg-Pro-Arg-Leu-Ser-His-Lys-Gly-Pro-Nle-Pro-Tyr)

A portion of 0.5 m mole of commercially available Boc-Tyr(Br-Z)-$OCH_2$-PAM resin (0.69 m mole/g resin) was set in a reaction chamber of a peptide synthesizer ABI 430A, to which Boc-Pro, Boc-Nle, Boc-Pro, Boc-Gly, Boc-Lys (Cl-Z), Boc-His(Bom), Boc-Ser(Bzl), Boc-Leu, Boc-Arg(Tos), Boc-Pro and Boc-Arg(Tos) were introduced in this order by means of Boc-strategy (NMP-HOBt) peptide synthesis method, and the desired protected peptide resin was obtained by acetylating with acetic anhydride after removing the last Boc group.
After 0.25 g of the resin was put in 5 ml of anhydrous hydrogen fluoride and stirred with 0.46 g of p-cresol at 0°C for 60 minutes, hydrogen fluoride was removed by evaporation under reduced pressure and the residue was added with diethyl ether to collect the precipitate by filtration and then extracted with aqueous acetic acid solution. After the extract was sufficiently concentrated and separately extracted by adding distilled water and diethyl ether, of which the water phase was collected to lyophilize, it was dissolved in a small amount of aqueous acetic acid solution to be applied to a Sephadex™ G-25 column (2.0 × 80 cm) packed with this solvent and developed with this solvent, and the major fractions were collected to obtain 53 mg of white powder by lyophilizing. After the product was applied to a reverse-phase chromato-column (2.6 × 60 cm) and washed with 200 ml of 0.1% TFA aqueous solution, a linear gradient elution with 300 ml of 0.1% TFA aqueous solution and 300 ml of 33% acetonitrile aqueous solution containing 0.1% TFA was carried out and fractions with an approximately 20% acetonitrile concentration to obtain 30 mg of white powder by lyophilizing.

$(M+H)^+$ by mass spectrometry: 1462.4
(calculated value: 1462.8)

Example 8 (Production of Ac-Arg-Pro-Arg-Leu-Ser-His-Lys-Gly-Pro-Nle-Pro)

Sequence amino acids were introduced to commercially available Boc-Pro-$OCH_2$-PAM resin in order, and synthesis and purification were carried out in the same manner as Example 7 to obtain 73 mg of white powder of the desired product.

$(M+H)^+$ by mass spectrometry: 1299.5
(calculated value: 1299.8)

Example 9 (Production of Ac-Arg-Pro-Arg-Leu-Ser-His-Lys-Gly-Pro-Nle)

Boc-Nle (0.57 mmol/g) was introduced to commercially available chloromethyl resin for peptide synthesis.
Synthesis and purification were carried out by using the above in the same manner to obtain 29 mg of white powder of the desired product.

$(M+H)^+$ by mass spectrometry: 1202.9
(calculated value: 1202.7)

Example 10 (Production of pGlu-Arg-Pro-Arg-Leu-Ser-His-Lys(Ac)-Gly-Pro-Met-Pro-Phe)

Commercially available Boc-Phe-$OCH_2$-PAM resin (0.72 m mole/g resin) was used, and Boc-Lys(Cl-Z) was changed to Boc-Lys(Ac) and Boc-Nle to Boc-Met, to which amino acids were introduced in the order of the sequence in the same manner as Example 7. Instead of the last acetylation, Z-pGlu was introduced under the same condition as each Boc-amino acid. The resin was treated with hydrogen fluoride in the same manner as Example 7 and purified to obtain 70 mg of white powder of the desired product.

$(M+H)^+$ by mass spectrometry: 1575.5

(calculated value: 1575.8)

Example 11 (Production of pGlu-Arg-Pro-Arg-Leu-Ser-His-Lys(Me)-Gly-Pro-Met-Pro-Phe)

Boc-Lys(Ac) in Example 10 was changed to Boc-Lys(Me-Boc), and synthesis and purification were carried out in the same manner to obtain 35 mg of white powder of the desired product.

$(M+H)^+$ by mass spectrometry: 1547.5
(calculated value: 1547.8)

Example 12 (Production of pGlu-Arg-Pro-Arg-Leu-Ser-His-Lys(Ac)-Gly-Pro-Nle-Pro-Phe)

Boc-Met in Example 10 was changed to Boc-Nle, and synthesis and purification were carried out in the same manner to obtain 58 mg of white powder of the desired product.

$(M+H)^+$ by mass spectrometry: 1558.1
(calculated value: 1557.9)

Example 13 (Production of pGlu-Arg-Pro-Arg-Leu-Ser-His-Lys(Me)-Gly-Pro-Nle-Pro-Phe)

Boc-Met in Example 11 was changed to Boc-Nle, and synthesis and purification were carried out in the same manner to obtain 58 mg of white powder of the desired product.

$(M+H)^+$ by mass spectrometry: 1529.6
(calculated value: 1529.9)

Example 14 (Production of pGlu-Arg-Pro-Arg-Leu-Ser-His-Lys(Tos)-Gly-Pro-Nle-Pro-Phe)

Boc-Lys(Ac) in Example 12 was changed to Boc-Lys(Tos), and synthesis and purification were carried out in the same manner to obtain 60 mg of white powder of the desired product.

$(M+H)^+$ by mass spectrometry: 1670.2
(calculated value: 1669.9)

Example 15 (Production of pGlu-Arg-Pro-Arg-Leu-Ser-His-Arg(Tos)-Gly-Pro-Nle-Pro-Phe)

The same Fmoc-Phe-O-Clt resin as Example 1 was used, to which target sequence amino acids were introduced by using in the same manner. For protecting groups for side chains of Fmoc amino acids, a Tos group was used only for the Arg that was introduced first and a Pbf group for other Arg, a tBu group for Ser, a Boc group for Trp and Lys, a Trt group for His, Asn and Gln. By using amino acids with side chains unprotected for other amino acids or using pGlu without protecting, synthesis and purification were carried out in the same manner as Example 1 to obtain 40 mg of white powder of the desired product.

$(M+H)^+$ by mass spectrometry: 1697.7
(calculated value: 1697.9)

Example 16 (Production of pGlu-Arg-Pro-Arg-Nle-Ser-His-Lys-Gly-Pro-Nle-Pro-Phe)

In the same manner as Example 15 and by using Pbf for all protecting groups for side chains of Arg, synthesis and purification were carried out to obtain 66 mg of white powder of the desired product.

$(M+H)^+$ by mass spectrometry: 1515.8
(calculated value: 1515.9)

Example 17 (Production of pGlu-Arg-Pro-Arg-Nle-Ser-His-Lys-Gly-Pro-Nle-Pro-Tyr)

Commercially available Boc-Tyr(Br-Z)-$OCH_2$-PAM resin (0.69 m mole/g resin) in Example 7 was used, and by changing Boc-Lys(Ac) in Example 10 to Boc-Lys(Cl-Z) and Boc-Leu to Boc-Nle, synthesis and purification were carried out in the same manner to obtain 37 mg of white powder of the desired product.

(M+H)$^+$ by mass spectrometry: 1531.6
(calculated value: 1531.9)

Example 18 (Production of pGlu-Arg-Pro-Arg-Leu-Ser-His-Lys-Gly-Pro-Nle-Pro-Thi)

Phe in Example 3 was changed to Thi, and synthesis and purification were carried out in the same manner to obtain 21 mg of white powder of the desired product.

(M+H)$^+$ by mass spectrometry: 1521.7
(calculated value: 1521.8)

Example 19 (Production of pGlu-Arg-Pro-Arg-Leu-Ser-His-Lys-Gly-Pro-Nle-Pro-Phg)

Phe in Example 3 was changed to Phg, and synthesis and purification were carried out in the same manner to obtain 16 mg of white powder of the desired product.

(M+H)$^+$ by mass spectrometry: 1501.4
(calculated value: 1501.8)

Example 20 (Production of pGlu-Arg-Pro-Arg-Leu-Ser-His-Lys-Gly-Pro-Nle-Pro-Pya(2))

Phe in Example 3 was changed to Pya(2), and synthesis and purification were carried out in the same manner to obtain 25 mg of white powder of the desired product.

(M+H)$^+$ by mass spectrometry: 1516.7
(calculated value: 1516.9)

Example 21 (Production of Arg-Pro-Arg-Leu-Ser-His-Lys-Gly-Pro-Nle-Pro-Tyr)

Resins were treated with hydrogen fluoride without acetylation in Example 7 and purified in the same manner as Example 3 to obtain 85 mg of white powder of the desired product.

(M+H)$^+$ by mass spectrometry: 1421.0
(calculated value: 1420.8)

Example 22 (Production of Leu-Val-Adi($NH_2$)-Pro-Arg-Gly-Ser-Arg-Asn-Gly-Pro-Gly-Pro-Trp-Gln-Gly-Gly-Arg-Arg-Lys-Phe-Arg-Arg-Gln-Arg-Pro-Arg-Leu-Ser-His-Lys-Gly-Pro-Nle-Pro-Phe)

The same resin as Example 10 was used, to which Boc-amino acids were introduced in the order of the sequence by using Cl-Z group for Lys, Bom group for His, Bzl group for Ser, Tos group for Arg, and For group for Trp. This was treated with hydrogen fluoride in the presence of both p-cresol and 1,4-butanediol and purified in the same manner as Example 3 to obtain 20 mg of white powder of the desired product.

(M+H)$^+$ by mass spectrometry: 4190.0
(calculated value: 4190.4)

Example 23 (Production of Leu-Val-Lys(Ac)-Pro-Arg-Thr-Ser-Arg-Thr-Gly-Pro-Gly-Ala-Trp-Gln-Gly-Gly-Arg-Arg-Lys-Phe-Arg-Arg-Gln-Arg-Pro-Arg-Leu-Ser-His-Lys-Gly-Pro-Nle-Pro-Tyr)

The same resin as Example 7 was used, to which Boc-amino acids were introduced in the order of the sequence, by using Ac for the first Lys only and Cl-Z group for other Lys, Bom group for His, Bzl group for Ser, Tos group for Arg and For group for Trp for protecting group for side chains of Fmoc amino acids, and deblocking and purification were carried out in the same manner as Example 22 to obtain 24 mg of white powder of the desired product.

(M+H)$^+$ by mass spectrometry: 4234.2
(calculated value: 4234.4)

Example 24 (Production of Tyr-Leu-Val-Lys-Pro-Arg-Thr-Ser-Arg-Thr-Gly-Pro-Gly-Ala-Trp-Gln-Gly-Gly-Arg-Arg-Lys-Phe-Arg-Arg-Gln-Arg-Pro-Arg-Leu-Ser-His-Lys-Gly-Pro-Nle-Pro-Phe)

For protecting group for side chains of Fmoc amino acids, Pbf group was used for Arg, a tBu group for Ser, Thr and Tyr, a Boc group for Trp and Lys, a Trt group for His, Asn and Gln, and synthesis and purification were carried out in the same manner as Example 1 to obtain 17 mg of white powder of the desired product.

| | |
|---|---|
| $(M+H)^+$ by mass spectrometry: | 4344.6<br>(calculated value: 4344.5) |

Example 25 (Production of Z-pGlu-Arg-Pro-Arg-Leu-Ser-His-Lys(Ac)-Gly-Pro-Nle-Pro-Phe)

Arg(Tos) in Example 15 was changed to Lys(Ac), and pGlu to Z-pGlu, and synthesis and purification were carried out in the same manner to obtain 67 mg of white powder of the desired product.

| | |
|---|---|
| $(M+H)^+$ by mass spectrometry: | 1692.2<br>(calculated value: 1691.9) |

Example 26 (Production of Arg-Arg-Gln-Arg-Pro-Arg-Leu-Ser-His-Lys-Gly-Pro-Met(O))

To commercially available Boc-Met(O)-$OCH_2$-PAM resin (0.72 m mole/g resin), Boc-amino acids of which a Cl-Z group was used for Lys, Bom group for His, Bzl group for Ser, Tos group for Arg for protecting group for side chains were introduced in the order of the sequence, and deblocking and purification were carried out in the same manner as Example. 7 to obtain 19 mg of white powder of the desired product.

| | |
|---|---|
| $(M+H)^+$ by mass spectrometry: | 1634.9<br>(calculated value: 1634.9) |

Example 27 (Production of Arg-Arg-Gln-Arg-Pro-Arg-Leu-Ser-His-Lys-Gly-Pro-Nle-Pro-Tyr)

When the sequence of Arg-Arg-Gln-Arg-Pro-Arg-Leu-Ser-His-Lys-Gly-Pro-Nle-Pro-Tyr was introduced in the production process of the compound in Example 2, the resin was taken out, treated by means of deblocking and purified in the same manner as Example 2 to obtain the desired product.

| | |
|---|---|
| $(M+H)^+$ by mass spectrometry: | 1860.9<br>(calculated value: 1861.1) |
| HPLC elution time: | 16.75 minutes |

Elution condition:

| | |
|---|---|
| Column : | YMC ODS AM-301, S-5 mm, 120A (4.6 × 100 mm) |
| Eluant : | With solution A: 0.1% TFA-water and solution B: acetonitrile containing 0.1% TFA, elution with a linear concentration gradient at A/B: 100/0 to 50/50 (25 min.) |
| Flow rate: | 1.0 ml/minute |

Example 28 (Production of pGlu-Arg-Pro-Arg-Leu-Ser-His-Lys-Gly-Pro-Met-Phe(Cl))

Fmoc-Phe(Cl)-O-Clt resin (0.42 mmol/g) that was obtained by introducing Fmoc-Phe(Cl)-OH to a commercially available 2-chlorotrityl resin (Clt resin, 1.3 mmol/g) was used, to which amino acids were introduced in the order of the sequence in the order of the sequence in the same manner as Example 15, and deblocking and purification were carried out to obtain the desired compound.

| | |
|---|---|
| $(M+H)^+$ by mass spectrometry: | 1471.0<br>(calculated value: 1470.7) |
| HPLC elution time: | 19.39 minutes (Elution condition: the same as Example 27) |

Example 29 (Production of pGlu-Arg-Pro-Arg-Leu-Ser-His-Lys-Gly-Pro-Met-Pro-Phe(Cl))

The desired product was obtained in the same manner as Example 28.

| | |
|---|---|
| $(M+H)^+$ by mass spectrometry: | 1567.7 (calculated value: 1567.8) |
| HPLC elution time: | 19.81 minutes (Elution condition: the same as Example 27) |

Example 30 (Production of Arg-Pro-Arg-Leu-Ser-His-Lys-Gly-Pro-Met-Pro-Nal(2))

Fmoc-Nal(2)-O-Clt resin (0.45 mmol/g) that was obtained by introducing Fmoc-Nal(2)-OH to a commercially available 2-chlorotrityl resin (Clt resin, 1.3 mmol/g) was used, to which amino acids were introduced in the order of the sequence in the order of the sequence in the same manner as Example 15, and deblocking and purification were carried out to obtain the desired compound.

| | |
|---|---|
| $(M+H)^+$ by mass spectrometry: | 1472.6 (calculated value: 1472.8) |
| HPLC elution time: | 20.48 minutes (Elution condition: the same as Example 27) |

Example 31 (Production of Arg-Pro-Arg-Leu-Ser-His-Lys-Gly-Pro-Met-Nal(2))

The desired product was obtained in the same manner as Example 30.

| | |
|---|---|
| $(M+H)^+$ by mass spectrometry: | 1375.5 (calculated value: 1375.7) |
| HPLC elution time: | 20.35 minutes (Elution condition: the same as Example 27) |

Example 32 (Production of Arg-Pro-Arg-Leu-Ser-His-Lys-Gly-Pro-Met-Pro-Phe(Cl))

Fmoc-Phe(Cl)-O-Clt resin (0.42 mmol/g) was used, and the desired product was obtained in the same manner as Example 28.

| | |
|---|---|
| $(M+H)^+$ by mass spectrometry: | 1456.5 (calculated value: 1456.7) |
| HPLC elution time: | 19.71 minutes (Elution condition: the same as Example 27) |

Example 33 (Production of Arg-Pro-Arg-Leu-Ser-His-Lys-Gly-Pro-Met-Phe(Cl))

The desired product was obtained in the same manner as Example 32.

| | |
|---|---|
| $(M+H)^+$ by mass spectrometry: | 1359.6 (calculated value: 1359.7) |
| HPLC elution time: | 19.32 minutes (Elution condition: the same as Example 27) |

Example 34 (Production of Arg-Pro-Arg-Leu-Ser-His-Lys-Gly-Pro-Met-Cha)

Fmoc-Cha-O-Clt resin (0.49 mmol/g) that was obtained by introducing Fmoc-Cha-OH to a commercially available 2-chlorotrityl resin (Clt resin, 1.3 mmol/g) was used, and the desired product was obtained in the same manner as Example 31.

| | |
|---|---|
| $(M+H)^+$ by mass spectrometry: | 1331.7 (calculated value: 1331.8) |
| HPLC elution time: | 19.46 minutes (Elution condition: |

the same as Example 27)

Example 35 (Production of pGlu-Arg-Pro-Arg-Leu-Ser-His-Lys-Gly-Pro-Cha-Pro-Phe)

Fmoc-Cha-O-Clt resin (0.32 mmol/g) was used, and the desired product was obtained in the same manner as Example 28.

| | |
|---|---|
| $(M+H)^+$ by mass spectrometry: | 1555.8 (calculated value: 1555.9) |
| HPLC elution time: | 21.35 minutes (Elution condition: the same as Example 27) |

Example 36 (Production of Arg-Arg-Gln-Arg-Pro-Arg-Leu-Ser-His-Lys-Gly-Pro-Cha)

The desired product was obtained in the same manner as Example 34.

$(M+H)^+$ by mass spectrometry: 1640.7 (calculated value: 1640.9)

Example 37 (Production of Arg-Arg-Gln-Arg-Pro-Arg-Leu-Ser-His-Lys-Gly-Pro-Met-Pro-Phe(Cl))

Fmoc-Phe(Cl)-O-Clt resin (0.42 mmol/g) was used, and the desired product was obtained in the same manner as Example 27.

$(M+H)^+$ by mass spectrometry: 1897.5 (calculated value: 1897.7)

Example 38 (Production of Arg-Arg-Gln-Arg-Pro-Arg-Leu-Ser-His-Nle-Gly-Pro-Met-Pro-Phe(Cl))

The desired product was obtained in the same manner as Example 37.

$(M+H)^+$ by mass spectrometry: 1882.3 (calculated value: 1882.6)

Example 39 (Production of Arg-Arg-Gln-Arg-Pro-Arg-Leu-Ser-His-Nle-Gly-Pro-Met-Pro-Tyr(I))

Fmoc-Tyr(I)-O-Clt resin (0.31 mmol/g) that was obtained by introducing Fmoc-Tyr(I)-OH to a commercially available 2-chlorotrityl resin (Clt resin, 1.3 mmol/g) was used, and the desired product was obtained in the same manner as Example 38.

$(M+H)^+$ by mass spectrometry: 1989.7 (calculated value: 1989.9)

Example 40 (Production of Arg-Arg-Gln-Arg-Pro-Arg-Leu-Ser-His-Nle-Gly-Pro-Met-Pro-Tyr(Me))

Fmoc-Tyr(Me)-O-Clt resin (0.39 mmol/g) that was obtained by introducing Fmoc-Tyr(Me)-OH to a commercially available 2-chlorotrityl resin (Clt resin, 1.3 mmol/g) was used, and the desired product was obtained in the same manner as Example 38.

$(M+H)^+$ by mass spectrometry: 1878.1 (calculated value: 1878.3)

Experimental example 1

Each of peptides in Examples 3 and 4, and the corresponding natural peptide to the compound in Example 1 (a peptide of which Met was substituted for Nle, a non-natural amino acid) was dissolved with sterile distilled water to the concentration of $1 \times 10^{-3}$ M, and prepared by serial dilution using a medium containing 0.1% BSA for site sensor. A10 receptor cDNA introduced CHO cells prepared in the same manner as WO 99/33976 (Japanese Patent Application No. 220853-1998) were set in a workstation of a site-sensor, and diluted solution of the peptide was allowed to react with the cells 1 minute 2 seconds by switching channels to introduce the solution to one of the channels for the site sensor after the acidification rate of each cell became steady. Changes in acidification rate at the point of the maximum reaction of the cells were calculated by the setting the value at the basal level as 100%, and the results are shown in Figure 1.

Experimental example 2 Assay on the suppression activity on Forskolin stiumulated cAMP production

Six cells of CHO-A10 clone described in Example 7 in WO 99/33976 (Japanese Patent Application No. 220853-1998) were inoculated in 24-well tissue culture plates with $3 \times 10^5$ cells per well and were cultured overnight. 0.2 mM 3-isobutyl-1-methylxantine (IBMX) and Hank's balanced salt solution (HBSS) containing 0.05% bovine serum albumin were prepared as an assay buffer, each well was washed with 500 μl of the assay buffer twice, and preincubation was carried out at 37°C for 30 minutes. Subsequently, after washing with 500 μl of the assay buffer once, 500 μl each of the sample dissolved in the assay buffer added with 1 μM of forskolin were put into each well to incubate at 37°C for 30 minutes. Wells incubated with assay buffer added with no forskolin were prepared to know the basic cAMP production of cells (basal level), and similarly, wells incubated with assay buffer added with forskolin were also prepared to know the maximum cAMP production with stimulation of foskolin (maximum level). After completion of incubation, each well was washed with 500 μl of the assay buffer once, added with 500 μl of the lysis buffer IB included in the cAMP EIA system (Amersham Pharmacia Biotech) and extraction of cAMP was carried out. Following the prescription for the kit, a portion of 100 μl each of the extracts was used to assay. The level of suppression activity on cAMP production was calculated by taking the difference between the maximum level and cAMP in the wells that was added with a sample (amount of the suppression of cAMP production), and by taking it as a percentage relative to the increased production (difference between maximum level and basal level) in cAMP associated with forskolin stimulation, and $EC_{50}$ was determined based on the dose response curve.

Activities of compounds in examples determined by using the method in experimental Example 2 are given in Table 1.

Table 1

| Compounds | $EC_{50}$ (nM) |
|---|---|
| Compound in Example 2 | 0.41 |
| Compound in Example 3 | 0.28 |
| Compound in Example 4 | 0.19 |
| Compound in Example 10 | 0.25 |
| Compound in Example 29 | 0.48 |
| Compound in Example 30 | 0.28 |
| Compound in Example 31 | 0.30 |
| Compound in Example 32 | 0.20 |
| Compound in Example 33 | 0.10 |
| Compound in Example 34 | 0.37 |
| Compound in Example 35 | 0.34 |
| Compound in Example 36 | 0.16 |
| Compound in Example 37 | 0.14 |
| Compound in Example 38 | 0.35 |
| Corresponding natural peptide to the compound (a peptide of which Met was substituted for Nle, a non-natural amino acid) in Example 1 | 0.52 |

INDUSTRIAL APPLICABILITY

Since the peptides or the like of the present invention are involved in activities of central nervous functions, circulatory functions, cardiac functions, immune functions, digestive functions, metabolic functions, or reproductive organ functions, agonists or antagonists described above can be used as therapeutic or prophylactic drugs against disorders, such as: dementia including dementia associated with senile dementia, cerebrovascular dementia, dementia associated with retroplastic diseases of the systemic degeneration type (e.g. Alzheimer's disease, Parkinson's disease, Pick's disease, Huntington's disease), dementia associated with infective diseases (e.g. slow virus infections such as Creutzfeldt-Jakob disease), dementia associated with endocrine disease, metabolic disease or toxic disease (e.g. hypothyroidism, vitamin B12 deficiency disease, alcoholism, and intoxication with various drugs, metal or organic compound), dementia associated with neoplastic diseases such (e.g. brain tumor), and dementia associated with traumatopathy (e.g. chronic subdural hematoma); depression, ADHD (minimal brain dysfunction) syndrome; disturbance of consciousness; anxiety disorder; schizophrenia; phobia, growth hormone secretion disorder (e.g. gigantism, acromegaly), hyperphagia, polyphagia, hypercholesterolemia, hyperglyceridemia, hyperlipidemia, hyperprolactinemia, hypoglycemia, hypopituitarism, hypophysial dwarfism, diabetes (e.g. diabetic complication, diabetic nephropathy, diabetic neuropathy and diabetic retinopathy); cancer (e.g. breast cancer, lymphatic leukemia, lung cancer, bladder cancer, ovarian cancer and prostate cancer); pancreatitis, renal diseases (e.g. chronic renal failure, nephritis); Turner's syndrome; neuropathy; rheumatoid arthritis; spinal cord injury; transient ischemic attack; amyotrophic lateral sclerosis; acute myocardial infarct; spinocerebellar degeneration; fracture; injury; atopic dermatitis; osteoporosis; asthma; epilepsy; infertility; arteriosclerosis; emphysema pulmonum; pulmonary edema or hypogalactia. Also they can be applied as a sedative hypnotic, an agent that improves the nutritional status of post-operative patients, vasopressor and depressor drug.

In addition, the peptides of the present invention can be used as therapeutic or prophylactic drugs against AIDS (Acquired Immune Deficiency Syndrome) or the like.

SEQUENCE LISTING

<110> Takeda Chemical Industries, Ltd.
<120> Peptide derivative
<130> 2549WOOP
<150> JP 10-271626
<151> 1998-9-25
<160> 42
<210> 1
<211> 36
<212> PRT
<213> Artificial Sequence
<223> Xaa means Nle.
<400> 1

```
Leu Val Gln Pro Arg Gly Ser Arg Asn Gly Pro Gly Pro Trp Gln Gly
1               5                   10                  15
Gly Arg Arg Lys Phe Arg Arg Gln Arg Pro Arg Leu Ser His Lys Gly
            20                  25                  30
Pro Xaa Pro Phe
        35
```

<210> 2
<211> 36
<212> PRT
<213> Artificial Sequence
<223> Xaa means Nle.
<400> 2

Leu Val Gln Pro Arg Gly Ser Arg Asn Gly Pro Gly Pro Trp Gln Gly

1 5 10 15

Gly Arg Arg Lys Phe Arg Arg Gln Arg Pro Arg Leu Ser His Lys Gly

20 25 30

Pro Xaa Pro Tyr

35

<210> 3
<211> 13
<212> PRT
<213> Artificial Sequence
<223> Xaa on the 1st position means pGlu, Xaa on the 11th position means Nle.
<400> 3

Xaa Arg Pro Arg Leu Ser His Lys Gly Pro Xaa Pro Phe

1 5 10

<210> 4
<211> 13
<212> PRT
<213> Artificial Sequence
<223> Xaa on the 1st position means pGlu, Xaa on the 11th position means Nle.
<400> 4

Xaa Arg Pro Arg Leu Ser His Lys Gly Pro Xaa Pro Tyr

1 5 10

<210> 5
<211> 12
<212> PRT
<213> Artificial Sequence
<223> Xaa on the 1st position means pGlu, Xaa on the 11th position means Nle.
<400> 5

Xaa Arg Pro Arg Leu Ser His Lys Gly Pro Xaa Pro

1 5 10

<210> 6
<211> 11
<212> PRT
<213> Artificial Sequence
<223> Xaa on the 1st position means pGlu, Xaa on the 11th position means Nle.
<400> 6

Xaa Arg Pro Arg Leu Ser His Lys Gly Pro Xaa
1 5 10

<210> 7
<211> 12
<212> PRT
<213> Artificial Sequence
<223> Xaa on the 1st position means Ac-Arg, Xaa on the 10th position means Nle.
<400> 7

Xaa Pro Arg Leu Ser His Lys Gly Pro Xaa Pro Tyr
1 5 10

<210> 8
<211> 11
<212> PRT
<213> Artificial Sequence
<223> Xaa on the 1st position means Ac-Arg, Xaa on the 10th position means Nle.
<400> 8

Xaa Pro Arg Leu Ser His Lys Gly Pro Xaa Pro
1 5 10

<210> 9
<211> 10
<212> PRT
<213> Artificial Sequence
<223> Xaa on the 1st position means Ac-Arg, Xaa on the 10th position means Nle.
<400> 9

Xaa Pro Arg Leu Ser His Lys Gly Pro Xaa
1 5 10

<210> 10
<211> 13
<212> PRT
<213> Artificial Sequence
<223> Xaa on the 1st position means pGlu, Xaa on the 8th position means Lys(Ac).
<400> 10

Xaa Arg Pro Arg Leu Ser His Xaa Gly Pro Met Pro Phe
1 5 10

<210> 11
<211> 13
<212> PRT
<213> Artificial Sequence

<223> Xaa on the 1st position means pGlu, Xaa on the 8th position means Lys(Me).
<400> 11

Xaa Arg Pro Arg Leu Ser His Xaa Gly Pro Met Pro Phe
1 5 10

<210> 12
<211> 13
<212> PRT
<213> Artificial Sequence
<223> Xaa on the 1st position means pGlu, Xaa on the 8th position means Lys(Ac), Xaa on the 11th position means Nle.
<400> 12

Xaa Arg Pro Arg Leu Ser His Xaa Gly Pro Xaa Pro Phe
1 5 10

<210> 13
<211> 13
<212> PRT
<213> Artificial Sequence
<223> Xaa on the 1st position means pGlu, Xaa on the 8th position means Lys (Me), Xaa on the 11th position means Nle.
<400> 13

Xaa Arg Pro Arg Leu Ser His Xaa Gly Pro Xaa Pro Phe
1 5 10

<210> 14
<211> 13
<212> PRT
<213> Artificial Sequence
<223> Xaa on the 1st position means pGlu, Xaa on the 8th position means Lys(Tos), Xaa on the 11th position means Nle.
<400> 14

Xaa Arg Pro Arg Leu Ser His Xaa Gly Pro Xaa Pro Phe
1 5 10

<210> 15
<211> 13
<212> PRT
<213> Artificial Sequence
<223> Xaa on the 1st position means pGlu, Xaa on the 8th position means Arg(Tos), Xaa on the 11th position means Nle.
<400> 15

Xaa Arg Pro Arg Leu Ser His Xaa Gly Pro Xaa Pro Phe
1 5 10

<210> 16
<211> 13
<212> PRT
<213> Artificial Sequence
<223> Xaa on the 1st position means pGlu, Xaa on the 5th position means Nle, Xaa on the 11th position means Nle.
<400> 16

Xaa Arg Pro Arg Xaa Ser His Lys Gly Pro Xaa Pro Phe
1 5 10

<210> 17
<211> 13
<212> PRT
<213> Artificial Sequence
<223> Xaa on the 1st position means pGlu, Xaa on the 5th position means Nle, Xaa on the 11th position means Nle.
<400> 17

Xaa Arg Pro Arg Xaa Ser His Lys Gly Pro Xaa Pro Tyr
1 5 10

<210> 18
<211> 13
<212> PRT
<213> Artificial Sequence
<223> Xaa on the 1st position means pGlu, Xaa on the 11th position means Nle, Xaa on the 13th position means Thi.
<400> 18

Xaa Arg Pro Arg Leu Ser His Lys Gly Pro Xaa Pro Xaa
1 5 10

<210> 19
<211> 13
<212> PRT
<213> Artificial Sequence
<223> Xaa on the 1st position means pGlu, Xaa on the 11th position, means Nle, Xaa on the 13th position means Phg.
<400> 19

Xaa Arg Pro Arg Leu Ser His Lys Gly Pro Xaa Pro Xaa
1 5 10

<210> 20
<211> 13
<212> PRT
<213> Artificial Sequence

<223> Xaa on the 1st position means pGlu, Xaa on the 11th position means Nle, Xaa on the 13th position means Pya(2).
<400> 20

Xaa Arg Pro Arg Leu Ser His Lys Gly Pro Xaa Pro Xaa
1 5 10

<210> 21
<211> 12
<212> PRT
<213> Artificial Sequence
<223> Xaa on the 10th position means Nle.
<400> 21

Arg Pro Arg Leu Ser His Lys Gly Pro Nle Pro Tyr
1 5 10

<210> 22
<211> 36
<212> PRT
<213> Artificial Sequence
<223> Xaa on the 3rd position means Adi ($NH_2$), Xaa on the 34th position means Nle.
<400> 22

Leu Val Xaa Pro Arg Gly Ser Arg Asn Gly Pro Gly Pro Trp Gln Gly
1 5 10 15
Gly Arg Arg Lys Phe Arg Arg Gln Arg Pro Arg Leu Ser His Lys Gly
20 25 30
Pro Xaa Pro Phe
35

<210> 23
<211> 36
<212> PRT
<213> Artificial Sequence
<223> Xaa on the 3rd position means Lys(Ac), Xaa on the 34th position means Nle.
<400> 23

Leu Val Xaa Pro Arg Thr Ser Arg Thr Gly Pro Gly Ala Trp Gln Gly
1 5 10 15
Gly Arg Arg Lys Phe Arg Arg Gln Arg Pro Arg Leu Ser His Lys Gly
20 25 30
Pro Xaa Pro Tyr
35

<210> 24
<211> 37
<212> PRT
<213> Artificial Sequence
<223> Xaa on 35th position means Nle.
<400> 24

Tyr Leu Val Lys Pro Arg Thr Ser Arg Thr Gly Pro Gly Ala Trp Gln
1 5 10 15
Gly Gly Arg Arg Lys Phe Arg Arg Gln Arg Pro Arg Leu Ser His Lys
20 25 30
Gly Pro Xaa Pro Phe
35

<210> 25
<211> 13
<212> PRT
<213> Artificial Sequence
<223> Xaa on the 1st position means Z-pGlu, Xaa on the 8th position means Lys(Ac), Xaa on the 11th position means Nle.
<400> 25

Xaa Arg Pro Arg Leu Ser His Xaa Gly Pro Xaa Pro Phe
1 5 10

<210> 26
<211> 380
<212> PRT
<213> Unknown
<220>
<223>
<400> 26

Met Glu Glu Gly Gly Asp Phe Asp Asn Tyr Tyr Gly Ala Asp Asn Gln
1 5 10 15
Ser Glu Cys Glu Tyr Thr Asp Trp Lys Ser Ser Gly Ala Leu Ile Pro
20 25 30
Ala Ile Tyr Met Leu Val Phe Leu Leu Gly Thr Thr Gly Asn Gly Leu
35 40 45
Val Leu Trp Thr Val Phe Arg Ser Ser Arg Glu Lys Arg Arg Ser Ala
50 55 60
Asp Ile Phe Ile Ala Ser Leu Ala Val Ala Asp Leu Thr Phe Val Val
65 70 75 80
Thr Leu Pro Leu Trp Ala Thr Tyr Thr Tyr Arg Asp Tyr Asp Trp Pro
85 90 95
Phe Gly Thr Phe Phe Cys Lys Leu Ser Ser Tyr Leu Ile Phe Val Asn
100 105 110
Met Tyr Ala Ser Val Phe Cys Leu Thr Gly Leu Ser Phe Asp Arg Tyr
115 120 125
Leu Ala Ile Val Arg Pro Val Ala Asn Ala Arg Leu Arg Leu Arg Val
130 135 140
Ser Gly Ala Val Ala Thr Ala Val Leu Trp Val Leu Ala Ala Leu Leu
145 150 155 160

Ala Met Pro Val Met Val Leu Arg Thr Thr Gly Asp Leu Glu Asn Thr
165 170 175

Thr Lys Val Gln Cys Tyr Met Asp Tyr Ser Met Val Ala Thr Val Ser
180 185 190

Ser Glu Trp Ala Trp Glu Val Gly Leu Gly Val Ser Ser Thr Thr Val
195 200 205

Gly Phe Val Val Pro Phe Thr Ile Met Leu Thr Cys Tyr Phe Phe Ile
210 215 220

Ala Gln Thr Ile Ala Gly His Phe Arg Lys Glu Arg Ile Glu Gly Leu
225 230 235 240

Arg Lys Arg Arg Arg Leu Leu Ser Ile Ile Val Val Leu Val Val Thr
245 250 255

Phe Ala Leu Cys Trp Met Pro Tyr His Leu Val Lys Thr Leu Tyr Met
260 265 270

Leu Gly Ser Leu Leu His Trp Pro Cys Asp Phe Asp Leu Phe Leu Met
275 280 285

Asn Ile Phe Pro Tyr Cys Thr Cys Ile Ser Tyr Val Asn Ser Cys Leu
290 295 300

Asn Pro Phe Leu Tyr Ala Phe Phe Asp Pro Arg Phe Arg Gln Ala Cys
305 310 315 320

Thr Ser Met Leu Cys Cys Gly Gln Ser Arg Cys Ala Gly Thr Ser His
325 330 335

Ser Ser Ser Gly Glu Lys Ser Ala Ser Tyr Ser Ser Gly His Ser Gln
340 345 350

Gly Pro Gly Pro Asn Met Gly Lys Gly Gly Glu Gln Met His Glu Lys
355 360 365

Ser Ile Pro Tyr Ser Gln Glu Thr Leu Val Val Asp
370 375 380

<210> 27
<211> 1140
<212> DNA

<213> Unknown
<220>
<223>
<400> 27

```
ATGGAGGAAG GTGGTGATTT TGACAACTAC TATGGGGCAG ACAACCAGTC TGAGTGTGAG     60
TACACAGACT GGAAATCCTC GGGGGCCCTC ATCCCTGCCA TCTACATGTT GGTCTTCCTC    120
CTGGGCACCA CGGGAAACGG TCTGGTGCTC TGGACCGTGT TTCGGAGCAG CCGGGAGAAG    180
AGGCGCTCAG CTGATATCTT CATTGCTAGC CTGGCGGTGG CTGACCTGAC CTTCGTGGTG    240
ACGCTGCCCC TGTGGGCTAC CTACACGTAC CGGGACTATG ACTGGCCCTT TGGGACCTTC    300
TTCTGCAAGC TCAGCAGCTA CCTCATCTTC GTCAACATGT ACGCCAGCGT CTTCTGCCTC    360
ACCGGCCTCA GCTTCGACCG CTACCTGGCC ATCGTGAGGC CAGTGGCCAA TGCTCGGCTG    420
AGGCTGCGGG TCAGCGGGGC CGTGGCCACG GCAGTTCTTT GGGTGCTGGC CGCCCTCCTG    480
GCCATGCCTG TCATGGTGTT ACGCACCACC GGGGACTTGG AGAACACCAC TAAGGTGCAG    540
TGCTACATGG ACTACTCCAT GGTGGCCACT GTGAGCTCAG AGTGGGCCTG GGAGGTGGGC    600
CTTGGGGTCT CGTCCACCAC CGTGGGCTTT GTGGTGCCCT TCACCATCAT GCTGACCTGT    660
TACTTCTTCA TCGCCCAAAC CATCGCTGGC CACTTCCGCA AGGAACGCAT CGAGGGCCTG    720
CGGAAGCGGC GCCGGCTGCT CAGCATCATC GTGGTGCTGG TGGTGACCTT TGCCCTGTGC    780
TGGATGCCCT ACCACCTGGT GAAGACGCTG TACATGCTGG GCAGCCTGCT GCACTGGCCC    840
TGTGACTTTG ACCTCTTCCT CATGAACATC TTCCCCTACT GCACCTGCAT CAGCTACGTC    900
AACAGCTGCC TCAACCCCTT CCTCTATGCC TTTTTCGACC CCCGCTTCCG CCAGGCCTGC    960
ACCTCCATGC TCTGCTGTGG CCAGAGCAGG TGCGCAGGCA CCTCCCACAG CAGCAGTGGG   1020
GAGAAGTCAG CCAGCTACTC TTCGGGGCAC AGCCAGGGGC CCGGCCCCAA CATGGGCAAG   1080
GGTGGAGAAC AGATGCACGA GAAATCCATC CCCTACAGCC AGGAGACCCT TGTGGTTGAC   1140
```

<210> 28
<211> 13
<212> PRT
<213> Artificial Sequence
<223> Xaa on the 13th position means Met(0).
<400> 28

```
Arg Arg Gln Arg Pro Arg Leu Ser His Lys Gly Pro Xaa
1               5                   10          13
```

<210> 29
<211> 15
<212> PRT
<213> Artificial Sequence

<223> Xaa on the 13th position means Nle.
<400> 29

Arg Arg Gln Arg Pro Arg Leu Ser His Lys Gly Pro Xaa Pro Tyr
1 5 10 15

<210> 30
<211> 12
<212> PRT
<213> Artificial Sequence
<223> Xaa on the 1st position means pGlu, Xaa on the 12th position means Phe(Cl).
<400> 30

Xaa Arg Pro Arg Leu Ser His Lys Gly Pro Met Xaa
1 5 10 12

<210> 31
<211> 13
<212> PRT
<213> Artificial Sequence
<223> Xaa on the 1st position means pGlu, Xaa on the 13th position means Phe(Cl).
<400> 31

Xaa Arg Pro Arg Leu Ser His Lys Gly Pro Met Pro Xaa
1 5 10 13

<210> 32
<211> 12
<212> PRT
<213> Artificial Sequence
<223> Xaa on the 12th position means Nal(2).
<400> 32

Arg Pro Arg Leu Ser His Lys Gly Pro Met Pro Xaa
1 5 10 12

<210> 33
<211> 11
<212> PRT
<213> Artificial Sequence
<223> Xaa on the 11th position means Nal(2).
<400> 33

Arg Pro Arg Leu Ser His Lys Gly Pro Met Xaa
1 5 10 11

<210> 34

<211> 13
<212> PRT
<213> Artificial Sequence
<223> Xaa on the 13th position means Phe(Cl).
<400> 34

Arg Pro Arg Leu Ser His Lys Gly Pro Met Pro Xaa
1 5 10 13

<210> 35
<211> 11
<212> PRT
<213> Artificial Sequence
<223> Xaa on the 11th position means Phe(Cl).
<400> 35

Arg Pro Arg Leu Ser His Lys Gly Pro Met Xaa
1 5 10 11

<210> 36
<211> 11
<212> PRT
<213> Artificial Sequence
<223> Xaa on the 11th position means Cha.
<400> 36

Arg Pro Arg Leu Ser His Lys Gly Pro Met Xaa
1 5 10 11

<210> 37
<211> 13
<212> PRT
<213> Artificial Sequence
<223> Xaa on the 1st position means pGlu, Xaa on the 11th position means Cha.
<400> 37

Xaa Arg Pro Arg Leu Ser His Lys Gly Pro Xaa Pro Phe
1 5 10 13

<210> 38
<211> 13
<212> PRT
<213> Artificial Sequence
<223> Xaa on the 13th position means Cha.
<400> 38

Arg Arg Gln Arg Pro Arg Leu Ser His Lys Gly Pro Xaa
1 5 10 13

<210> 39
<211> 15
<212> PRT
<213> Artificial Sequence
<223> Xaa on the 15th position means Phe(Cl).
<400> 39

Arg Arg Gln Arg Pro Arg Leu Ser His Lys Gly Pro Met Pro Xaa
1 5 10 15

<210> 40
<211> 15
<212> PRT
<213> Artificial Sequence
<223> Xaa on the 10th position means Nle, Xaa on the 15th position means Phe(Cl).
<400> 40

Arg Arg Gln Arg Pro Arg Leu Ser His Xaa Gly Pro Met Pro Xaa
1 5 10 15

<210> 41
<211> 15
<212> PRT
<218> Artificial Sequence
<228> Xaa on the 10th position means Nle, Xaa on the 15th position means Tyr(I).
<400> 41

Arg Arg Gln Arg Pro Arg Leu Ser His Xaa Gly Pro Met Pro Xaa
1 5 10 15

<210> 42
<211> 15
<212> PRT
<213> Artificial Sequence
<223> Xaa on the 10th position means Nle, Xaa on the 15th position means Tyr(Me).
<400> 42

Arg Arg Gln Arg Pro Arg Leu Ser His Nle Gly Pro Met Pro Xaa
1 5 10 15

## Claims

**1.** A peptide represented by the formula:

$$X^1\text{-Arg-Pro-Arg-}X^2\text{-Ser-His-}X^3\text{-Gly-Pro-}X^4\text{-}X^5$$

[wherein $X^1$ represents a hydrogen atom or an amino acid residue comprising 1 to 25 amino acids each of which, whether identical or not, has a side chain which may be substituted, or a peptide chain; $X^2$ represents a neutral amino acid residue having a side chain which may be substituted; $X^3$ represents a neutral amino acid residue having a side chain which may be substituted, an aromatic amino acid residue having a side chain which may be substituted or a basic amino acid residue having a side chain which may be substituted; $X^4$ represents a bond or a neutral or aromatic amino acid residue having a side chain which may be substituted; $X^5$ represents ① an amino acid residue having a side chain which may be substituted or an amino acid derivative with the C-terminal carboxyl group reduced to a hydroxymethyl group or a formyl group, ② a hydroxyl group or ③ a dipeptide chain comprising an amino acid residue having a side chain which may be substituted and an amino acid residue having a side chain which may be substituted, bound together, or a peptide derivative with the C-terminal carboxyl group reduced to a hydroxymethyl group or a formyl group; and a side chain in each amino acid residue in -Arg-Pro-Arg-, -Ser-His- or -Gly-Pro- in the formula may be substituted; except the case where $X^2$ represents Leu, $X^3$ represents Lys, $X^4$ represents Met, $X^5$ represents ① Pro or ② Pro-Phe, and -Arg-Pro-Arg- in the formula represents unsubstituted -Arg-Pro-Arg-, -Ser-His-represents unsubstituted -Ser-His-, and -Gly-Pro-represents unsubstituted -Gly-Pro-], or an ester thereof, or a salt thereof.

**2.** A peptide, as claimed in claim 1, wherein $X^1$ is an amino acid residue having a side chain which may be substituted, or an ester thereof or a salt thereof.

**3.** A peptide as claimed in claim 1, wherein $X^1$ is pGlu which may be substituted or Gln having a side chain which may be substituted, or an ester thereof or a salt thereof.

**4.** A peptide as claimed in claim 1, wherein $X^1$ is represented by the formula $Y^1$-$Y^2$ (wherein $Y^1$ represents an amino acid residue comprising 1 to 17 amino acids each of which, whether identical or not, has a side chain which may be substituted, or a peptide chain, and $Y^2$ represents an amino acid residue comprising 1 to 8 amino acids each of which, whether identical or not, has a side chain which may be substituted, or a peptide chain), or an ester thereof or a salt thereof.

**5.** A peptide as claimed in claim 4, wherein $Y^2$ is a peptide chain represented by ① the formula $B^1$-$B^2$-$B^3$-$B^4$-$B^5$-$B^6$-$B^7$-$B^8$ (wherein each of $B^1$ through $B^8$, whether identical or not, represents an amino acid residue having a side chain which may be substituted), ② the formula $B^2$-$B^3$-$B^4$-$B^5$-$B^6$-$B^7$-$B^8$ (wherein the symbols have the same definitions as those given above), ③ the formula $B^3$-$B^4$-$B^5$-$B^6$-$B^7$-$B^8$ (wherein the symbols have the same definitions as those given above), ④ the formula $B^4$-$B^5$-$B^6$-$B^7$-$B^8$ (wherein the symbols have the same definitions as those given above), ⑤ the formula $B^5$-$B^6$-$B^7$-$B^8$ (wherein the symbols have the same definitions as those given above), ⑥ the formula $B^6$-$B^7$-$B^8$ (wherein the symbols have the same definitions as those given above), ⑦ the formula $B^7$-$B^8$, (wherein the symbols have the same definitions as those given above), or ⑧ the formula $B^8$ (wherein $B^8$ has the same definition as that given above), or an ester thereof or a salt thereof.

**6.** A peptide as claimed in claim 5, wherein $B^1$ is a neutral amino acid residue having a side chain which may be substituted, or an ester thereof or a salt thereof.

**7.** A peptide as claimed in claim 6, wherein $B^1$ is Gly which may be substituted, or an ester thereof or a salt thereof.

**8.** A peptide as claimed in claim 5, wherein each of $B^2$, $B^3$ and $B^4$, whether identical or not, is a basic amino acid residue having a side chain which may be substituted, or an ester thereof or a salt thereof.

**9.** A peptide as claimed in claim 5, wherein $B^5$ is an amino acid residue having an aromatic side chain, or an ester thereof or a salt thereof.

**10.** A peptide as claimed in claim 5, wherein each of $B^6$ and $B^7$, whether identical or not, is a basic amino acid residue having a side chain which may be substituted, or an ester thereof or a salt thereof.

**11.** A peptide as claimed in claim 5, wherein $B^8$ is Gln having a side chain which may be substituted, or an ester thereof or a salt thereof.

**12.** A peptide as claimed in claim 4, wherein $Y^1$ is an amino acid residue or peptide chain represented by (a) the formula $A^1$-$A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein each of $A^1$ to $A^{17}$, whether identical or not, represents an amino acid residue having a side chain which may be substituted), (b) the formula $A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (c) the formula $A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (d) the formula $A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (e) the formula $A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein each symbols have the same definitions as those given above), (f) the formula $A^6$-$A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (g) the formula $A^7$-$A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (h) the formula $A^8$-$A^9$-$A^{10}$-$A^{11}$-$A^{12}$-$A^{13}$-$A^{14}$-$A^{15}$-$A^{16}$-$A^{17}$ (wherein the symbols have the same definitions as those given above), (i) the formula $A^9$-$A^{10}$-$A^{11}$-$A^{12}$-$A^{13}$-$A^{14}$-$A^{15}$-$A^{16}$-$A^{17}$ (wherein the symbols have the same definitions as those given above), (j) the formula $A^{10}$-$A^{11}$-$A^{12}$-$A^{13}$-$A^{14}$-$A^{15}$-$A^{16}$-$A^{17}$ (wherein the symbols have the same definitions as those given above), (k) the formula $A^{11}$-$A^{12}$-$A^{13}$-$A^{14}$-$A^{15}$-$A^{16}$-$A^{17}$ (wherein the symbols have the same definitions as those given above), (l) the formula $A^{12}$-$A^{13}$-$A^{14}$-$A^{15}$-$A^{16}$-$A^{17}$ (wherein the symbols have the same definitions as those given above), (m) the formula $A^{13}$-$A^{14}$-$A^{15}$-$A^{16}$-$A^{17}$ (wherein the symbols have the same definitions as those given above), (n) the formula $A^{14}$-$A^{15}$-$A^{16}$-$A^{17}$ (wherein the symbols have the same definitions as those given above), (o) the formula $A^{15}$-$A^{16}$-$A^{17}$ (wherein the symbols have the same definitions as those given above), (p) the formula $A^{16}$-$A^{17}$ (wherein the symbols have the same definitions as those given above), or (q) $A^{17}$ (wherein $A^{17}$ has the same definition as that given above), or an ester thereof or a salt thereof.

**13.** An amino acid residue or peptide as claimed in claim 12, wherein $A^1$ is an amino acid residue having an aromatic side chain, or an ester thereof or a salt thereof.

**14.** A peptide as claimed in claim 12, wherein $A^2$ and $A^3$ are neutral amino acid residues having a side chain which may be substituted, or an ester thereof or a salt thereof.

**15.** A peptide as claimed in claim 12, wherein $A^4$ is a neutral or a basic L-amino acid residue having a side chain which may be substituted, or an ester thereof or a salt thereof.

**16.** A peptide as claimed in claim 12, wherein $A^5$ is Pro having a side chain which may be substituted, or an ester thereof or a salt thereof.

**17.** A peptide as claimed in claim 12, wherein each of $A^6$ and $A^9$, whether identical or not, is a basic amino acid residue having a side chain which may be substituted, or an ester thereof or a salt thereof.

**18.** A peptide as claimed in claim 12, wherein each of $A^7$ and $A^{10}$, whether identical or not, is an amino acid residue having a hydroxy group in a side chain thereof or a neutral amino acid residue having a side chain which may be substituted, or an ester thereof or a salt thereof.

**19.** A peptide as claimed in claim 12, wherein $A^8$ is an amino acid residue having a hydroxy group in a side chain thereof, or an ester thereof or a salt thereof.

**20.** A peptide as claimed in claim 12, wherein $A^8$ is Ser, Pro or Hyp, or an ester thereof or a salt thereof.

**21.** A peptide as claimed in claim 12, wherein $A^{10}$ is an amino acid residue having a hydroxy group in a side chain thereof, or an ester thereof or a salt thereof.

**22.** A peptide as claimed in claim 12, wherein $A^{11}$ through $A^{14}$, whether identical or not, is a neutral amino acid residue having a side chain which may be substituted, or an ester thereof or a salt thereof.

**23.** A peptide as claimed in claim 12, wherein $A^{15}$ is an amino acid residue having an aromatic side chain, or an ester thereof or a salt thereof.

**24.** A peptide as claimed in claim 23, wherein $A^{15}$ is Trp, or an ester thereof or a salt thereof.

**25.** An amino acid residue or peptide as claimed in claim 12, wherein each of $A^{16}$ and $A^{17}$, whether identical or not, represents a neutral amino acid residue having a side chain which may be substituted, or an ester thereof or a salt thereof.

**26.** A pharmaceutical containing a peptide, as claimed in claim 1, or an ester thereof or a salt thereof.

**27.** A pharmaceutical as claimed in claim 26 which is a central nervous function regulator, a circulatory function regulator, a cardiac function regulator, an immune function regulator, a digestive function regulator, a metabolic function regulator or a reproductive organ function regulator.

**28.** A pharmaceutical as claimed in claim 26 which is an agonist of a protein containing an amino acid sequence shown by Sequence ID No.:26 or a salt thereof.

**Patentansprüche**

**1.** Peptid mit der Formel:

$$X^1\text{-Arg-Pro-Arg-}X^2\text{-Ser-His-}X^3\text{-Gly-Pro-}X^4\text{-}X^5$$

[worin $X^1$ ein Wasserstoffatom oder einen Aminosäurerest mit 1 bis 25 Aminosäuren bedeutet, von denen jede, unabhängig davon, ob sie identisch sind oder nicht, eine Seitenkette, die substituiert sein kann, oder eine Peptidkette aufweist; $X^2$ einen neutralen Aminosäurerest mit einer Seitenkette, die substituiert sein kann, bedeutet; $X^3$ einen neutralen Aminosäurerest mit einer Seitenkette, die substituiert sein kann, einen aromatischen Aminosäurerest mit einer Seitenkette, die substituiert sein kann, oder einen basischen Aminosäurerest mit einer Seitenkette, die substituiert sein kann, bedeutet; $X^4$ eine Bindung oder einen neutralen oder aromatischen Aminosäurerest mit einer Seitenkette, die substituiert sein kann, bedeutet; $X^5$ ① einen Aminosäurerest mit einer Seitenkette, die substituiert sein kann, oder ein Aminosäurederivat, dessen C-terminale Carboxylgruppe zu einer Hydroxymethylgruppe oder Formylgruppe reduziert ist, ② eine Hydroxylgruppe oder ③ eine Dipeptidkette mit einem Aminosäurerest mit einer Seitenkette, die substituiert sein kann, und einem Aminosäurerest mit einer Seitenkette, die substituiert sein kann, die aneinander gebunden sind, oder ein Peptidderivat, dessen C-terminale Carboxylgruppe zu einer Hydroxymethylgruppe oder Formylgruppe reduziert ist, bedeutet; und wobei eine Seitenkette in jedem Aminosäurerest in -Arg-Pro-Arg-, -Ser-His- oder -Gly-Pro- in der Formel substituiert sein kann; ausgenommen den Fall, worin $X^2$ Leu bedeutet, $X^3$ Lys bedeutet, $X^4$ Met bedeutet, $X^5$ ① Pro oder ② Pro-Phe bedeutet, und -Arg-Pro-Arg- in der Formel unsubstituiertes -Arg-Pro-Arg- bedeutet, -Ser-His- unsubstituiertes -Ser-His- bedeutet und -Gly-Pro- unsubstituiertes -Gly-Pro- bedeutet] oder ein Ester davon oder ein Salz davon.

**2.** Peptid nach Anspruch 1, worin $X^1$ ein Aminosäurerest mit einer Seitenkette ist, die substituiert sein kann, oder ein Ester davon oder ein Salz davon.

**3.** Peptid nach Anspruch 1, worin $X^1$ pGlu ist, das substituiert sein kann, oder Gln mit einer Seitenkette ist, die substituiert sein kann, oder ein Ester davon oder ein Salz davon.

**4.** Peptid nach Anspruch 1, worin $X^1$ durch die Formel $Y^1$-$Y^2$ dargestellt wird (worin $Y^1$ einen Aminosäurerest mit 1 bis 17 Aminosäuren, von denen jede, unabhängig davon, ob sie identisch sind oder nicht, eine Seitenkette hat, die substituiert sein kann, oder eine Peptidkette bedeutet, und $Y^2$ einen Aminosäurerest mit 1 bis 8 Aminosäuren, von denen jede, unabhängig davon, ob sie identisch sind oder nicht, eine Seitenkette aufweist, die substituiert sein kann, oder eine Peptidkette bedeutet), oder ein Ester davon oder ein Salz davon.

**5.** Peptid nach Anspruch 4, worin $Y^2$ eine Peptidkette ist, die durch ① die Formel $B^1$-$B^2$-$B^3$-$B^4$-$B^5$-$B^6$-$B^7$-$B^8$ (worin jeder der Reste $B^1$ bis $B^8$, unabhängig davon, ob sie identisch sind oder nicht, einen Aminosäurerest bedeutet mit einer Seitenkette, die substituiert sein kann), ② die Formel $B^2$-$B^3$-$B^4$-$B^5$-$B^6$-$B^7$-$B^8$ (worin die Symbole die gleichen Bedeutungen, wie oben angegeben, haben), ③ die Formel $B^3$-$B^4$-$B^5$-$B^6$-$B^7$-$B^8$ (worin die Symbole die gleichen Definitionen, wie oben angegeben, haben), ④ die Formel $B^4$-$B^5$-$B^6$-$B^7$-$B^8$ (worin die Symbole die gleichen Definitionen, wie oben angegeben, haben), ⑤ die Formel $B^5$-$B^6$-$B^7$-$B^8$ (worin die Symbole die gleichen Definitionen, wie oben angegeben, haben), ⑥ die Formel $B^6$-$B^7$-$B^8$ (worin die Symbole die gleichen Definitionen, wie oben angegeben, haben), ⑦ die Formel $B^7$-$B^6$ (worin die Symbole die gleichen Definitionen, wie oben angegeben, haben) oder ⑧ die Formel $B^8$ (worin $B^8$ die gleiche Definition, wie oben angegeben, hat) dargestellt wird, oder ein Ester davon oder ein Salz davon.

**6.** Peptid nach Anspruch 5, worin $B^1$ ein neutraler Aminosäurerest mit einer Seitenkette ist, die substituiert sein kann, oder ein Ester davon oder ein Salz davon.

**7.** Peptid nach Anspruch 6, worin $B^1$ Gly ist, das substituiert sein kann, oder ein Ester davon oder ein Salz davon.

**8.** Peptid nach Anspruch 5, worin jeder der Reste $B^2$, $B^3$ und $B^4$, unabhängig davon, ob sie identisch sind oder nicht, ein basischer Aminosäurerest ist mit einer Seitenkette, die substituiert sein kann, oder ein Ester davon oder ein Salz davon.

**9.** Peptid nach Anspruch 5, worin $B^5$ ein Aminosäurerest ist mit einer aromatischen Seitenkette, oder ein Ester davon oder ein Salz davon.

**10.** Peptid nach Anspruch 5, worin jeder der Reste $B^6$ und $B^7$, unabhängig davon, ob sie identisch sind oder nicht, ein basischer Aminosäurerest ist mit einer Seitenkette, die substituiert sein kann, oder ein Ester davon oder ein Salz davon.

**11.** Peptid nach Anspruch 5, worin $B^8$ Gln ist mit einer Seitenkette, die substituiert sein kann, oder ein Ester davon oder ein Salz davon.

**12.** Peptid nach Anspruch 4, worin $Y^1$ ein Aminosäurerest oder eine Peptidkette ist, die dargestellt wird durch (a) die Formel $A^1$-$A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (worin jeder der Reste $A^1$ bis $A^{17}$, unabhängig davon, ob sie identisch sind oder nicht, einen Aminosäurerest bedeutet mit einer Seitenkette, die substituiert sein kann), (b) die Formel $A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (worin die Symbole die gleichen Definitionen, wie oben angegeben, haben), (c) die Formel $A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (worin die Symbole die gleichen Definitionen, wie oben angegeben, haben), (d) die Formel $A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (worin die Symbole die gleichen Definitionen, wie oben angegeben, haben), (e) die Formel $A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ (worin die Symbole die gleichen Definitionen, wie oben angegeben, haben), (f) die Formel $A^6$-$A^7$-$A^8$-$A^9$-$A^{10}$-$A^{11}$-$A^{12}$-$A^{13}$-$A^{14}$-$A^{15}$-$A^{16}$-$A^{17}$ (worin die Symbole die gleichen Definitionen, wie oben angegeben, haben), (g) die Formel $A^7$-$A^8$-$A^9$-$A^{10}$-$A^{11}$-$A^{12}$-$A^{13}$-$A^{14}$-$A^{15}$-$A^{16}$-$A^{17}$ (worin die Symbole die gleichen Definitionen, wie oben angegeben, haben) , (h) die Formel $A^8$-$A^9$-$A^{10}$-$A^{11}$-$A^{12}$-$A^{13}$-$A^{14}$-$A^{15}$-$A^{16}$-$A^{17}$ (worin die Symbole die gleichen Definitionen, wie oben angegeben, haben), (i) die Formel $A^9$-$A^{10}$-$A^{11}$-$A^{12}$-$A^{13}$-$A^{14}$-$A^{15}$-$A^{16}$-$A^{17}$ (worin die Symbole die gleichen Definitionen, wie oben angegeben, haben), (j) die Formel $A^{10}$-$A^{11}$-$A^{12}$-$A^{13}$-$A^{14}$-$A^{15}$-$A^{16}$-$A^{17}$ (worin die Symbole die gleichen Definitionen, wie oben angegeben, haben), (k) die Formel $A^{11}$-$A^{12}$-$A^{13}$-$A^{14}$-$A^{15}$-$A^{16}$-$A^{17}$ (worin die Symbole die gleichen Definitionen, wie oben angegeben, haben), (l) die Formel $A^{12}$-$A^{13}$-$A^{14}$-$A^{15}$-$A^{16}$-$A^{17}$ (worin die Symbole die gleichen Definitionen, wie oben angegeben, haben), (m) die Formel $A^{13}$-$A^{14}$-$A^{15}$-$A^{16}$-$A^{17}$ (worin die Symbole die gleichen Definitionen, wie oben angegeben, haben), (n) die Formel $A^{14}$-$A^{15}$-$A^{16}$-$A^{17}$ (worin die Symbole die gleichen Definitionen, wie oben angegeben, haben), (o) die Formel $A^{15}$-$A^{16}$-$A^{17}$ (worin die Symbole die gleichen Definitionen, wie oben angegeben, haben), (p) die Formel $A^{16}$-$A^{17}$ (worin die Symbole die gleichen Definitionen, wie oben angegeben, haben) oder (q) $A^{17}$ (worin $A^{17}$ die gleiche Definition, wie oben angegeben, hat), oder ein Ester davon oder ein Salz davon.

**13.** Aminosäurerest oder Peptid nach Anspruch 12, worin $A^1$ ein Aminosäurerest mit einer aromatischen Seitenkette ist, oder ein Ester davon oder ein Salz davon.

**14.** Peptid nach Anspruch 12, worin $A^2$ und $A^3$ neutrale Aminosäurereste sind mit einer Seitenkette, die substituiert sein kann, oder ein Ester davon oder ein Salz davon.

**15.** Peptid nach Anspruch 12, worin $A^4$ ein neutraler oder basischer L-Aminosäurerest ist mit einer Seitenkette, die substituiert sein kann, oder ein Ester davon oder ein Salz davon.

**16.** Peptid nach Anspruch 12, worin $A^5$ Pro ist mit einer Seitenkette, die substituiert sein kann, oder ein Ester davon oder ein Salz davon.

**17.** Peptid nach Anspruch 12, worin jeder der Reste $A^6$ und $A^9$, unabhängig davon, ob sie identisch sind oder nicht, ein basischer Aminosäurerest ist mit einer Seitenkette, die substituiert sein kann, oder ein Ester davon oder ein Salz davon.

**18.** Peptid nach Anspruch 12, worin jeder der Reste $A^7$ und $A^{10}$, unabhängig davon, ob sie identisch sind oder nicht, ein Aminosäurerest ist mit einer Hydroxygruppe in einer Seitenkette davon oder ein neutraler Aminosäurerest ist mit einer Seitenkette, die substituiert sein kann, oder ein Ester davon oder ein Salz davon.

**19.** Peptid nach Anspruch 12, worin $A^8$ ein Aminosäurerest mit einer Hydroxygruppe in einer Seitenkette davon ist oder ein Ester davon oder ein Salz davon.

**20.** Peptid nach Anspruch 12, worin $A^8$ Ser, Pro oder Hyp ist, oder ein Ester davon oder ein Salz davon.

**21.** Peptid nach Anspruch 12, worin $A^{10}$ ein Aminosäurerest mit einer Hydroxygruppe in einer Seitenkette davon ist, oder ein Ester davon oder ein Salz davon.

**22.** Peptid nach Anspruch 12, worin $A^{11}$ bis $A^{14}$, unabhängig davon, ob sie identisch sind oder nicht, neutrale Aminosäurereste sind mit einer Seitenkette, die substituiert sein kann, oder ein Ester davon oder ein Salz davon.

**23.** Peptid nach Anspruch 12, worin $A^{15}$ ein Aminosäurerest mit einer aromatischen Seitenkette ist, oder ein Ester davon oder ein Salz davon.

**24.** Peptid nach Anspruch 23, worin $A^{15}$ Trp ist, oder ein Ester davon oder ein Salz davon.

**25.** Aminosäurerest oder Peptid nach Anspruch 12, worin jeder der Reste $A^{16}$ und $A^{17}$, unabhängig davon, ob sie identisch sind oder nicht, einen neutralen Aminosäurerest mit einer Seitenkette bedeutet, die substituiert sein kann, oder ein Ester davon oder ein Salz davon.

**26.** Arzneimittel enthaltend ein Peptid nach Anspruch 1 oder einen Ester davon oder ein Salz davon.

**27.** Arzneimittel nach Anspruch 26, das ein Regulator für die zentrale Nervenfunktion, ein Regulator für die Kreislauffunktion, ein Regulator für die Herzfunktion, ein Regulator für die Immunfunktion, ein Regulator für die Verdauungsfunktion, ein Regulator für die metabolische Funktion oder ein Regulator für die Funktion der reproduktiven Organe ist.

**28.** Arzneimittel nach Anspruch 26, das ein Agonist eines Proteins ist, das eine Aminosäuresequenz enthält, die in SEQ ID Nr. 26 gezeigt wird, oder ein Salz davon.

**Revendications**

**1.** Peptide représenté par la formule :

$$X^1\text{–Arg–Pro–Arg–}X^2\text{–Ser–His–}X^3\text{–Gly–Pro–}X^4\text{–}X^5$$

dans laquelle

$X^1$ représente un atome d'hydrogène, ou un résidu d'acide aminé comportant 1 à 25 résidus d'acides aminés, identiques ou non, dont chacun comporte une chaîne latérale éventuellement substituée, ou une chaîne peptidique ;
$X^2$ représente un résidu d'acide aminé neutre à chaîne latérale éventuellement substituée ;
$X^3$ représente un résidu d'acide aminé neutre à chaîne latérale éventuellement substituée, un résidu d'acide aminé aromatique à chaîne latérale éventuellement substituée, ou un résidu d'acide aminé basique à chaîne latérale éventuellement substituée ;
$X^4$ représente une liaison ou un résidu d'acide aminé aromatique ou neutre à chaîne latérale éventuellement substituée ;
et $X^5$ représente

1) le résidu d'un acide aminé à chaîne latérale éventuellement substituée, ou d'un dérivé d'acide aminé dont le groupe carboxyle C-termi-nal a été réduit en un groupe formyle ou hydroxyméthyle,
2) un groupe hydroxyle,
3) ou une chaîne dipeptidique comportant un résidu d'acide aminé à chaîne latérale éventuellement substituée et un autre résidu d'acide aminé à chaîne latérale éventuellement substituée, liés ensemble, ou un dérivé de peptide dont le groupe carboxyle C-terminal a été réduit en un groupe formyle ou hydroxyméthyle ;

la chaîne latérale de chacun des résidus d'acide aminé des fragments -Arg-Pro-Arg-, -Ser-His- et -Gly-Pro- de la formule étant éventuellement substituée ;
étant exclu le cas dans lequel $X^2$ représente Leu, $X^3$ représente Lys, $X^4$ représente Met, $X^5$ représente Pro ou Pro-Phe, et où, dans la formule, -Arg-Pro-Arg- représente un fragment -Arg-Pro-Arg- non substitué, -Ser-His- représente un fragment -Ser-His- non substitué, et -Gly-Pro- représente un fragment -Gly-Pro- non substitué ; ou ester d'un tel peptide, ou sel d'un tel peptide.

**2.** Peptide conforme à la revendication 1, dans lequel $X^1$ représente un résidu d'acide aminé à chaîne latérale éventuellement substituée, ou ester d'un tel peptide, ou sel d'un tel peptide.

**3.** Peptide conforme à la revendication 1, dans lequel $X^1$ représente un résidu pGlu éventuellement substitué ou Gln à chaîne latérale éventuellement substituée, ou ester d'un tel peptide, ou sel d'un tel peptide.

**4.** Peptide conforme à la revendication 1, dans lequel $X^1$ est représenté par la formule

$$Y^1\text{-}Y^2$$

dans laquelle

$Y^1$ représente un résidu d'acide aminé comportant 1 à 17 résidus d'acides aminés, identiques ou non, dont chacun comporte une chaîne latérale éventuellement substituée, ou une chaîne peptidique ;
et $Y^2$ représente un résidu d'acide aminé comportant 1 à 8 résidus d'acides aminés, identiques ou non, dont chacun comporte une chaîne latérale éventuellement substituée, ou une chaîne peptidique ;

ou ester d'un tel peptide, ou sel d'un tel peptide.

**5.** Peptide conforme à la revendication 4, dans lequel $Y^2$ représente une chaîne peptidique représentée par :

1) la formule $B^1$-$B^2$-$B^3$-$B^4$-$B^5$-$B^6$-$B^7$-$B^8$ dans laquelle chacun des symboles B1 à B8 représente un résidu d'acide aminé à chaîne latérale éventuellement substituée, ces résidus pouvant être identiques ou non ;
2) la formule $B^2$-$B^3$-$B^4$-$B^5$-$B^6$-$B^7$-$B^8$ dans laquelle les symboles ont les mêmes significations que celles indiquées ci-dessus ;
3) la formule $B^3$-$B^4$-$B^5$-$B^6$-$B^7$-$B^8$ dans laquelle les symboles ont les mêmes significations que celles indiquées ci-dessus ;
4) la formule $B^4$-$B^5$-$B^6$-$B^7$-$B^8$ dans laquelle les symboles ont les mêmes significations que celles indiquées ci-dessus ;
5) la formule $B^5$-$B^6$-$B^7$-$B^8$ dans laquelle les symboles ont les mêmes significations que celles indiquées ci-dessus ;
6) la formule $B^6$-$B^7$-$B^8$ dans laquelle les symboles ont les mêmes mêmes significations que celles indiquées ci-dessus ;
7) la formule $B^7$-$B^8$ dans laquelle les symboles ont les mêmes significations que celles indiquées ci-dessus ;
8) la formule $B^8$ dans laquelle $B^8$ a la même signification que celle indiquée ci-dessus ;

ou ester d'un tel peptide, ou sel d'un tel peptide.

**6.** Peptide conforme à la revendication 5, dans lequel $B^1$ représente un résidu d'acide aminé neutre à chaîne latérale éventuellement substituée, ou ester d'un tel peptide, ou sel d'un tel peptide.

**7.** Peptide conforme à la revendication 6, dans lequel $B^1$ représente un résidu Gly éventuellement substitué, ou ester d'un tel peptide, ou sel d'un tel peptide.

8. Peptide conforme à la revendication 5, dans lequel $B^2$, $B^3$ et $B^4$ représentent chacun un résidu d'acide aminé basique à chaîne latérale éventuellement substituée, ces résidus pouvant être identiques ou non, ou ester d'un tel peptide, ou sel d'un tel peptide.

9. Peptide conforme à la revendication 5, dans lequel $B^5$ représente un résidu d'acide aminé à chaîne latérale aromatique, ou ester d'un tel peptide, ou sel d'un tel peptide.

10. Peptide conforme à la revendication 5, dans lequel $B^6$ et $B^7$ représentent chacun un résidu d'acide aminé basique à chaîne latérale éventuellement substituée, ces résidus pouvant être identiques ou non, ou ester d'un tel peptide, ou sel d'un tel peptide.

11. Peptide conforme à la revendication 5, dans lequel $B^8$ représente un résidu Gln à chaîne latérale éventuellement substituée, ou ester d'un tel peptide, ou sel d'un tel peptide.

12. Peptide conforme à la revendication 4, dans lequel $Y^1$ représente une chaîne peptidique représentée par:

a) la formule $A^1$-$A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ dans laquelle chacun des symboles $A^1$ à $A^{17}$ représente un résidu d'acide aminé à chaîne latérale éventuellement substituée, ces résidus pouvant être identiques ou non ;
b) la formule $A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ dans laquelle les symboles ont les mêmes significations que celles indiquées ci-dessus ;
c) la formule $A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ dans laquelle les symboles ont les mêmes significations que celles indiquées ci-dessus ;
d) la formule $A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ dans laquelle les symboles ont les mêmes significations que celles indiquées ci-dessus ;
e) la formule $A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ dans laquelle les symboles ont les mêmes significations que celles indiquées ci-dessus ;
f) la formule $A^6$-$A^7$-$A^8$-$A^9$-$A^{10}$-$A^{11}$-$A^{12}$-$A^{13}$-$A^{14}$-$A^{15}$-$A^{16}$-$A^{17}$ dans laquelle les symboles ont les mêmes significations que celles indiquées ci-dessus ;
g) la formule $A^7$-$A^8$-$A^9$-$A^{10}$-$A^{11}$-$A^{12}$-$A^{13}$-$A^{14}$-$A^{15}$-$A^{16}$-$A^{17}$ dans laquelle les symboles ont les mêmes significations que celles indiquées ci-dessus;
h) la formule $A^8$-$A^9$-$A^{10}$-$A^{11}$-$A^{12}$-$A^{13}$-$A^{14}$-$A^{15}$-$A^{16}$-$A^{17}$ dans laquelle les symboles ont les mêmes significations que celles indiquées ci-dessus ;
i) la formule $A^9$-$A^{10}$-$A^{11}$-$A^{12}$-$A^{13}$-$A^{14}$-$A^{15}$-$A^{16}$-$A^{17}$ dans laquelle les symboles ont les mêmes significations que celles indiquées ci-dessus ;
j) la formule $A^{10}$-$A^{11}$-$A^{12}$-$A^{13}$-$A^{14}$-$A^{15}$-$A^{16}$-$A^{17}$ dans laquelle les symboles ont les mêmes significations que celles indiquées ci-dessus ;
k) la formule $A^{11}$-$A^{12}$-$A^{13}$-$A^{14}$-$A^{15}$-$A^{16}$-$A^{17}$ dans laquelle les symboles ont les mêmes significations que celles indiquées ci-dessus ;
l) la formule $A^{12}$-$A^{13}$-$A^{14}$-$A^{15}$-$A^{16}$-$A^{17}$ dans laquelle les symboles ont les mêmes significations que celles indiquées ci-dessus ;
m) la formule $A^{13}$-$A^{14}$-$A^{15}$-$A^{16}$-$A^{17}$ dans laquelle les symboles ont les mêmes significations que celles indiquées ci-dessus ;
n) la formule $A^{14}$-$A^{15}$-$A^{16}$-$A^{17}$ dans laquelle les symboles ont les mêmes significations que celles indiquées ci-dessus ;
o) la formule $A^{15}$-$A^{16}$-$A^{17}$ dans laquelle les symboles ont les mêmes significations que celles indiquées ci-dessus ;
p) la formule $A^{16}$-$A^{17}$ dans laquelle les symboles ont les mêmes significations que celles indiquées ci-dessus ;
q) ou $A^{17}$, symbole qui a la même signification que celle indiquée ci-dessus ;
r) ou ester d'un tel peptide, ou sel d'un tel peptide.

13. Peptide conforme à la revendication 12, dans lequel $A^1$ représente un résidu d'acide aminé à chaîne latérale aromatique, ou ester d'un tel peptide, ou sel d'un tel peptide.

14. Peptide conforme à la revendication 12, dans lequel $A^2$ et $A^3$ représentent des résidus d'acides aminés neutres à chaîne latérale éventuellement substituée, ou ester d'un tel peptide, ou sel d'un tel peptide.

**15.** Peptide conforme à la revendication 12, dans lequel $A^4$ représente un résidu d'acide L-aminé neutre ou basique à chaîne latérale éventuellement substituée, ou ester d'un tel peptide, ou sel d'un tel peptide.

**16.** Peptide conforme à la revendication 12, dans lequel $A^5$ représente un résidu Pro à chaîne latérale éventuellement substituée, ou ester d'un tel peptide, ou sel d'un tel peptide.

**17.** Peptide conforme à la revendication 12, dans lequel $A^6$ et $A^9$ représentent chacun un résidu d'acide aminé basique à chaîne latérale éventuellement substituée, ces résidus pouvant être identiques ou non, ou ester d'un tel peptide, ou sel d'un tel peptide.

**18.** Peptide conforme à la revendication 12, dans lequel $A^7$ et $A^{10}$ représentent chacun un résidu d'acide aminé dont la chaîne latérale porte un groupe hydroxyle ou un résidu d'acide aminé neutre à chaîne latérale éventuellement substituée, ces résidus pouvant être identiques ou non, ou ester d'un tel peptide, ou sel d'un tel peptide.

**19.** Peptide conforme à la revendication 12, dans lequel $A^8$ représente un résidu d'acide aminé dont la chaîne latérale porte un groupe hydroxyle, ou ester d'un tel peptide, ou sel d'un tel peptide.

**20.** Peptide conforme à la revendication 12, dans lequel $A^8$ représente un résidu Ser, Pro ou Hyp, ou ester d'un tel peptide, ou sel d'un tel peptide.

**21.** Peptide conforme à la revendication 12, dans lequel $A^{10}$ représente un résidu d'acide aminé dont la chaîne latérale porte un groupe hydroxyle, ou ester d'un tel peptide, ou sel d'un tel peptide.

**22.** Peptide conforme à la revendication 12, dans lequel les symboles $A^{11}$ à $A^{14}$ représentent chacun un résidu d'acide aminé neutre à chaîne latérale éventuellement substituée, ces résidus pouvant être identiques ou non, ou ester d'un tel peptide, ou sel d'un tel peptide.

**23.** Peptide conforme à la revendication 12, dans lequel $A^{15}$ représente un résidu d'acide aminé à chaîne latérale aromatique, ou ester d'un tel peptide, ou sel d'un tel peptide.

**24.** Peptide conforme à la revendication 23, dans lequel $A^{15}$ représente un résidu Trp, ou ester d'un tel peptide, ou sel d'un tel peptide.

**25.** Peptide conforme à la revendication 12, dans lequel $A^{16}$ et $A^{17}$ représentent chacun un résidu d'acide aminé neutre à chaîne latérale éventuellement substituée, ces résidus pouvant être identiques ou non, ou ester d'un tel peptide, ou sel d'un tel peptide.

**26.** Produit pharmaceutique contenant un peptide conforme à la revendication 1, ou un ester d'un tel peptide, ou un sel d'un tel peptide.

**27.** Produit pharmaceutique conforme à la revendication 26, qui est un régulateur des fonctions du système nerveux central, un régulateur des fonctions circulatoires, un régulateur des fonctions cardiaques, un régulateur des fonctions immunitaires, un régulateur des fonctions digestives, un régulateur des fonctions métaboliques ou un régulateur des fonctions des organes de reproduction.

**28.** Produit pharmaceutique conforme à la revendication 26, qui est un agoniste d'une protéine contenant la séquence d'acides aminés présentée dans la séquence n° 26, ou un sel d'un tel composé.

Figure 1

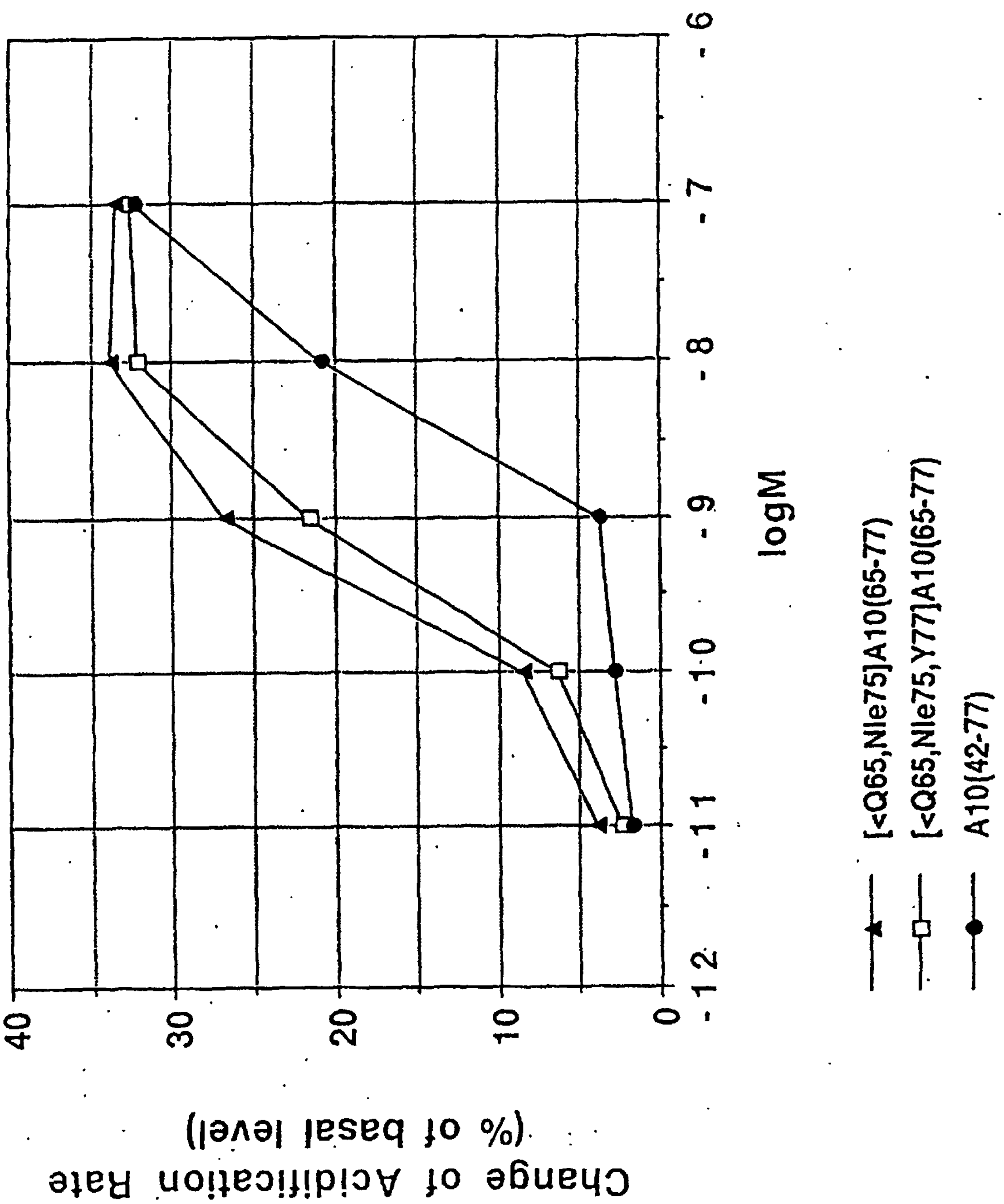
Change of Acidification Rate
(% of basal level)
40
30
20
10
0
-12
-11
-10
-9
-8
-7
-6
logM
[<Q65,Nle75]A10(65-77)
[<Q65,Nle75,Y77]A10(65-77)
A10(42-77)